# EUROPEAN PATENT SPECIFICATION

(11) **EP 1 914 239 B1**
(45) Date of publication and mention of the grant of the patent: **09.06.2010**
(21) Application number: 07018562.4
(22) Date of filing: 05.04.2002
(51) Int. Cl.: C07K 14/285, C12N 1/21, A61K 39/102, A61K 38/40, C07K 16/12, C12Q 1/68, G01N 33/53, A61K 35/74

(54) **Attentuated gram negative bacteria**
Abgeschwächte gramnegative Bakterien
Bactérie négative à gramme attentuée

(43) Date of publication of application: 23.04.2008
(62) Divisional of application: 02290861.0
(73) Proprietor: MERIAL, 69358 Lyon Cédex 07 (FR)
(72) Inventor: Crooke, Helen Rachel, Wokingham Berkshire RG41 5TU (GB); Shea, Jacqueline Elizabeth, Wokingham Berkshire RG41 5TU (GB); Feldman, Robert Graham, Wokingham Berkshire RG41 5TU (GB); Goutebroze, Sylvain Gabriel, 38118 Saint Baudille de la Tour (FR); Le Gros, François-Xavier, 69230 Saint-Genis-Laval (FR)
(74) Representative: Harding, Charles Thomas

(56) References cited:
- EP-A- 0 889 120
- WO-A-00/61724
- WO-A-94/11024
- WO-A-97/49416
- WO-A-03/086277
- TOWNSEND KIRSTY M ET AL: "Genetic organization of Pasteurella multocida cap loci and development of a multiplex capsular PCR typing system." JOURNAL OF CLINICAL MICROBIOLOGY, vol. 39, no. 3, March 2001 (2001-03), pages 924-929, XP002202782 ISSN: 0095-1137
- FULLER TROY E ET AL: "Identification of Pasteurella multocida virulence genes in a septicemic mouse model using signature-tagged mutagenesis." MICROBIAL PATHOGENESIS, vol. 29, no. 1, July 2000 (2000-07), pages 25-38, XP002202784 ISSN: 0882-4010
- MAY BARBARA J ET AL: "Complete genomic sequence of Pasteurella multocida, Pm70." PROCEEDINGS OF THE NATIONAL ACADEMY OF SCIENCES OF THE UNITED STATES, vol. 98, no. 6, 13 March 2001 (2001-03-13), pages 3460-3465, XP002202785 March 13, 2001 ISSN: 0027-8424
- HENSEL M ET AL: "SIMULTANEOUS IDENTIFICATION OF BACTERIAL VIRULENCE GENES BY NEGATIVE SELECTION" SCIENCE, AMERICAN ASSOCIATION FOR THE ADVANCEMENT OF SCIENCE,, US, vol. 269, 21 July 1995 (1995-07-21), pages 400-403, XP000645478 ISSN: 0036-8075
- FLEISCHMANN R D ET AL: "WHOLE-GENOME RANDOM SEQUENCING AND ASSEMBLY OF HAEMOPHILUS INFLUENZAE RD" SCIENCE, AMERICAN ASSOCIATION FOR THE ADVANCEMENT OF SCIENCE,, US, vol. 5223, no. 269, 28 July 1995 (1995-07-28), pages 496-512, XP001019123 ISSN: 0036-8075
- LEE MARGIE D ET AL: "Tn10 insertional mutagenesis in Pasteurella multocida." VETERINARY MICROBIOLOGY, vol. 50, no. 1-2, 1996, pages 143-148, XP001084679 ISSN: 0378-1135
- HENSEL M: "Whole genome scan for habitat-specific genes by signature-tagged mutagenesis." ELECTROPHORESIS. GERMANY APR 1998, vol. 19, no. 4, April 1998 (1998-04), pages 608-612, XP001085303 ISSN: 0173-0835

## Description

### FIELD OF THE INVENTION

This invention relates to live attenuated Gram negative bacteria that can be used in immunogenic compositions or in vaccine compositions for the prevention of bacterial infections. The attenuated bacteria can act as an expression vector for expressing immunogens from other pathogenic agents. The polypeptides encoded by the nucleotide sequences or genes found to be appropriate targets for attenuation of bacteria can be used as immunogenic compounds notably in immunogenic compositions or in vaccine compositions.

### BACKGROUND TO THE INVENTION

It is well established that live attenuated micro-organisms can be highly effective vaccines; immune responses elicited by such vaccines are often of greater magnitude and of longer duration than those produced by non-replicating immunogens. One explanation for this may be that live attenuated strains establish limited infections in the host and mimic the early stages of natural infection. In addition, unlike killed preparations, live vaccines are able to induce potent cell-mediated responses which may be connected with their ability to replicate in antigen-presenting cells, such as macrophages.

There has been a long history of the use of live attenuated vaccines in animals and humans, notably using chemical mutagenesis techniques. However, empirically attenuated vaccines can revert to virulence.

Modem molecular biology techniques, coupled with the increasing knowledge of bacterial pathogenesis, has led to the identification of several genes that are involved in the growth and survival of the micro-organisms *in vivo.* This has provided new gene targets for attenuation, and to the concept that future vaccine strains could be 'rationally' attenuated by introducing defined non-reverting mutations into selected genes known to be involved in virulence, see for example WO-A-00161724, WO-A-00/68261 and EP-A-0889120.

Although many attenuated strains have been produced in laboratories, only a few have qualified as potential vaccine candidates for use in animals. This may be due in part to the need to balance the immunogenicity of the vaccine with the possibility of the micro-organism to revert, becoming reactive and pathogenic.

It is clear that the selection of appropriate genes for attenuation, which will resit in a suitable vaccine candidate, is not straightforward and cannot easily be predicted. Many factors may influence the acceptability of an attenuated mutant as a vaccine, and consequently research effort is required to identify and select suitable attenuating genes. Many attenuation experiments were conducted only *in vitro* and their results cannot be extrapolated *in vivo,* notably in rotation to residual pathogenicity of the resulting mutants for the vaccinated animals.

It is therefore desirable to characterise genes involved in attenuation and on this basis to develop attenuated bacteria as well as attenuated vaccines, in particular having a high degree of immunogenicity and which exhibit a good safety profile with limited or no side effects.

### DESCRIPTION OF THE INVENTION

The present description relates to the identification of nucleotide sequences and genes involved in the attenuation of a micro-organism. Mutations introduced into these nucleotide sequences and genes produce novel attenuated mutants. These mutants are useful for the production of live attenuated immunogenic compositions or live attenuated vaccines having a high degree of immunogenicity.

The present invention provides a mutant of a Gram negative bacterium, wherein said bacterium has a mutation in a nucleotide sequence which codes for a polypeptide having an identity which is equal or more than 80%, 85%, 90%, 95%, 96%, 97%, 98% or 99% with an amino acid sequence coded by the nucleotide sequence SEQ ID NO: 90 said mutation resulting in attenuated virulence of the bacterium.

In another aspect, the present invention provides an immunogenic composition comprising an attenuated mutant according to the present invention, and a pharmaceutically acceptable diluent, carrier or excipient.

Further, the present invention provides a vaccine comprising an attenuated mutant according to the present invention, and a pharmaceutically acceptable diluent, carrier or excipient.

The present invention provides, in another aspect, the use of a mutant of a Gram negative bacterium according to the present invention in the production of a live attenuated immunogenic composition or preparation of a live attenuated vaccine composition.

The present invention provides, in a further aspect, a Gram negative bacterium according to the present invention, an immunogenic composition according to the present invention, or a vaccine according to the present invention, for use in the immunisation against or prevention of bacterial infection in an animal.

In a further aspect, the present invention provides the use of a mutant of a Gram negative bacterium according to the present invention, an immunogenic composition according to the present invention, or a vaccine according to the present invention, in the preparation of a medicament for the immunisation against or prevention of bacterial infection in an animal.

In a first aspect, the description provides bacteria containing an attenuating mutation in a nucleotide sequence or a gene wherein the mutation modifies, reduces or abolishes the expression and/or the biological activity of a polypeptide or protein encoded by a gene, resulting in attenuated virulence of the bacterium.

The mutation is not necessarily located within a gene to disrupt its function, leading to attenuation. The mutation can also be made in nucleotide sequences involved in the regulation of the expression of the gene, in particular regions that regulate transcription initiation, translation and transcription termination. Thus also included are promoters and ribosome binding regions (in general these regulator elements lie approximately between 60 and 250 nucleotides upstream of the start codon of the gene ; Doree S M et al., J. Bacteriol. 2001, 183(6): 1-983-9 ; Pandher K et al., Infect. Imm. 1998, 66(12): 5613-9 ; Chung J Y et al., FEMS Microbiol letters 1998, 166: 289-296), transcription terminators (in general the terminator is located within approximately 50 nucleic acids downstream of the stop codon of the gene ; Ward C K et al., Infect. Imm. 1998, 66(7): 3326-36). In the case of an operon, such regulatory regions may be located in a greater distance upstream of the gene. Sometimes, a mutation in an intergenic region may also lead to attenuation.

A mutation within such regulatory sequences associated with the gene so that the mutation of this nucleotide sequence modifies, inhibits or abolishes the expression and/or the biological activity of the polypeptide or the protein encoded by the gene, resulting in attenuated virulence of the bacterium would be an equivalent to a mutation within a gene identified herein.

Attenuation reduces or abolishes the pathogenicity of the bacteria and the gravity of the clinical signs or lesions, decreases the growth rate of the bacteria and prevents the death.

The invention concerns Gram negative bacteria, more particularly the *Pastaurellaceae* family, notably *Pasteurella multocida, Pasteurella haemolytica, Pasteurella anatipestifer* and *Actinobacillus pleuropneumoniae.* The preferred bacteria is *Pasteurella multocida.*

*Pasteurella multocida* is a Gram-negative bacterium, which is the causative agent of various diseases of production animals and an opportunistic human pathogen. It is the aetiologic agent of severe pasteurellosis, such as fowl cholera in domestic and wild birds, bovine haemorrhagic septicaemia and porcine atrophic rhinitis (Hunt ML et al., Vet Microbiol 2000, 72(1-2): 3-25). Isolates may be grouped serologically based on the capsular antigens into serogroups (A, B, D, E and F) or into 16 serotypes based on somatic LPS antigens.

Potential nucleotide sequences involved in attenuation of bacteria have been identified using Signature Tagged Mutagenesis (STM). This method has been described in detail in WO-A-96/17951.

Briefly, the STM method involves the insertion of a unique, signature-tagged, transposon into the genome of a micro-organism. At the locus of insertion, the genome nucleotide sequence is disrupted and the resulting mutation is checked for attenuation. The sequence of the disrupted region (e.g. gene or open reading frame (ORF)) for each attenuated mutant is determined by PCR-amplification (polymerase chain reaction), cloning and sequencing of the DNA regions flanking the transposon. The STM method described in WO-A-96/17951 was adapted to be functional in *Pasteurella multocida.* These adaptations are notably the use of the Tn10 transposon rather than Tn5, the use for selection of a CDM medium without leucine rather than a streptomycin resistance selection. More details are given in the examples.

A further selection of genes involved in attenuation from the potential genes identified by the STM method, is based on absence of mortality after inoculation of the mutant bacteria to animals. For veterinary application, one advantageous aspect of the description comprises the implementation of an experimental selection directly in the target animal, rather than in an animal model. This method allows a more accurate selection for appropriate mutations of the mutant bacteria. For *Pasteurella multocida,* experiments are done directly in turkeys, one of its natural target host Turkeys are Inoculated intramuscularly with a sufficient amount of pools of signature-tagged *P*. *multocida* mutants (e.g. 0.5 ml, 10⁷ CFU per animal). The mutants that are not re-isolated at a certain time after inoculation are considered as potentially attenuated. The mutants which are not re-isolated are distinguished from those in the pool that are re-isolated by PCR amplification and analysis of the signature tags. Each potentially attenuated mutant is then Injected by the intramuscular route into turkeys (e.g. 0.5 ml, 10⁴ CFU per animal). The mortality of the turkeys is recorded daily for 7 days after the inoculation. The mutants not leading to death are considered as attenuated.

The nucleotide sequences flanking the locus of the transposon insertion are designated SEQ ID NO: 1, 4, 5, 8, 11, 14, 15, 18, 21, 24, 27, 30, 33, 36, 39, 42, 45, 48, 51, 54, 57, 60, 63, 66, 69, 72, 73, 77, 80, 83, 86, 89.

The genes where insertion of the transposon occurred have been characterized. Their nucleotide sequence in *Pasteurella multocida* strain PM70 are designated SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90. The attenuating mutation can be made within these nucleotides sequences or genes as well as in the complementary sequences thereof. The attenuating mutation can also be made in nucleotide sequences involved in the regulatory region of the said genes.

The term of "complementary" means herein the nucleotide sequence of the other strand in the double-stranded genome, so covers the anti-sense strand as complement of the sense strand, and conversely. The term "nucleotide" also encompasses deoxyribonucleotide (so constituted with deoxyribonucleic acids or DNA), ribonucleotide (so.constituted with ribonucleic acids or RNA) and messenger ribonucleotide (mRNA).

More generally attenuating mutations can be introduced into the genome of a bacterium in particular a Gram negative bacterium, more particularly a *Pasteurellacaea* family member, e.g. *P. multocida, P. haemolytica, P. anatipestifer, A. pleuropneumoniae,* and more preferably in the genome of any one of the various strains of *P. multocida,* in at least one nucleotide sequence which codes for an amino acid sequence that has at least about 70% identity, at least about 75% identity, at least about 80% identity, at least about 85%, at least about 90% identity, and at least about 95, 96, 97, 98, or 99% or more identity to one of the amino acid sequences coded by a nucleotide sequence identified as SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90. This encompasses the genetic code degeneration. The percentage of identity between two amino acid sequences can be established by the NCBI (National Center for Biotechnology Information) pairwise blast and the blosum62 matrix, using the standard parameters. The verb "code" used herein does not mean that the nucleotide sequence is limited to an actual coding sequence but also encompasses the whole gene including its regulatory sequences which are non-coding sequences.

The description concerns the mutation of the nucleotide sequences or genes encoding polypeptides or proteins having the same biological function. The similarity of function may be checked by the conservation of active sites. This can be done by a NCBI DART research (Domain Architecture Retrieval Tool).

The present invention thus provide attenuated mutants of a bacterium as described above, comprising an attenuating mutation as defined above.

The mutants can comprise more than one mutation, which may result in additive or synergistic degrees of attenuation, and may result in a better prevention of the reversion of attenuation.

These multiple mutations may associate mutation(s) into nucleotide sequences or genes known for their attenuating properties such as aro genes, for example aroA (Homchampa P. et al., Veterinary Microbiology, 1994, 42: 35-44), and mutations into nucleotide sequences or genes according to the description.

The mutations may be introduced into the micro-organism using any known technique, such as for example recombinant DNA-technology in order to introduce a well-defined mutation in the selected gene. Such a mutation may be an insertion of heterologous nucleic acid sequence, a deletion, a substitution, say a replacement of at least one nucleotide by another one or a combination thereof. In a first preferred embodiment, the mutation is a deletion mutation, where disruption of the gene is caused by the deletion of part and preferably by the deletion of the whole nucleic acid sequence of the gene. Deletion of nucleic acids avoids the reversion to pathogenicity. In a second preferred embodiment the mutation is an insertion made in a locus which corresponds to the transposon insertion loci described in the examples. These loci are in particular those located between: nucleotides 180-181 in SEQ ID NO: 2, 77-78 or 1027-1028 in SEQ ID NO: 6, 416-417 in SEQ ID NO: 9, 389-390 in SEQ ID NO: 12, 381-382 in SEQ ID NO: 16, 219-220 in SEQ ID NO: 19, 1353-1354 in SEQ ID NO: 22, 136-137 in SEQ ID NO: 25, 384-385 in SEQ ID NO: 28, 222-223 in SEQ ID NO: 31, 217-218 in SEQ ID NO: 34, 1411-1412 in SEQ ID NO: 37, 943-944 in SEQ ID NO: 40, 855-856 in SEQ ID NO: 43, 369-370 in SEQ ID NO: 46, 111-112 in SEQ ID NO: 49, 443-444 in SEQ ID NO: 52, 4-5 in SEQ ID NO: 55, immediately before the nucleotide 1 in SEQ ID NO: 58, 573-574 in SEQ ID NO: 61, 875-876 in SEQ ID NO: 64, immediately before the nucleotide 1 in SEQ ID NO: 67, 218-219 in SEQ ID NO: 70, 1072-1087 in SEQ ID NO: 75, 64-65 in SEQ ID NO: 78,282-283 in SEQ ID NO: 81, 1431-1432 in SEQ ID NO: 84, 974-975 in SEQ ID NO: 87, 802-803 in SEQ ID NO: 90.

Deletion mutants include those wherein all or part of a specific gene sequence is deleted. In one aspect, the mutation results in deletion of at least one nucleic acid, of at least about 10%, at least about 20%, at least about 30%, at least about 40%, at least about 50%, at least about 60%, at least about 70%, at least about 80%, at least about 90%, at least about 95%, at least about 98%, or at least about 99% of said gene. Preferably the whole gene is deleted.

Methods to introduce the mutations into the specific genomic regions are well-known and will be apparent to the skilled person. For instance, the whole gene to be mutated or a fragment is cloned into a vector and modified in order to abolish its expression and/or its biological activity. The modified DNA fragment is reintroduced into the bacterial genome by genetic recombination, preferably by homologous recombination between the bacterial chromosome and the vector. As an example the vector can be a suicide plasmid as described in Cardenas (Cardenas M et al., Vet Microbiol 2001 May 3; 80(1): 53-61). Advantageously this vector comprises further between the two flanking arms a polystop sequence (6 stop codons, one in each reading frame) to block any possible translation.

The attenuated micro-organism of the invention, e.g. *P. multocida,* may further comprise at least one heterologous nucleic acid sequence inserted into its genome, wherein said heterologous nucleic acid sequence preferably codes for an immunogen from a pathogenic viral, parasitic or bacterial agent which is different from the attenuated micro-organism. This heterologous sequence may encode an immunogen from another *P. multocida* strain. An immunogen is a protein, polypeptide or peptide which is able to induce an immune response against the pathogenic agent or a secreted antigen.

Heterologous nucleic acid sequences which are suitable for this use in such a vector will be apparent to the skilled person (Fedorova ND and Highlander SK, Infect Immun 1997, 65(7): 2593-8) and include for example those coming from *Pasteurellaceae* family members (notably *Pasteurella multocida, Pasteurella haemolytica, Pasteurella anatipestifer, Actinobacillus pleuropneumoniae*)*,* or from bacteria like *E. coli, Salmonella, Campylobacter*

The heterologous sequence is inserted so as to be expressed by the micro-organism in the host when administered in order to develop an immune response against both the attenuated micro-organism and said expressed immunogen. The heterologous sequence is preferably inserted with the regulatory elements allowing its expression, such as a promoter. Nucleotide sequences useful for the addressing and the secretion of the protein may also be added.

In one embodiment the heterologous sequence is inserted within the selected nucleotide sequence or the selected gene used for the attenuation, in particular inserted in one of the loci corresponding to the transposon insertion loci identified herein.

To improve the expression, the codon usage can be adapted to the bacterial vector used.

The attenuated mutants of the invention may also comprise a nucleic acid sequence encoding a therapeutic protein, an allergen, a growth factor or a cytokine.

According to a further aspect of the invention attenuated micro-organisms are used to produce live attenuated immunogenic compositions or live attenuated vaccine compositions. According to one particular aspect of the invention, the attenuated micro-organism is *P. multocida.* Advantageously as described before the micro-organism may act as a recombinant vector to immunise animals or a human against infections caused by other agents than *Pasteurella.*

The immunogenic compositions or the vaccine compositions comprise the attenuated mutant and a pharmaceutically acceptable excipient, diluent or vehicle, and optionally a stabiliser and/or an adjuvant.

The term of "immunogenic composition covers herein any composition able, once it has been injected to animals or to a human to elicit an immune response against the targeted pathogen. The term of "vaccine composition" or "vaccine" covers herein any composition able, once it has been injected to animals or to a human to induce a protective immune response against the targeted pathogen.

The pharmaceutically acceptable vehicle may be water or saline, but it may e.g. also comprise bacteria culture medium.

The live attenuated bacteria according to the invention may be freeze-dried preferably with a stabiliser. Freeze-drying can be done according to well-known standard freeze-drying procedures. The pharmaceutically acceptable stabilisers may be carbohydrates (e.g. sorbitol, mannitol, lactose, sucrose, glucose, dextran, trehalose), sodium glutamate (Tsvetkov T et al., Cryobiology 1983, 20(3): 318-23 ; Israeli E et al., Cryobiology 1993, 30(5): 519-23), proteins such as peptone, albumin, lactalbumin or casein, protein containing agents such as skimmed milk (Mills CK et al., Cryobiology 1988, 25(2): 148-52 ; Wolff E et al., Cryobiology 1990, 27(5): 569-75), and buffers (e.g. phosphate buffer, alkaline metal phosphate buffer).

An adjuvant may be used to make soluble the freeze-dried preparations.

Examples of adjuvants are oil-in-water, water-in-oil-in-water emulsions based on mineral oil and/or vegetable oil and non ionic surfactants such as block copolymers, Tween®, Span®. Other suitable adjuvants are for example vitamin E, saponins, and Carbopol®, aluminium hydroxide or aluminium phosphate ("Vaccine Design, The subunit and adjuvant approach", Pharmaceutical Biotechnology, vol. 6, Edited by Michael F. Powell and Mark J. Newman, 1995, Plenum Press New York).

The live attenuated bacteria may be stored at -70°C in a medium containing glycerol.

Optionally, the immunogenic composition or vaccine can be combined with one or more immunogens selected from other pathogenic micro-organisms or viruses in an inactivated or live form.

Another aspect of the description is the use of the nucleotide sequences or genes according to the description, for the expression and the production of immunogens. In a first embodiment, the polypeptides encoded by these nucleotide sequences or genes may be used as subunit immunogens in immunogenic compositions or vaccines.

The preferred polypeptides are those having the amino acid sequences identified as SEQ ID NO: 3, 7, 10, 13, 17, 20, 23, 26, 29, 32, 35, 38, 41, 44, 47, 50, 53, 56, 59, 62, 65, 68, 71, 76, 79, 82, 85, 88, 91, or those encoded by the nucleotide sequences SEQ ID NO: 2, 6, 9, 12, 16, 19, 22, 25, 28, 31, 34, 37, 40, 43, 46, 49, 52, 55., 58, 61, 64, 67, 70, 75, 78, 81, 84, 87, 90.

The description encompasses the equivalent polypeptides from another bacterium, in particular a Gram negative bacterium, more particularly a *Pasteurellacaea* family member, e.g. *P. multocida, P. haemolytica, P. anatipestifer, A. pleuropneumoniae,* and more preferably in the genome of any one of the various strains of *P. multocida.* Are thus included by equivalence polypeptides whose amino acid sequences have at least about 70%, at least about 75%, at least about 80%, at least about 85%, at least about 90%, at least about 95%, and at least about 96, 97, 98 or 99% identity to one of the amino acid sequences identified as SEQ ID NO: 3, 7, 10, 13, 17, 20, 23, 26, 29, 32, 35, 38, 41, 44, 47, 50, 53, 56, 59, 62, 65, 68, 71, 76, 79, 82, 85, 88, 91, and/or polypeptides that have the same biological function(s) than the polypeptides identified above with SEQ. The criteria for establishing the identity or the same biological function have been described above.

The description also embraces the immunogenic fragments of these polypeptides, having at least a chain of 10 amino acids of the polypeptide, at least 20, in particular at least 30, preferably at least 50 and more preferably at least 70.

The polypeptides or fragments are produced preferably by *in vitro* expression. The nucleotide sequences according to the description (e.g. SEQ ID NO: 2, 6, 9, 12, 16, 19, 22, 25, 28, 31, 34, 37, 4.0, 43, 46, 49, 52, 55, 58, 61, 64, 67, 70, 75, 78, 81, 84, 87, 90) or fragments thereof are inserted into a vector, operably linked to regulatory elements such as promoter, ribosome binding region and terminator, and start codon and stop codon. Preferred vectors are plasmids useful for *in vitro* expression in bacteria i.e. *Escherichia coli* (Mahona F et al., Biochimie 1994, 46(1): 9-14 ; Watt M A et al., Cell Stress Chaperones 1997, 2(3): 180-90 ; Frey J Res. Microbiol. 1992, 143(3): 263-9).

These polypeptides can also be synthesised chemically (Luo Y et al., Vaccine 1999, 17(7-8): 821-31).

An object of the description is thus an immunogenic composition or vaccine comprising at least one polypeptide or fragment according to the description (sub-unit immunogenic composition or vaccine) or at least one *in vivo* expression vector as described above (live recombinant immunogenic composition or vaccine), and a pharmaceutically acceptable excipient, diluent or vehicle, and optionally an adjuvant. Examples of such ingredients have been described above in relation to the live vaccine.

In a second embodiment, these nucleotide sequences or their fragments may be inserted into recombinant vectors to produce live recombinant immunogenic compositions or vaccines able to express *in vivo* in the host the polypeptide encoded by this nucleotide sequence or fragment

The *in vivo* expression vector can be a polynucleotide vector or plasmid (EP-A2-1001025 ; Chaudhuri P Res. Vet Sci. 2001, 70(3), 255-6), viruses (e.g. adenovirus, poxvirus such as fowlpox (US-A 5,174,993 US-A-5,505,941 and US-A-5,766,599) or canarypox (US-A-5,756,103)) or bacteria i.e. *Escherichia coli* or *Salmonella sp.*

Polypeptides and fragments of the description may also be used in therapy.

Said polypeptides and fragments may also be used as reagents in antibody-antigen reaction. Another objects of the description are thus a diagnostic method for detecting infection by the Gram negative bacterium, as well as kits (e.g. ELISA) including at least one polypeptide or fragment according to the description.

Antibodies against the above polypeptides or fragments can be used as a diagnostic reagent or in passive immunization or vaccination or in therapy. Another objects of the description are an antibody preparation comprising an antibody specific to a polypeptide or a fragments according to the description and methods of diagnosis using the same. Antibodies can be polyclonal or monoclonal. Methods for producing antibodies are well-known to the man skilled in the art. If polyclonal antibodies are desired, a selected animal (e.g. mouse, rabbit, goat, horse, etc.) is immunized with a polypeptide or a fragment Serum from the immunized animal is collected and treated according to known procedures and possibly purified. See, e.g. Jurgens et al. J. Chrom., 1985, 348: 363-370. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. See, e.g. J. E. Liddell "A practical guide to monoclonal antibodies" ed. John Wiley and sons, 1991, p.188; S. J. de StGroth et al. J. Immunol. Methods, 1980, 35(1-2), 1-21.

The nucloetide sequences according to the description and their fragments may be used as a probe for hybridization, e.g. in a diagnostic method.

Stringent hybridisation conditions are preferably used. One can refer to those described by Sambrook et al., Molecular Cloning, A Laboratory Manual, Cold Spring Harbor Laboratory Press (1989), 1.101-1.104. Hybridisation under stringent conditions means that a positive hybridisation signal is still observed after washing for 1 hour with 1 x SSC buffer and 0.1 % SDS at 55°C, preferably at 62°C and most preferably at 68°C, in particular, for 1 hour in 0.2 x SSC buffer and 0.1 % SDS at 55°C, preferably at 62°C and most preferably at 68°C.

The nucleotide sequences according to the description and their fragments may be used as primers for PCR or similar in particular for detection of Gram negative bacteria in any media, for example tissue samples, biological fluids, water, food.

Preferably use is made of nucleotide sequence fragments which have at least 20, at least 30, at least 50, at least 70 or at least 100 nucleic adds of nucleotide sequences or genes according to the description, in particular of SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90.

Further the present description relates to methods to immunise against or to prevent bacterial infection in animals, preferably in avian, rabbit, bovine and porcine species and more preferably for avian species in chicken, turkey and duck (this includes breeders, broilers and layers) or in a human. According to these methods, (1) a live attenuated immunogenic composition or vaccine, (2) a sub-unit immunogenic composition or vaccine, (3) a live recombinant immunogenic composition or vaccine, or their combinations, are administered.

The administration may be notably made by intramuscular (IM), intradermal (ID) or subcutaneous (SC) injection or via intranasal, intratracheal or oral administration. The immunogenic composition or the vaccine according to the invention is administered by syringe, needleless apparatus (like for example Pigjet, Avijet, Dermojet or Biojector (Bioject, Oregon, USA)), spray, drinking water, eye-drop.

Preferred administrations for the live attenuated immunogenic composition or vaccine are *in ovo,* via the oral (e.g. drinking water, whole body spray), ocular (e.g. eye-drop, whole body spray), tracheal. (e.g. spray), intradermal, subcutaneous (SC) or intramuscular (IM) routes.

The quantity of live attenuated micro-organisms can be determined and optimised by the skilled person. However, generally an animal may be administered approximately 10⁴-10⁹ CFUs, preferably approximately 10⁵-10⁸ CFUs and more preferably approximately 10⁸-10⁷ CFUs in a single dosage unit.

By intramuscular route an avian animal may be administered approximately 10⁴-10⁷ CFUs, preferably approximately 10⁵-10⁶ CFUs in a single dosage unit. The volume of one single dosage unit can be between 0.2 ml and 0.5 ml and preferably 0.3 ml. By oral, tracheal or ocular route an avian animal may be administered approximately 10⁵-10⁸ CFUs, preferably approximately 10⁸-10⁷ CFUs in a single dosage unit. For spray administration the volume is depending to the apparatus and the size of droplets, from about 30 to about 600 ml for 1000 animals and preferably 0.2 ml per animal.

For bovine and porcine animals, the preferred routes are IM and SC. The animal may be administered approximately 10⁴-10⁹ CFUs, preferably approximately 10⁵-10⁸ CFUs in a single dosage unit. The volume of one single dosage unit can be between 0.2 ml and 5.0 ml and preferably between 0.5 ml and 2.0 ml and more preferably 1.0 ml.

Rabbits may be administered via IM or SC route approximately 10⁴-10⁸ CFUs, preferably approximately 10⁵-10⁷ CFUs in a single dosage unit The volume of one single dosage unit can be between 0.2 ml and 0.5 ml and preferably 0.5 ml. They may also be administered via ID route approximately 10⁴-10⁸ CFUs, preferably approximately 10⁵-10⁷ CFUs in a single dosage unit. The volume of one single dosage unit can be between 0.1 ml and 0.2 ml.

The following examples illustrate the description. None are attended to limit its scope of applicability.

### EXAMPLES

### Example 1: Construction of a library of signature tagged P. multocida transposon mutants (STM screening)

### Construction of the tagged SM10λpir pLOF/km transformants

Tags were produced as described in Hensel et. al., (Science, 1995, 269:400-403). Initially tagged-pUTminiTn5Km2 plasmids were selected that contain tags that hybridise well but do not cross hybridise with each other. The mini-Tn5 transposon in the tagged pUTminiTn5Km2 vector was found not to transpose in several strains of *Pasteurella multocida.* In contrast the transposon mini-Tn10 has been shown to function in *P. multocida* (Lee et. al., Vet Microbiol, 1996, 50:143-8). The pre-selected tags were therefore transferred from the pUTminiTn5 vectors into the mini-Tn10 containing plasmid pLOF/Km (Herrero et al., J. Bacteriology, 1990, 172: 6557-6567). The tags were amplified by PCR using primers that bind to the pUTminiTn5Km2 vector, either side of the *Kpn*I site into which the tags were cloned. These primers included sequences for the restriction enzyme *Sal*I. The PCR products were then digested with *Sal*I and cloned into the *Sal*I site of a modified version of the pLOF/Km vector, in which a *Sal*I site has replaced the unique *Sfi*I cloning site. The tagged pLOF/Km plasmids were then transformed into the *E. coli* strain 8M10λpir (Km^{r}, *thi, thr, leu, tonA, lacY, supE,* recA::RP4-2-Tc::Muλpir) (Miller V.L. et al., J. Bacteriol., 1988, 170: 2575-83). This strain can mobilise plasmids such as pLOF/Km into recipient bacteria by conjugation.

### Methods of selecting and counter-selecting

The conjugation process requires a method of selecting for the recipient *P. multocida,* which have acquired the plasmid pLOF/KM and a method of counter-selection against the *E. coli* SM10λpir donor.

In the method of selecting the recipient *P*. *multocida* are selected for using kanamycin, which is encoded by the mini-Tn10 transposon.

Initially for counter-selection against the *E. coli* donor in conjugations, a spontaneously streptomycin resistant mutant of Pasteurella strain P-1059 was used. However it appeared that this strain was attenuated in virulence for turkeys and thus was not usable here. *Pasteurella multocida* strains can grow on a chemically defined media (CDM) (Hu et. al., Infection and Immunity 1986, 804-810). A modified version of this chemically defined medium containing agar but not containing leucine was utilised to allow counter-selection against the *E. coli* donor. The Pasteurella strain was able to grow on this medium, the composition of which is given in table 1, whereas the *E. coli* SM10λpir strain, which is a leucine auxotrophe did not.

**Table 1:**

| Component | Concentration G/litre |
|---|---|
| Noble Agar | 20 |
| Na₂HPO₄-12H₂O | 32.31 |
| KH₂PO₄ | 1.368 |
| NaCl | 1.196 |
| Glucose | 6.0 |
| L-Arginine hydrochloride | 0.24 |
| L- cysteine Hydrochloride | 0.12 |
| L-Serine | 0.2 |
| L-glutamic acid | 0.15 |
| L-isoleucine | 0.064 |
| L-phenylalanine | 0.095 |
| L-Aspartic acid | 1.6 |
| L-tyrosine | 0.08 |
| Thiamine hydrochloride | 0.0002 |
| MgSO₄.7H₂0 | 0.246 |
| Calcium pantothenate | 0.004 |
| Nicotinamide | 0.01 |
| Orotic acid | 0.003 |

### Passaging of P.multocida strain on CDM media

A lyophilised ampoule of P. multocida strain (USDA P-1059, available before the American Type Culture Collection, accession number ATCC 15742) was revived by the addition of 200µl of BHI (brain-heart infusion) and an aliquot of the suspension streaked onto a BHI agar plate and the plate incubated at 37°C overnight. Colony material from this plate was used to inoculate a BHI broth culture, which was incubated with shaking at 37°C overnight. Glycerol was added to a final concentration of 15% v/v and aliquots were stored frozen at -80°C. A sample from of one of these frozen aliquots was streaked onto a BHI agar plate and incubated overnight. Colony material from this BHI plate was then streaked onto CDM agar plates with the composition given in table 1 and incubated at 37°C for 3 days. Colony material from this CDM plate was inoculated into a BHI broth culture and incubated with shaking at 37°C overnight. Glycerol was added to this culture to a final concentration of 15% v/v and aliquots frozen at -80°C. This strain was termed 16084 (CDM).

### Construction of the mutant bank

The tagged SM10λpir pLOF/km transformants were conjugated with the 16084 (CDM) *P. multocida* strain. To minimise the isolation of sibling mutants (mutants with the transposon located in the same position that arise due to replication of the mutant during the conjugation procedure) each tagged SM10λpir transformant was conjugated with the *P.multocida* strain in at least three separate conjugations. Pasteurella transposon mutants were selected on CDM agar plates supplemented with 50µg/ml kanamycin. The kanamycin resistant mutants for each of the tagged transposons were then streaked to form single colonies twice on BHI kanamycin 50µg/ml agar plates. Single colonies were then inoculated into BHI broth cultures, grown overnight at 37°C with shaking. Glycerol was then added to a final concentration of 15% v/v and the mutants stored at -80°C in individual vials.

### Example 2: Screening of the signature-tagged Pasteurella mutant bank for mutants attenuated in virulence for turkeys.

Cultures of the P.multocida mutants were grown for inoculation of turkeys by mixing 20µl of each of the glycerol stocks of the mutants obtained in example I with 200µl of BHI culture medium, supplemented with 50µg/ml of kanamycin, and placing in 96 well microtitre dishes. These microtitre dishes were incubated in static conditions for about 18 hours at 37°C. Then 10µl aliquots of the 18 hour cultures of each mutant were mixed with 200µl of BHI culture medium supplemented with 50µg/ml of kanamycin in a fresh microtitre plate and the plate incubated at 37°C for approximately 4 hours. The cultures were stopped in the exponential phase of growth and 100µl of the cultures of each mutant were transferred to a fresh microtitre plate and used for determination of the optical density (OD) at 650 nm.

The inocula or input pools were formed by mixing the remaining 100µl of the 4 hour cultures. Each input pool consisted of 48 different mutants. The titre of these pooled suspensions were determined by FACS (fluorescence activated cell sorter) analysis of 1000µl aliquots. Aliquots (1ml) of the pooled suspension were then diluted in physiologically buffered water to obtain a suspension with a titre of 2.10⁷cfu/ml. Groups of 5 three-week-old turkeys were then inoculated intramuscularly with 0.5ml aliquots of this suspension (10⁷ cfu per animal). The serological status of the turkeys prior to inoculation was determined by screening for the presence of antibodies to Pasteurella in blood samples taken one day before inoculation. The cells from the remainder of the input pools were harvested by centrifugation and chromosomal DNA extracted from the cell pellets.

Approximately 14 hours after inoculation 1 ml blood samples were taken from 3 of the 5 turkeys. Dilution series (10⁻¹ to 10⁻⁷) of the blood samples were plated onto Columbia agar plates supplemented with 5% sheeps blood. The plates were incubated at 37°C for 24 hours after which time approximately 10000 Pasteurella colonies were resuspended in BHI medium. These suspensions, which are termed the output pool, were then centrifuged and chromosomal DNA extracted from the cell pellet.

Pasteurella mutants that were present in the input pool but were not re-isolated from the turkeys were identified by PCR amplification of the signature tags present in DNA samples from the input and output pools, and hybridisation of the amplified PCR products against dot blots loaded with DNA encoding the signature tags, as described in Hensel et al. (Science 1995, 269:400-403). These mutants were considered as potentially attenuated in virulence. This attenuation was confirmed by screening for a lack of mortality after single infections of the potentially mutants in turkeys.

### Example 3: Confirmation of the attenuation In virulence for turkeys of the P.multocida mutants.

The transposon mutants identified as potentially attenuated in example 2 or the mutants which have limited ability to grow in culture, were revived by mixing 20µl of the glycerol stocks with 200µl of BHI culture medium supplemented with 50µg/ml of kanamycin in microtitre dishes. These microtitre dishes were incubated in static conditions for 18 hours at 37°C. Then 10µl aliquots of each mutant of these cultures were taken and mixed with 200µl of BHI medium, supplemented with 50µg/ml of kanamycin in a fresh microtitre plate and this plate incubated in static conditions for about 4 hours. The cultures were stopped in the exponential phase of growth and 100µl of the cultures of each mutant were transferred to a fresh microtitre plate and used for determination of the optical density (OD) at 650nm. The cultures of each of the mutants were then diluted 1 in 10000 in physiologically buffered water to obtain a concentration of approximately 2.10⁴ cfu/ml. Aliquots (0.5ml) of these dilutions were then inoculated intramuscularly into 2 five-week-old turkeys (10⁴cfu per animal). The serological status of a few animals from each group of turkeys was determined from blood samples taken the day before inoculation. The turkeys were monitored for the following 7 days for mortality. Of the mutants tested 72 did not result in mortality in either of the two birds inoculated. These 72 mutants were considered attenuated in virulence.

### Example 4: Characterisation of transposon insertion mutants identified after screening in Turkeys

The transposon insertion sites in the genome of attenuated P. *multocida* mutants were identified by cloning the DNA flanking one side of the transposon insertion, either by Inverse PCR or by arbitrarily primed PCR.

These mutants were revived from the -80°C glycerol stocks by streaking an aliquot onto BHI kanamycin 50µg/ml agar plates. Single colonies were then used to inoculate BHI broth cultures from which chromosomal DNA was prepared.

For Inverse PCR the chromosomal DNA was digested with a restriction enzyme that has a 4 base pairs recognition site, such as Tsp509I, αTaqI or Rsal. The DNA is then ligated in a large volume to encourage intra-molecular ligation. The DNA flanking the transposon is then amplified from this ligated DNA template using outwardly facing primers that anneal to known sequence of the transposon. These Inverse PCR products are then cloned and sequenced.

For arbitrarily primed PCR the chromosomal DNA was used as a template in a first round PCR reaction with one outwardly facing primer that anneals to the transposon and an arbitrary primer. The arbitrary primer has 5 known bases at the 3' end and then 10 random bases. The 20 bases at the 5' end of the primer are of known sequence. The annealing temperature of this first round PCR reaction is initially set very low, with the annealing temperature being raised in subsequent cycles. A portion of the products of this first round PCR is then used as a template in a second round PCR. This second round PCR utilises another outwardly facing primer, which anneals to the transposon at a position that is closer to the end of the transposon than the primer used in the first round PCR. The other primer used in this second round PCR has the same sequence as the 20 bases of known sequence at the 5' end of the arbitrary primer used in the first round PCR. The PCR products of this second PCR are then cloned and sequenced.

The sequences obtained were then analysed to identify open reading frames (ORF), which may be disrupted by the transposon and were also used to search for similar sequences currently available in the EMBL database, and in the genome sequence of the *Pasteurella multocida* strain PM70, determined by the University of Minnesota (May BJ et al., Proc. Natl. Acad. Sci. USA, 2001, 98(6): 3460-5).

For information, in the following nucleotide sequences, N is corresponding to any nucleic acid (A or C or G or T).

### Mutant 1G4

The DNA sequence flanking the transposon insertion site is given in SEQ ID NO: 1 (775 mer). The transposon is inserted immediately at the 5' end of this sequence.
A start codon is located at positions 179-181.
Four other *Pasteurellaceae* proteins and genes were identified by blasts done with a 60 amino acid sequence encoded by SEQ ID NO: 1.

| Strain | Gene (Genbank ref.) | Protein (Genbank ref.) | % of identity |
|---|---|---|---|
| P. multocida taxon 747 | PhyA (AF067175) | PhyA (AAC67248) | 100% over 60 amino acids |
| P. multocida PM70 | PM0773 (AE006115) | PhyA (AAK02857) | 98% over 60 amino acids |
| P. multocida P4218 | PhyA (AF302467) | PhyA (AAK17919) | 98% over 60 amino acids |
| P. multocida P934 | PhyA (AF302465) | PhyA (AAK17907) | 95% over 60 amino acids |

The location of the transposon in mutant 1G4 corresponds to position 8507-8508 of the Pasteurella multocida PM70 genome sequence, Genbank Accession number AE006115. The transposon disrupts a homologue of the PM70 gene PM0773, PhyA. The PhyA gene is predicted to be involved in capsule synthesis. The nucleotide sequence of PM0773 is herein identified as SEQ ID NO: 2 and its amino acid sequence as SEQ ID NO: 3.

### Mutants 1G8 and 9D1

In mutant 1G8, the transposon is inserted immediately at the 3' end of the sequence SEQ ID NO: 4 (226 mer). This sequence has two open reading frames (+2 and -2) encoding potential longer proteins. The ORF described herein is in frame 2.

The transposon inserted in mutant 9D1 is immediately at the 3' end of the sequence SEQ ID NO: 5 (87 mer).
The transposons in mutants 1G8 and 9D1 disrupt a homologue of the PM70 gene, PM0871. The locations of the transposons in these mutants correspond to positions 9849-9850 (mutant 1G8) or 8899-8900 (mutant 9D1) of the *Pasteurella multocida* PM70 genome sequence Genbank accession number AE006125. The nucleotide sequence of PM0871 is herein identified as SEQ ID NO: 6 and its amino acid sequence as SEQ ID NO: 7.
One other Pasteurellaceae gene/protein was identified by blasts done with SEQ ID NO: 7. This is *Haemophilus influenzae* HI1586 (Genbank accession numbers U32832 and AAC23234). We find an identity of 72% over 507 amino acids between PM0871 and Hl1586.

### Mutant 2F2

The DNA sequence flanking the transposon insertion site is given in SEQ ID NO: 8 (78 mer). The transposon is immediately at the 5' end of this sequence. This sequence has three open reading frames (+1, +2 and -1) encoding potential longer proteins. The ORF described herein is in frame +2.
The transposon in mutant 2F2 disrupts a homologue gene of PM70 gene PM1727. This transposon is located at a position which corresponds to 644-645 of the *Pasteurella multocida* PM70 sequence, Genbank accession number AE006210 (PM1727). The nucleotide sequence of PM1727 is herein identified as SEQ ID NO: 9 and its amino acid sequence as SEQ ID NO: 10. One other Pasteurellaceae gene/protein was identified by blasts done with SEQ ID NO: 10. This is *Haemophilus Influenzae* Hl0621 (Genbank accession numbers U32744 and AAC22281). We find an identity of 77% over 183 amino acids between PM1727 and HI0621.
PM1727 is a member of a superfamily of hydrolases, in particular it is related to histidinol phosphate phosphatases.

### Mutant 3A2

The DNA sequence flanking the transposon insertion site is given in SEQ ID NO: 11 (467 mer). The transposon is immediately at the 5' end of this sequence.
A stop codon is located at positions 428-430.
The transposon in mutant 3A2 is located at a position which correspond to 5103-5104 of the *Pasteurella multocida* PM70 sequence, Genbank accession number AE006094 (PM0586). The nucleotide sequence of PM0586 is herein identified as SEQ ID NO: 12 and its amino sequence as SEQ ID NO: 13. Two other Pasteurellaceae genes and proteins were identified by blasts done with SEQ ID NO: 13. These genes and proteins are *Pasteurella haemolytica* A1 PIpD (Genbank accession numbers AF058703 and AAC32565) and *Haemophilus somnus* 31 kDa (Genbank accession numbers L07795 and AAA24941). We find an identity of 73% over 276 amino acids between PM0586 and *P. haemolytica* PIpD and of 71% over 273 amino acids between PM0586 and *H. somnus* 31 kDa.
PlpD and PM0586 are members of the ompA protein family.

### Mutant 3D3

The DNA sequences flanking the both sides of the transposon insertion site are given in SEQ ID NO: 14 (204 mer, transposon at the 5' end) and SEQ ID NO: 15 (35 mer, transposon at the 5' end).
A stop codon is located at positions 7-9 of SEQ ID NO: 14 and at positions 33-35 of SEQ ID NO: 15.

One other *Pasteurellaceae* gene/protein was identified by blasts done with SEQ ID NO: 14 and its encoded amino acid sequence (65 amino acids). We find an identity of 100% over 65 amino acids with PM0064 protein. The location of the transposon in mutant 3D3 corresponds to positions 4778-4779 or 4787-4788 of the *Pasteurella multocida* PM70 genome sequence, Genbank accession number AE006042, positions deduced from SEQ ID NO: 14 and 15 respectively. This difference is likely to be due to insertion of the transposon resulting in the duplication of a few nucleotides at the transposon insertion site. Position 4788 is located in the PM0064 gene. Position 4778 is 6 bp downstream of the stop codon of PM0064. The nucleotide sequence of PM0064 is herein identified as SEQ ID NO: 16 and its amino acid sequence as SEQ ID NO: 17.
Other Gram negative bacteria genes and proteins were identified by blasts done with SEQ ID NO: 17.

| Strain | Gene (Genbank ref.) | Protein (Genbank ref.) | % of identity |
|---|---|---|---|
| Haemophilus H10017 influenzae | (U32687) | HI0017 (AAC21695) 81% | over 127 amino acids |
| Escherichia coli K12 | YfiD (AE000344) | yfid (AAC75632) | 81% over 127 amino acids |
| Salmonella typhi | STY2839 (AL627275) | yfiD (CAD02795) | 81 % over 127 amino acids |
| Salmonella typhimurium | STM2646 (AE008820) | yfiD (AAL21540) | 81% over amino acids |
| Serratia liquefaciens | OrfX (X66505) | OrfX (CAA47136) | 79% over 127 amino acids |
| Yersinia pests | YPO2705 (AJ414153) | YP02705 (CAC92944) | 79% over 127 amino acids |
| Vibrio cholerae | VC2361 (AE004306) | VC2361 (AAF95504) | 73% over 127 amino acids |

### Mutant 3D8

The DNA sequence flanking the transposon insertion site is given in SEQ ID NO: 18 (75 mer). The transposon is immediately at the 3' end of this sequence.
The location of the transposon in mutant 3D8 corresponds to between positions 7769-7770 of the *Pasteurella multocida* PM70 genome sequence, Genbank accession number AE006080. The transposon disrupts a homologue of the PM70 gene PM0445. The nucleotide sequence of PM0445 is herein identified as SEQ ID NO: 19 and its amino acid sequence as SEQ ID NO: 20.

### Mutant 3E1

The DNA sequence flanking the transposon insertion site is given in SEQ ID NO: 21 (229 mer). The transposon is immediately at the 3' end of this sequence.
The location of the transposon in mutant 3E1 corresponds to between positions 9195-9196 of *Pasteurella multocida* PM70 genome sequence, Genbank accession number AE006133. The transposon disrupts a homologue of the PM70 gene PM0940. The nucleotide sequence of PM0940 is herein identified as SEQ ID NO: 22 and its amino acid sequence as SEQ ID NO: 23.
Other Gram negative bacteria genes and proteins were identified by blasts done with SEQ ID NO: 17.

| Strain | Gene (Genbank ref.) | Protein (Genbank ref.) | % of identity |
|---|---|---|---|
| Haemophilus influenzae | HI0075 (U32693) | nrdD (AAC21751) | 87% over 713 amino acids |
| Escherichia coll K12 | nrdD (AE000495) | nrdD (AAC77195) | 76% over 709 amino acids |
| Salmonella typhi | STY4791 (AL627283) | nrdD (CAD06912) | 76% over 709 amino acids |
| Salmonella typhimurium | STM4452 (AE008908) | nrdD (AAL23272) | 76% over 709 amino acids |
| Yersinia pestis | YPO3464 (AJ414157) | nrdD (CAC92683) | 75% over 709 amino acids |
| Vibrio cholerae | VCA0511 (AE004381) | VCA0511 (AAF96414) | 74% over 709 amino adds |

### Mutant 3H2

The DNA sequence flanking the transposon insertion site is given in SEQ ID NO: 24 (58 mer). The transposon is immediately at the 3' end of this sequence. This sequence has two open reading frames (+1 and -3) encoding potential longer proteins. The ORF described herein is in frame +1. One other *Pasteurellaceae* gene was identified by blasts done with SEQ ID NO: 24 and with its encoded amino acid sequence (19 amino acids). We find an identity of 100% over 19 amino acids with PM1951 protein. The location of the transposon in mutant 3H2 corresponds to between positions 9418-9419 of the *Pasteurella multocida* PM70 sequence, Genbank accession number AE006231 (PM1951, uvrA). The nucleotide sequence of PM1951 is herein identified as SEQ ID NO: 25 and its amino acid sequence as SEQ ID NO: 26. Other Gram negative bacteria genes and proteins were identified by blasts done with SEQ ID NO: 26.

| Strain | Gene (Genbank ref.) | Protein (Genbank ref.) | % of identity |
|---|---|---|---|
| Haemophilus influenzae | UvrA (U32711) | UvrA (AAC21915) | 89% over 943 amino acids |
| Escherichia coli K12 | UvrA (AE000479) | UvrA (AAC77028) | 80% over 940 amino acids |
| Yersinia pestis | UvrA (AJ414142) | UvrA (CAC89185) | 80% over 943 amino acids |
| Vibrio cholerae | UvrA (AE004127) | UvrA (AAF93567) | 80% over 940 amino acids |
| Salmonella typhi | UvrA (AL627282) | UvrA (CAD09238) | 80% over 941 amino acids |
| Salmonella typhimurium | UvrA (AE008898) | UvrA (AAA27250) | 80% over 941 amino acids |
| Pseudomonas aeruginosa | UvrA (AE004840) | UvrA (AAG07622) | 75% over 943 amino acids |

UvrA is a DNA repair ABC excision nuclease.

### Mutant 4D6

The DNA sequence flanking the transposon insertion site is given in SEQ ID NO: 27 (54 mer). The transposon is immediately at the 5' end of this sequence. This sequence has two open reading frames (+1 and -2) encoding potential longer proteins. The ORF described herein is in frame +1. The location of the transposon in mutant 4D6 corresponds to between positions 6492-6493 of the *Pasteurella multocida* PM70 sequence, Genbank accession number AE006036. The transposon disrupts a homologue of the PM70 genePM0032 or hktE. The nucleotide sequence of PM0032 is herein identified as SEQ ID NO: 28 and its amino acid sequence as SEQ ID NO: 29. One other Pasteurellaceae gene/protein was identified by blasts done with SEQ ID NO: 29. This is *Actinobacillus actinomycetemcomitans* catalase (Genbank accession numbers AF162654 and AAF17882). We find an identity of 85% over 482 amino acids between PM0032 and A. *actinomycetemcomitans* catalase.
HktE is a catalase.

### Mutant 4F4

The DNA sequence flanking the transposon insertion site is given in SEQ ID NO: 30 (172 mer). The transposon is immediately at the 3' end of this sequence.
Four other *Pasteurellaceae* genes and proteins were identified by blasts done with the 57 amino acid sequence encoded by SEQ ID NO: 30.

| Strain | Gene (Genbank ref.) | Protein (Genbank ref.) | % of identity |
|---|---|---|---|
| P. multocida taxon 747 | HyaC (AF067175) | HyaC (AAC67251) | 96% over 57 amino acids |
| P. multocida PM70 | PM0776 (AE006116) | AAK02860 | 96% over 57 amino acids |
| P. multocida P4218 | FcbC (AF302467) | FcbC (AAK17922) | 91% over 57 amino acids |
| P. multocida P934 | DcbC (AF302465) | DcbC (AAK17904) | 88% over 57 amino acids |

The location of the transposon in mutant 4F4 corresponds to between positions 5272-5273 of the *Pasteurella multocida* PM70 genome sequence, Genbank accession number AE006116. The transposon disrupts a homologue of the PM70 gene PM0776. The nucleotide sequence of PM0776 is herein identified as SEQ ID NO: 31 and its amino acid sequence as SEQ ID NO: 32. These proteins are UDP glucose dehydrogenases.

### Mutant 4F12

The DNA sequence flanking the transposon insertion site is given in SEQ ID NO: 33 (226 mer). The transposon is immediately at the 5' end of this sequence.
The location of the transposon in mutant 4F12 corresponds to between positions 9263-9264 of the *Pasteurella multocida* PM70 sequence, Genbank accession number AE006038. The transposon disrupts a homologue of the PM70 gene PM0048 or fadR. The nucleotide sequence of PM0048 is herein identified as SEQ ID NO: 34 and its amino acid sequence as SEQ ID NO: 35. FadR is a homologue of an *E. coli* protein which is a transcription regulator of fatty acid metabolism, affecting several fatty acid biosynthesis (fab) and fatty acid degradation (fad) genes.

### Mutant 4G11

The DNA sequence flanking the transposon insertion site is given in SEQ ID NO: 36 (214 mer). The transposon is immediately at the 3' end of this sequence.
One other *Pasteurellaceae* gene was identified by blasts done with SEQ ID NO: 36 and its encoded amino acid sequence (70 amino acids). We find an identity of 100% over 70 amino acids with PM1024 protein. The location of the transposon in mutant 4G11 corresponds to between positions 3532-3533 of the *Pasteurella multocida* PM70 genome sequence, Genbank accession number AE006143. The transposon disrupts a homologue of the PM70 gene PM1024 or HtpG. The nucleotide sequence of PM1024 is herein identified as SEQ ID NO: 37 and its amino acid sequence as SEQ ID NO: 38.
Other Gram negative bacteria genes and proteins were identified by blasts done with SEQ ID NO: 38.

| Strain | Gene (Genbank ref.) | Protein (Genbank ref.) | % of identity |
|---|---|---|---|
| Actinobacillus actinomycetem comitans | HtpG (U26968) | HtpG (AAC44732) | 88% over 625 amino acids |
| Haemophilus influenzae | HtpG (U32695) | HtpG (AAC21778) | 86% over 625 amino acids |
| Escherichia coli K12 | HtpG (AE000153) acid | HtpG (AAC73575) | 76% over 621 amino |
| Yersinia pestis | HtpG (AJ414155) | HtpG (CAC92355) | 76% over 622 amino acids |
| Salmonella typhi | STY0531 (AL627267) | HtpG (CAD04972) | 76% over 621 amino acids |
| Salmonella typhimurium | HtpG (AE008718) | HtpG (AAL19441) | 75% over 621 amino acids |

HtpG is a heat shock protein.

### Mutant 5D5

The DNA sequence flanking the transposon insertion site is given in SEQ ID NO: 39 (252 mer). The transposon is immediately at the 3' end of this sequence.
The location of the transposon in mutant 5D5 corresponds to between positions 5695-5696 of the *Pasteurella multocida* PM70 genome sequence, Genbank accession number AE006188. The transposon disrupts a homologue of the PM70 gene PM1517 or PIpE). The nucleotide sequence of PM1517 is herein identified as SEQ ID NO: 40 and its amino acid sequence as SEQ ID NO: 41.
PIpE is predicted to be a membrane lipoprotein.

### Mutant 5F11

The DNA sequence flanking the transposon insertion site is given in SEQ ID NO: 42 (546 mer). The transposon is immediately at the 5' end of this sequence.
A stop codon is located at positions 148-150.
The location of the transposon in mutant 5F11 corresponds to between positions 572-573 of the *Pasteurella multocida* PM70 genome, Genbank accession number AE006150. The transposon disrupts a homologue of the PM70 gene PM1087 or NifR3. The nucleotide sequence of PM1087 is herein identified as SEQ ID NO: 43 and its amino acid sequence as SEQ ID NO: 44. One other Pasteurellaceae gene/protein was identified by blasts done with SEQ ID NO: 44. This is *Haemophilus influenzae* HI0979 (Genbank accession numbers U32778 and AAC22639). We find an identity of 78% over 332 amino acids between PM1087 and HI0979.
NifR3 is a nitrogenase regulatory gene.

### Mutant 5G9

The DNA sequence flanking the transposon insertion site is given in SEQ ID NO: 45 (43 mer). The transposon is immediately at the 3' end of this sequence. This sequence has three open reading frames (+2, +3 and -1) encoding potential longer proteins. The ORF described herein is in frame +2.
Four other *Pasteurellaceae* genes and proteins were identified by blasts done with 14 amino acid sequence encoded by SEQ ID NO: 45.

| Strain | Gene (Genbank ref.) | Protein (Genbank ref.) | % of identity |
|---|---|---|---|
| P. multocida P4218 | FcbE (AF302467) | FcbE (AAK17920) | 100% over 14 amino acids |
| P. multocida PM70 | PM0774 (AE006116) | HyaE (AAK02858) | 100% over 14 amino acids |
| P. multocida taxon 747 | HyaE (AF067175) | HyaE (AAC67249) | 100% over 14. amino acids |
| P. multocida P934 | DcbE (AF302465) | DcbE (AAK17906) | 71% over 14 amino acids |

The location of the transposon in mutant 5G9 corresponds to between positions 573-574 of the *Pasteurella multocida* PM70 genome sequence, Genbank accession number AE006116. The transposon disrupts a homologue of the PM70 gene PM0774 or HyaE. The nucleotide sequence of PM0774 is herein identified as SEQ ID NO: 46 and its amino acid sequence as SEQ ID NO: 47. These genes are involved in the capsule synthesis.

### Mutant 6E5

The DNA sequence flanking the transposon insertion site is given in SEQ ID NO: 48 (279 mer). The transposon is immediately at the 3' end of this sequence.
A start codon is located at positions 169-171.
The location of the transposon in mutant 6E5 corresponds to between positions 6673-6674 of the *Pasteurella multocida* PM70 genome, Genbank accession number AE006182. The transposon disrupts a homologue of the PM70 gene PM1459 or pgtB. The nucleotide sequence of PM1459 is herein identified as SEQ ID NO: 49 and its amino acid sequence as SEQ ID NO: 50. PgtB is a phosphoglycerate transport regulatory protein.

### Mutant 6E6

The DNA sequence flanking the transposon insertion site is given in SEQ ID NO: 51 (93 mer). The transposon is immediately at the 3' end of this sequence.
A stop codon is located at positions 12-14.
The location of the transposon in mutant 6E6 corresponds to between positions 9051-9052 of the *Pasteurella multocida* PM70 genome sequence, Genbank accession number AE006096. The transposon disrupts a homologue of the PM70 gene PM0605. The nucleotide sequence of PM0605 is herein identified as SEQ ID NO: 52 and its amino acid sequence as SEQ ID NO: 53.

### Mutant 6F12

The DNA sequence flanking the transposon insertion site is given in SEQ ID NO: 54 (772 mer). The transposon is immediately at the 5' end of this sequence.
A start codon is located at positions 2-4. The location of the transposon in mutant 6F12 corresponds to between positions 5362-5363 of the *Pasteurella multocida* PM70 genome sequence, Genbank accession number AE006192. The transposon disrupts a homologue of the PM70 gene PM1556 or comF gene. The nucleotide sequence of PM1556 is herein identified as SEQ ID NO: 55 and its amino acid sequence as SEQ ID NO: 56.
ComF is the competence protein F.

### Mutant 6G4

The DNA sequence flanking the transposon insertion site is given in SEQ ID NO: 57 (700 mer). The transposon is immediately at the 5' end of this sequence.
The location of the transposon in mutant 6G4 corresponds to between positions 3758-3759 of the *Pasteurella multocida* PM70 genome sequence, Genbank accession number AE006206. The insertion is between the PM1696 and PM1697 genes. The transposon is inserted between the promoter region and the start codon of PM1696.
The start codon of PM1696 is located at positions 26-28 in the SEQ ID NO: 57 sequence.
The nucleotide sequence of PM1696 is herein identified as SEQ ID NO: 58 and its amino acid sequence as SEQ ID NO: 59.
Other Gram negative bacteria genes and proteins were identified by blasts done with SEQ ID NO: 59.

| Strain | Gene (Genbank ref.) | Protein (Genbank ref.) | % of identity |
|---|---|---|---|
| Haemophilus influenzae | HI0266 (U32713) | HI0266 (AAC21932) | 87% over 184 amino acids |
| Salmonella typhi | STY3386 (AL627278) | STY3386 (CAD07732) | 71% over 185 amino acids |
| Salmonella typhimurium | STM3207 (AE008847) | ygiH (AAL22081) | 71% over 185 amino acids |

### Mutant 6H1

The DNA sequence flanking the transposon insertion site is given in SEQ ID NO: 60 (188 mer). The transposon is immediately at the 3' end of this sequence.
The location of the transposon in mutant 6H1 corresponds to between positions 4139-4140 of the *Pasteurella multocida* PM70 genome sequence, Genbank accession number AE006119. The transposon disrupts a homologue of the PM70 gene PM0806 or speF gene. The nucleotide sequence of PM0806 is herein identified as SEQ ID NO: 61 and its amino acid sequence as SEQ ID NO: 62.
Two other Pasteurellaceae and Vibrionaceae genes were identified by blasts done with SEQ ID NO: 62. These genes are *Haemophilus influenzae* speF (Genbank accession numbers U32740 and AAC22248) and *Vibrio cholerae* ornithine decarboxylase (AE004431 and AAF96957). We find an identity of 83% over 719 amino acids between PM0806 and *H*. *influenzae* speF.
SpeF is an ornithine decarboxylase.

### Mutant 6H6

The DNA sequence flanking the transposon insertion site is given in SEQ ID NO: 63 (101 mer). The transposon is immediately at the 3' end of this sequence. This sequence has two open-reading frames (+1 and -1) encoding potential longer proteins. The ORF described herein is in frame +1. The location of the tansposon in mutant 6H6 corresponds to between positions 983-984 of the *Pasteurella multocida* PM70 genome sequence, Genbank accession number AE006155. The transposon disrupts a homologue of the PM70 gene PM1138. The nucleotide sequence of PM1138 is herein identified as SEQ ID NO: 64 and its amino acid sequence as SEQ ID NO: 65.

### Mutant 7A7

The DNA sequence flanking the transposon insertion site is given in SEQ ID NO: 66 (222 mer). The transposon is immediately at the 5' end of this sequence.
The location of the transposon in mutant 7A7 corresponds to between positions 7853-7854 of the *Pasteurella multocida* PM70 genome sequence, Genbank accession number AE006170 (in the intergenic region between PM1321 and PM1322). The transposon is inserted between the terminator region and the stop codon of PM1322.
The stop codon is located at positions 25-27 in the SEQ ID NO: 66 sequence. The nucleotide sequence of PM1322 is herein identified as SEQ ID NO: 67 and its amino acid sequence as SEQ ID NO: 68.

### Mutant 7F8

The DNA sequence flanking the transposon insertion site is given in SEQ ID NO: 69 (55 mer). The transposon is immediately at the 3' end of this sequence. This sequence has three open reading frames (+1, +3 and -3) encoding potential longer proteins. The ORF described herein is in frame +3.
The location of the transposon in mutant 7F8 corresponds to between positions 8292-8293 of the *Pasteurella multocida* PM70 genome sequence, Genbank accession number AE006224. The transposon disrupts a homologue of the PM70 gene PM1866. The nucleotide sequence of PM1866 is herein identified as SEQ ID NO: 70 and its amino acid sequence as SEQ ID NO: 71.

### Mutant 9C8

The DNA sequences flanking the both sides of the transposon insertion site are given in SEQ ID NO: 72 (598 mer, transposon at the 5' end) and SEQ ID NO: 73 (561 mer, transposon at the 5' end).
A stop codon is located at positions 26-28 of SEQ ID NO: 72. Sequences SEQ ID NO: 72 and 73 are combined together and limited to the ORF. The resulting sequence is designated SEQ ID NO: 74 (575 mer).
The location of the transposon in mutant 9C8 corresponds to between positions 2224-2225 or 2210-2211 of the *Pasteurella multocida* PM70 genome sequence, Genbank accession number AE006132, positions deduced from SEQ ID NO: 72 and 73 respectively. Both positions are inside the PM0926 (fimA) gene. The nucleotide sequence of PM0926 is herein identified as SEQ ID NO: 75 and its amino acid sequence as SEQ ID NO: 76.
One other Pasteurellaceae gene/protein was identified by blasts done with SEQ ID NO: 76. This is *Haemophilus influenzae* FimA (Genbank accession numbers AF053125 and AAC08991). We find an identity of 77% over 171 amino acids between PM0926 and H. influenzae FimA.
FimA is an adhesin, a fimbrial protein.

### Mutant 10G11

The DNA sequence flanking the transposon insertion site is given in SEQ ID NO: 77 (70 mer). The transposon is immediately at the 5' end of this sequence. A start codon is located at positions 62-64.
The location of the transposon in mutant 10G11 corresponds to between positions 2938-2939 of the *Pasteurella multocida* PM70 genome sequence, Genbank accession number AE006056. The transposon disrupts a homologue of the PM70 gene PM0220 (rpL31_1). The nucleotide sequence of PM0220 is herein identified as SEQ ID NO: 78 and its amino acid sequence as SEQ ID NO: 79.
RpL31_1 is a 50S ribosomal protein.

### Mutant 11 E8

The DNA sequence flanking the transposon insertion site is given in SEQ ID NO: 80 (506 mer). The transposon is immediately at the 5' end of this sequence.
A start codon is located at positions 195-197 of SEQ ID NO: 80.
The location of the transposon in mutant 11E8 corresponds to between positions 282-283 of the *Pasteurella multocida* PM70 genome sequence, Genbank accession number AE006085. The transposon disrupts a homologue of the PM70 gene PM0488. The nucleotide sequence of PM0488 is herein identified as SEQ ID NO: 81 and its amino acid sequence as SEQ ID NO: 82.

### Mutant 12A1

The DNA sequence flanking the transposon insertion site is given in SEQ ID NO: 83 (243 mer). The transposon is immediately at the 3' end of this sequence.
One other *Pasteurellaceae* gene was identified by blasts done with SEQ ID NO: 83 and its encoded amino acid sequence (81 amino acids). We find an identity of 100% over 81 amino acids with PM0063 protein. The location of the transposon in mutant 12A1 corresponds to between positions 2880-2881 of the *Pasteurella multocida* PM70 genome sequence, Genbank accession number AE006042. The transposon disrupts a homologue of the PM70 gene PM0063 or IepA gene. The nucleotide sequence of PM0063 is herein identified as SEQ ID NO: 84 and its amino acid sequence as SEQ ID NO: 85. Other Gram negative bacteria genes and proteins were identified by blasts done with SEQ ID NO: 85.

| Strain | Gene (Genbank ref.) | Protein (Genbank ref.) | % of identity |
|---|---|---|---|
| Haemophilus influenzae | HI0016 (U32687) | LepA (AAC21694) | 95% over 598 amino acids |
| Yersinia pestis | YPO2716 (AJ414153) | LepA (CAC92955) | 88% over 597 amino acids |
| Escherichia coli K12 | LepA (AE000343) | LepA (AAC75622) | 89% over 597 amino acids |
| Salmonella typhi | STY2829 (AL627275) | LepA (CAD02785) | 89% over 597 amino acids |
| Salmonella typhimurium | LepA (AE008817) | LepA (AAL21477) | 89% over 597 amino acids |
| Vibrio cholerae | VC2463 (AE004316) | LepA 84% (AAF95605) | over 597 amino acids |
| Pseudomonas aeruginosa | PA0767 (AE004511) | LepA (AAG04156) | 75% over 594 amino acids |

LepA is a GTP-binding membrane protein.

### Mutant 12B3

The DNA sequence flanking the transposon insertion site is given in SEQ ID NO: 86 (147 mer). The transposon is immediately at the 3' end of this sequence.
The location of the transposon in mutant 12B3 corresponds to between positions 4028-4029 of the *Pasteurella multocida* PM70 genome sequence, Genbank accession number AE006152. The transposon disrupts a homologue of the PM70 gene PM1112 or deaD gene. The nucleotide sequence of PM1112 is herein identified as SEQ ID NO: 87 and its amino acid sequence as SEQ ID NO: 88.
One other Pasteurellaceae gene/protein was identified by blasts done with SEQ ID NO: 88. This is *Haemophilus influenzae* HI0231 (Genbank accession numbers U32709 and AAC21900). We find an identity of 80% over 605 amino acids between PM1112 and HI0231.
DeaD is an RNA helicase.

### Mutant 13E1

The DNA sequence flanking the transposon insertion site is given in SEQ ID NO: 89 (187 mer). The transposon is immediately at the 3' end of this sequence. This sequence has two open reading frames (+1 and -3) encoding potential longer proteins. The ORF according to the invention is in frame -3.
The location of the transposon in mutant 13E1 corresponds to between positions 2173-2174 of the *Pasteurella multocida* PM70 genome sequence, Genbank accession number AE006138 (PM0989). The nucleotide sequence of PM0989 is herein identified as SEQ ID NO: 90 and its amino acid sequence as SEQ ID NO: 91.
One other Pasteurellaceae gene/protein was identified by blasts done with SEQ ID NO: 91. This is *Haemophilus influenzae* HI0325 (Genbank accession numbers U32717 and AAC21988). We find an identity of 79% over 414 amino acids between PM0989 and HI0325.

### Example 5: Construction of defined deletion mutants

Firstly, the targeted gene plus flanking DNA sequences are amplified by PCR using high fidelity polymerase and cloned into a suitable cloning vector. PCR primers are designed which delete the gene when used in inverse PCR to generate an initial construct. The PCR primers may contain an *Xba*I site to introduce a new restriction site and thus provide a marker for the gene deletion. The deletion construct are then transferred to a suicide vector pCVD442 or equivalent (Donnenberg et al., Infection and Immunity, 1991, 59: 4310-4317) for transfer to the *Pasteurella* chromosome. This construct is introduced into the desired strain by electroporation or conjugation and recombinants containing the plasmid integrated into the chromosome at the homologous site (merodiploids) are selected using the antibiotic resistance marker present on the plasmid. The pCVD442 plasmid requires the Pir protein for replication. This protein is encoded by the pir gene, which is present as a lambda phage lysogen in the donor strain SM10lambda pir, but not in the recipient *P. multocida.* So the pCVD442 plasmid does not replicate in the recipient *P. multocida* strain: antibiotic resistant colonies are therefore only obtained if the plasmid integrates into the chromosome. This suicide vector also contains the *sacB* gene that encodes the enzyme levan sucrase, which is toxic to most Gram-negative bacteria in the presence of sucrose. Sucrose selection can therefore be employed as a counter selection to isolate colonies where a second recombination event has occurred, resulting in loss of the plasmid from the chromosome. This second recombination event can result in two outcomes, re-generation of the wild type allele or generation of a deletion mutant Colonies containing the deletion mutation are identified by colony PCR.

### Example 6: Vaccine and test of efficacy

The attenuated deletion mutants obtained in example 5 were cultured in CDM culture medium (Hu et. al., Infection and Immunity 1986, 804-810) under shaking condition for 24 to 48 hours.
The culture was harvested when the growth stops, which was followed by optical density (OD) or pH measurement.
The bacterial concentration was determined by optical density and when needed the concentration was adjusted to a final concentration of 10⁹ CFU per ml with fresh culture medium.
The efficacy of the vaccine was tested in 3 week-old turkeys by vaccination and challenge.
The turkeys were checked prior to vaccination for the absence of Pasteurella antibodies by ELI SA of blood samples.
A first group of turkeys was vaccinated by injection of 10⁸ CFU in 0.1 ml via ocular route.
A second group remained unvaccinated (control group).
All animals were challenged on D21 or D23 with *P. multocida* P-1059 strain by ocular route (10⁶ CFU in 0.1 ml per animal).
The mortality was observed every day until D35.
A lower mortality was observed in the vaccinated animals compared to the controls.

### SUMMARY PARAGRAPHS

The present invention is defined in the claims and the accompanying description.

For convenience other aspects of the present description are presented herein by way of numbered paragraphs (para.s).
1- A mutant of a Gram negative bacterium, wherein said bacterium has a mutation in a nucleotide sequence which codes originally for a polypeptide having an identity which is equal or more than 70%, 75%, 80%, 85%, 90%, 95%, 96%, 97%, 98%, or 99% with an amino acid sequence coded by a nucleotide sequence selected from the group consisting of nucleotide sequences identified SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90 said mutation resulting in attenuated virulence of the bacterium.
2- The mutant of para. 1, wherein the bacterium is a *Pasteurellaceae.*
3- The mutant of para. 2, wherein the bacterium is chosen among the group of: *Pasteurella multocida, Pasteurella haemolytica, Pasteurella anatipestifer* and *Actinobacillus pleuropneumoniae.*
4- The mutant of para. 3, wherein the bacterium is Pasteurella *multocida.*
5- The mutant of any one of para.s 1 to 4, wherein the mutation is a deletion in the nucleotide sequence, or an insertion into it or replacement of nucleic acids.
6- The mutant of para. 5, wherein the mutation is the deletion of the whole nucleotide sequence.
7- The mutant of para. 5, wherein the insertion is done between: nucleotides 180-181 in SEQ ID NO: 2, 77-78 or 1027-1028 in SEQ ID NO: 6, 416-417 in SEQ ID NO: 9, 389-390 in SEQ ID NO:12, 381-382 in SEQ ID NO: 16, 219-220 in SEQ ID NO: 19, 1353-1354 in SEQ ID NO: 22, 136-137 in SEQ ID NO: 25, 384-385 in SEQ ID NO: 28, 222-223 in SEQ ID NO: 31, 217-218 in SEQ ID NO: 34, 1411-1412 in SEQ ID NO: 37, 943-944 in SEQ ID NO: 40, 855-856 in SEQ ID NO: 43, 369-370 in SEQ ID NO: 46, 111-112 in SEQ ID NO: 49, 443-444 in SEQ ID NO: 52, 4-5 in SEQ ID NO: 55, immediately before the nucleotide 1 in SEQ ID NO: 58, 573-574 in SEQ ID NO: 61, 875-876 in SEQ ID NO: 64, immediately before the nucleotide 1 in SEQ ID NO: 67, 218-219 in SEQ ID NO: 70, 1072-1087 in SEQ ID NO: 75, 64-65 in SEQ ID NO: 78, 282-283 in SEQ ID NO: 81, 1431-1432 in SEQ ID NO: 84, 974-975 in SEQ ID NO: 87, 802-803 in SEQ ID NO: 90.
8- The mutant of any one of para.s 1 to 7, which comprises an heterologous nucleic acid sequence coding for an immunogen from a pathogenic viral, parasitic or bacterial agent, for a therapeutic protein, for an allergen, for a growth factor or for a cytokine.
9- An immunogenic composition comprising an attenuated mutant according to any one of para.s 1 to 8, and a pharmaceutically acceptable diluent, carrier or excipient.
10- The immunogenic composition of para. 9 comprising further an adjuvant.
11- A vaccine comprising an attenuated mutant according to any one of para.s 1 to 8, and a pharmaceutically acceptable diluent, carrier or excipient.
12- The vaccine of para. 11 comprising further an adjuvant.
13- An immunogenic composition comprising a polypeptide having an identity which is equal or more than 70%, 75%. 80%. 85%, 90%, 95%, 96%, 97%, 98%, or 99% with an amino acid sequence coded by a nucleotide sequence selected from the group consisting of nucleotide sequences identified SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90 and a pharmaceutically acceptable diluent, carrier or excipient, and optionnaly an adjuvant.
14- An antibody preparation comprising an antibody specific to a polypeptide having an identity which is equal or more than 70%, 75%, 80%, 85%, 90%, 95%, 96%, 97%, 98%, or 99% with an amino acid sequence coded by a nucleotide sequence selected from the group consisting of nucleotide sequences identified SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90.
15- Diagnostic method for detecting infection by a Gram negative bacterium, using a polypeptide having an identity which is equal or more than 70%, 75%, 80%, 85%, 90%, 95%, 96%, 97%, 98%, or 99% with an amino acid sequence coded by a nucleotide sequence selected from the group consisting of nucleotide sequences identified SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90, or an antibody specific to said polypeptide.
16- Use of an antibody preparation according to para. 14 for the production of a passive immunization composition or a therapeutic composition against Gram negative bacteria.
17- Use of a nucleotide sequence selected from the group consisting of nucleotide sequences identified SEQ ID NO: 2, 6, 9, 12, 16, 19, 22, 25, 28, 31, 34, 37, 40, 43, 46, 49, 52, 55, 58, 61, 64, 67, 70, 75, 78, 81, 84, 87, 90, or a fragment of at least 20 nucleotides, as primers for PCR for detection of Gram negative bacteria in a media.

## Claims

1. A mutant of a Gram negative bacterium, wherein said bacterium has a mutation in a nucleotide sequence which codes for a polypeptide having an identity which is equal or more than 80%, 85%, 90%, 95%, 96%, 97%; 98% or 99% with an amino acid sequence coded by the nucleotide sequence SEQ ID NO: 90 said mutation resulting in attenuated virulence of the bacterium.

2. The mutant of claim 1, wherein the bacterium is a *Pasteurellaceae.*

3. The mutant of claim 2, wherein the bacterium is chosen among the group of: *Pasteurella multocida, Pasteurella haemolytica, Pasteurella anatipestifer* and *Actinobacillus pleuropneumoniae.*

4. The mutant of claim 3, wherein the bacterium is *Pasteurella multocida,*

5. The mutant of any one of claims I to 4, wherein the mutation is a deletion in the nucleotide sequence, or an insertion into it or replacement of nucleic acids.

6. The mutant of claim 5, wherein the mutation is the deletion of the whole nucleotide sequence.

7. The mutant of claim 5, wherein the insertion is done between nucleotides 802-803 in SEQ ID NO: 90.

8. The mutant of any one of claims 1 to 7, which comprises an heterologous nucleic acid sequence coding for an immunogens from a pathogenic viral, parasitic or bacterial agent, for a therapeutic protein, for an allergen, for a growth factor or for a cytokine.

9. An immunogenic composition comprising an attenuated mutant according to any one of claims 1 to 8, and a pharmaceutically acceptable diluent, carrier or excipient.

10. The immunogenic composition of claim 9 comprising further an adjuvant.

11. A vaccine comprising an attenuated mutant according to any one of claims 1 to 8, and a pharmaceutically acceptable diluent, carrier or excipient.

12. The vaccine of claim 11 comprising further an adjuvant.

13. Use of a mutant of a Gram negative bacterium according to any one of claims 1 to 8 in the production of a live attenuated immunogenic composition or preparation of a live attenuated vaccine composition.

14. A Gram negative bacterium according to any one of claims 1 to 8, an immunogenic composition according to claim 9 or 10, or a vaccine according to claim 11 or 12, for use in the immunisation against or prevention of bacterial infection in an animal.

15. Use of a mutant of a Gram negative bacterium according to any one of claims 1 to 8, an immunogenic composition according to claim 9 or 10, or a vaccine according to claim 11 or 12, in the preparation of a medicament for the immunisation against or prevention of bacterial infection in an animal.

## Patentansprüche

1. Mutante eines gramnegativen Bakteriums, wobei das Bakterium eine Mutation in einer Nucleotidsequenz hat, die ein Polypeptid codiert, das mit einer Aminosäuresequenz, die von der Nucleotidsequenz SEQ ID NO:90 codiert wird, eine Identität hat, die gleich oder höher als 80%, 85%, 90%, 95%, 96%, 97%, 98% oder 99% ist, wobei die Mutation zu einer abgeschwächten Virulenz des Bakteriums führt.

2. Mutante nach Anspruch 1, wobei das Bakterium *Pasteurellaceae* ist.

3. Mutante nach Anspruch 2, wobei das Bakterium ausgewählt ist aus der Gruppe bestehend aus: *Pasteurella multocida, Pasteurella haemolytica, Pasteurella anatipestifer* und *Actinobacillus pleuropneumoniae.*

4. Mutante nach Anspruch 3, wobei das Bakterium *Pasteurella multocida* ist.

5. Mutante nach einem der Ansprüche 1 bis 4, wobei die Mutation eine Deletion in der Nucleotidsequenz ist oder eine Insertion darin oder Ersatz von Nucleinsäuren.

6. Mutante nach Anspruch 5, wobei die Mutation die Deletion der gesamten Nucleotidsequenz ist.

7. Mutante nach Anspruch 5, wobei die Insertion zwischen Nucleotide 802-803 in SEQ ID NO:90 erfolgt ist.

8. Mutante nach einem der Ansprüche 1 bis 7, die eine heterologe Nucleinsäuresequenz umfasst, die ein Immunogen von einem pathogenen viralen, parasitären oder bakteriellen Agens, ein therapeutisches Protein, ein Allergen, einen Wachstumsfaktor oder ein Cytokin codiert.

9. Immunogene Zusammensetzung, umfassend eine abgeschwächte Mutante nach einem der Ansprüche 1 bis 8 und ein pharmazeutisch verträgliches Verdünnungsmittel oder einen pharmazeutisch verträglichen Träger oder Exzipienten.

10. Immunogene Zusammensetzung nach Anspruch 9, des Weiteren ein Adjuvans umfassend.

11. Impfstoff, umfassend eine abgeschwächte Mutante nach einem der Ansprüche 1 bis 8 und ein pharmazeutisch verträgliches Verdünnungsmittel oder einen pharmazeutisch verträglichen Träger oder Exzipienten.

12. Impfstoff nach Anspruch 11, des Weiteren ein Adjuvans umfassend.

13. Verwendung einer Mutante eines gramnegativen Bakteriums nach einem der Ansprüche 1 bis 8 in der Herstellung einer lebenden attenuierten immunogenen Zusammensetzung oder der Zubereitung einer attenuierten Lebendimpfstoff-Zusammensetzung.

14. Gramnegatives Bakterium nach einem der Ansprüche 1 bis 8, immunogene Zusammensetzung nach Anspruch 9 oder 10 oder Impfstoff nach Anspruch 11 oder 12 zur Verwendung in der Immunisierung gegen oder der Prävention einer bakteriellen Infektion bei einem Tier.

15. Verwendung einer Mutante eines gramnegativen Bakteriums nach einem der Ansprüche 1 bis 8, einer immunogenen Zusammensetzung nach Anspruch 9 oder 10 oder eines Impfstoffs nach Anspruch 11 oder 12 in der Herstellung eines Medikaments für die Immunisierung gegen oder die Prävention einer bakteriellen Infektion bei einem Tier.

## Revendications

1. Mutant d'une bactérie à Gram négatif, dans lequel ladite bactérie comporte une mutation dans une séquence de nucléotides qui code pour un polypeptide ayant une identité qui est égale ou supérieure à 80 %, 85 %, 90 %, 95 %, 96 %, 97 %, 98 % ou 99 % avec une séquences d'acides aminés codée par la séquence de nucléotides SEQ ID NO: 90, ladite mutation entraînant une virulence atténuée de la bactérie.

2. Mutant selon la revendication 1, dans lequel la bactérie est une *Pasteurellaceae.*

3. Mutant selon la revendication 2, dans lequel la bactérie est choisie dans le groupe consistant en : *Pasteurella multocida, Pasteurella haemolytica, Pasteurella anatipestifer* et *Actinobacillus pleuropneumoniae.*

4. Mutant selon la revendication 3, dans lequel la bactérie est *Pasteurella multocida.*

5. Mutant selon l'une quelconque des revendications 1 à 4, dans lequel la mutation est une délétion dans la séquence de nucléotides, ou une insertion dans celle-ci ou un remplacement d'acides nucléiques.

6. Mutant selon la revendication 5, dans lequel la mutation est la délétion de l'intégralité de la séquence de nucléotides.

7. Mutant selon la revendication 5, dans lequel l'insertion est réalisée entre les nucléotides 802-803 dans SEQ ID NO: 90.

8. Mutant selon l'une quelconque des revendications 1 à 7, qui comprend une séquence d'acide nucléique hétérologue qui code pour un immunogène issu d'un agent pathogène viral, parasitaire ou bactérien, pour une protéine thérapeutique, pour un allergène, pour un facteur de croissance ou pour une cytokine.

9. Composition immunogène comprenant un mutant atténué selon l'une quelconque des revendications 1 à 8, et un diluant, véhicule ou excipient pharmaceutiquement acceptable.

10. Composition immunogène selon la revendication 9, comprenant en outre un adjuvant.

11. Vaccin comprenant un mutant atténué selon l'une quelconque des revendications 1 à 8, et un diluant, véhicule ou excipient pharmaceutiquement acceptable.

12. Vaccin selon la revendication 11, comprenant en outre un adjuvant.

13. Utilisation d'un mutant d'une bactérie à Gram négatif selon l'une quelconque des revendications 1 à 8, dans la production d'une composition immunogène vivante atténuée ou dans la préparation d'une composition de vaccin vivant atténué.

14. Bactérie à Gram négatif selon l'une quelconque des revendications 1 à 8, composition immunogène selon la revendication 9 ou 10, ou vaccin selon la revendication 11 ou 12, pour une utilisation dans l'immunisation contre ou la prévention d'une infection bactérienne chez un animal.

15. Utilisation d'un mutant d'une bactérie à Gram négatif selon l'une quelconque des revendications 1 à 8, d'une composition immunogène selon la revendication 9 ou 10, ou d'un vaccin selon la revendication 11 ou 12, dans la préparation d'un médicament pour l'immunisation contre ou la prévention d'une infection bactérienne chez un animal.


### SEQUENCE LISTING

<110> MERIAL
<120> Attenuated gram negative bacteria
<130> XXXX
<160> 91
<170> PatentIn version 3.1
<210> 1
   <211> 775
   <212> DNA
   <213> Pasteurella multocida
<220>
   <221> misc_feature
   <222> (579)..(579)
   <223> A or C or G or T
<220>
   <221> misc_feature
   <222> (580)..(580)
   <223> A or C or G or T
<220>
   <221> misc_feature
   <222> (598)..(598)
   <223> A or C or G or T
<220>
   <221> misc_feature
   <222> (599)..(599)
   <223> A or C or G or T
<220>
   <221> misc_feature
   <222> (616)..(616)
   <223> A or C or G or T
<220>
   <221> misc_feature
   <222> (617)..(617)
   <223> A or C or G or T
<220>
   <221> misc_feature
   <222> (636)..(636)
   <223> A or C or G or T
<220>
   <221> misc_feature
   <222> (637)..(637)
   <223> A or C or G or T
<220>
   <221> misc_feature
   <222> (667)..(667)
   <223> A or C or G or T
<220>
   <221> misc_feature
   <222> (668)..(668)
   <223> A or C or G or T
<220>
   <221> misc_feature
   <222> (697)..(697)
   <223> A or C or G or T
<220>
   <221> misc_feature
   <222> (698)..(698)
   <223> A or C or G or T
<220>
   <221> misc_feature
   <222> (702)..(702)
   <223> A or C or G or T
<220>
   <221> misc_feature

   <222> (703)..(703) <223> A or C or G or T
<220>
   <221> misc_feature
   <222> (707)..(707)
   <223> A or C or G or T
<22C>
   <221> misc_feature
   <222> (706) .. (706)
   <223> A or C or G or T
<220>
   <221> misc_feature

   <222> (714)..(715)
   <223> A or C or G or T
<220>

   <221> misc_feature
   <222> (715) .. (716)
   <223> A or C or G or T
<220>

   <221> misc_feature
   <222> (717)..(718)
   <223> A or C or 3 or T.
<220>
   <221> misc_feature
   <222> (718)..(719)
   <223> A or C or G or T
<220>
   <221> misc_feature
   <222> (721)..(721)
   <223> A or C or G or T
<220>
   <221> misc_feature
   <222> (722)..(722)
   <223> A or C or G or T
<220>
   <221> misc_feature
   <222> (742)..(744)
   <223> A or C or G or T
<220>
   <221> misc_feature
   <222> (743)..(745)
   <223> A or C or G or T
<220>
   <221> misc_feature
   <222> (746)..(749)
   <223> A or C or G or T
<220>
   <221> misc_feature
   <222> (747)..(750)
   <223> A or C or G or T
<220>
   <221> misc_feature
   <222> (755)..(756)
   <223> A or C or G or T
<220>
   <221> misc_feature
   <222> (756)..(757)
   <223> A or C or G or T
<220>
   <221> misc_feature
   <222> (767)..(768)
   <223> A or C or G or T
<220>
   <221> misc_feature
   <222> (766)..(767)
   <223> A or C or G or T
<400> 1
   gtgctttata tccccattct aaaatacatg ttctctcctt tttccatgtg acaaatggag      60
   agaacatttt caagcgttgg gtaaaaaagc cgcttaaata aggaattttt aacatccctt    120
   tagaaaaaat aagaaactct tgatacatat ttaatctaat atagtcatat aaagttgaca    180
   tatcatatat taaacatgac tagttaatca ttaaatatta aacaacctca acttaataaa    240
   acaaataata aacaaacaag gtaaaaaaca aactaatact gagcaaataa aaaacggatt    300
   aatataataa cgatatatca acctctaaaa cagaccaaaa ataaatcaca cgagacaaaa    360
   gaacaattat aatccaaata ttaattaata aataaacacc tagcgcaacg aataatcaaa    420
   caaaatcaca tttagattta tttaaattaa aaatatagat tatattttaa atataatgct    480
   agaattcggc accaaaattt ttctccagct gtaaattaga gataaagata tgaaaaaggt    540
   tattatcatg ggacataaac agtctaacta tcaagatgtn gaaaaggttt ttcaatgtna    600
   tgggatgaat ccccgcntcc atcaaaacgt gaaaaangtc cccatcgaac ttttgctgag    660
   tgaggatnag atagggcaaa tctgcaaatt catccacngc cgncgcngac tcatnnanng    720
   cnaatcgcca tagtagttat acnnncnnnn gtttanngtt gaccgcnnag gcgag          775
<210> 2
   <211> 2091
   <212> DNA
   <213> Pasteurella multocida
<400> 2
   atgtcaattt tatatgacta tattagatta aatatgtatc aagagtttct tattttttct     60
   aaagggatgt taaaaattcc ttatttaagc ggttttttta cccaacgctt gaaaatgttc    120
   tctccatttg tcacatggaa aaaggagaga acatgtattt tagaatgggg atataaagca    180
   tcatcaaaga aagcgaggca ttttgcacaa caacatgatt taccttatgc gacgatagaa    240
   gatggttttt tacgttctat tggactgggt gtggatgggt atccaccttt ctcattagtg    300
   tacgatgata ttggtattta ttatgatatc aatcagcctt ctcgtttaga aaacttaatt    360
   ctttctcaag atgtattact tcaggaaaaa gtcgatcaag ttgaatatgc aattgaatta    420
   atttgtacac ataacctttc caaatataac cacgctattg atacaccttt acagaatacg    480
   aagaggccga ttgtcttagt cgttgatcaa acatatggcg atatggcagt tactttcggg    540
   aatgccgagc agagtgattt tctacatatg ttagaacgtg cgattattga aaatccaaca    600
   gcagaaatct ggttaaaaac ccatcctgat gtaatgtgtg gtaaaaaaca aggctattta    660
   acggaatatc agcaatttcc aagagtaaaa gtattatcag aagattttaa ttctatctca    720
   ctactaaagc atgttgataa agtttattgc gtgacatctc acactgggtt tgaggccctt    780
   ttgttaggaa aaactgtcgt gacttttggg gctgcctggt tttctggttg gggattgacg    840
   gatgatcggc atgcgtatat tcgtcagcta aaacagagta agagaagagc gaagcgttca    900
   ttgttgcagt tattctatgc tgcttatttc caatattgtc gttatattaa tcctaatacg    960
   ggtaaatcag gcacattgtt tgatgtcatt gattatctga ttcaagcaaa aaaagtaacc   1020
   aatcagttag ctggtgatat ttattgtgtg ggtatgcgct tttggaaacg taaagtggtt   1080
   caaccctttt ttcaatttcc acgctgtcgt ttacattttg tgctgaatgt gcatgagcta   1140
   aagcgatgta ttcacgagaa atctcaggct aaaatagtgg tgtggggaca ttcacacatt   1200
   gaagtggttg aatatgccaa gcaacagcaa cttcctcttt tgagaatgga agatggtttt   1260
   ttacgttcag ttgggttagg gtctaattta acgccaccga tatcattagt tttagatgac   1320
   gttggcattt attttgacgc ccaatctcgt tcccgattag aggatattct acagcatcaa   1380
   tcctttactc taaaggattt acagcgcgca gaaacgttaa agaaaacact gattgagcaa   1440
   catattggta agtataatgt gggacataca cacttatgcc taacacacat cagacaaaat   1500
   aaacttttag ttgtgggaca agtggaaaat gatgcttcaa ttcaatatgg ttcaccgcat   1560
   attcgtacga atgcagagtt attatgtacg gtcagaaaaa ataatcccca agcctatatt   1620
   atttataaac ctcatcctga tgtggttgca ggcaatcgta aaaacacaga tcgtctagat   1680
   gattatcgac agtatgctga tttcgtggtt gagaaagcca atatattgga ttgcattaac   1740
   caagtggatg aagtgcatac gatgacctct ttagcggggt ttgaagcgtt actgcgcgag   1800
   aaaaaagtac attgttatgg cttgcctttt tattctaact gggggctaac agtggatcat   1860
   ctttctctaa accgaagaag tcggaagtta agtcttttag aattaattgc tggcgtgctg   1920
   atttattacc cacaatatat tgacccaaaa acaaaaacaa tgatcgatgt gcagcgagcg   1980
   gttgatattc tgatcgagca acgtcgaaaa ataaaaaata ataaattaca tacaaattat   2040
   tttatgaaca tttttatgaa attaaaaaat gtttattctg ttttgaggta g            2091
<210> 3
   <211> 696
   <212> PRT
   <213> Pasteurella multocida
<400> 3
   Met Ser Ile Leu Tyr Asp Tyr Ile Arg Leu Asn Met Tyr Gln Glu Phe
   1               5                   10                  15
   Leu Ile Phe Ser Lys Gly Met Leu Lys Ile Pro Tyr Leu Ser Gly Phe
               20                  25                  30
   Phe Thr Gln Arg Leu Lys Met Phe Ser Pro Phe Val Thr Trp Lys Lys
           35                  40                  45
   Glu Arg Thr Cys Ile Leu Glu Trp Gly Tyr Lys Ala Ser Ser Lys Lys
       50                  55                  60
   Ala Arg His Phe Ala Gln Gln His Asp Leu Pro Tyr Ala Thr Ile Glu
   65                  70                  75                  80
   Asp Gly Phe Leu Arg Ser Ile Gly Leu Gly Val Asp Gly Tyr Pro Pro
                   85                  90                  95
   Phe Ser Leu Val Tyr Asp Asp Ile Gly Ile Tyr Tyr Asp Ile Asn Gln
               100                 105                 110
   Pro Ser Arg Leu Glu Asn Leu Ile Leu Ser Gln Asp Val Leu Leu Gln
           115                 120                 125
   Glu Lys Val Asp Gln Val Glu Tyr Ala Ile Glu Leu Ile Cys Thr His
       130                 135                 140
   Asn Leu Ser Lys Tyr Asn His Ala Ile Asp Thr Pro Leu Gln Asn Thr
   145                 150                 155                 160
   Lys Arg Pro Ile Val Leu Val Val Asp Gln Thr Tyr Gly Asp Met Ala
                   165                 170                 175
   Val Thr Phe Gly Asn Ala Glu Gln Ser Asp Phe Leu His Met Leu Glu
               180                 185                 190
   Arg Ala Ile Ile Glu Asn Pro Thr Ala Glu Ile Trp Leu Lys Thr His
           195                 200                 205
   Pro Asp Val Met Cys Gly Lys Lys Gln Gly Tyr Leu Thr Glu Tyr Gln
       210                 215                 220
   Gln Phe Pro Arg Val Lys Val Leu Ser Glu Asp Phe Asn Ser Ile Ser
   225                 230                 235                 240
   Leu Leu Lys His Val Asp Lys Val Tyr Cys Val Thr Ser His Thr Gly
                   245                 250                 255
   Phe Glu Ala Leu Leu Leu Gly Lys Thr Val Val Thr Phe Gly Ala Ala
               260                 265                 270
   Trp Phe Ser Gly Trp Gly Leu Thr Asp Asp Arg His Ala Tyr Ile Arg
           275                 280                 285
   Gln Leu Lys Gln Ser Lys Arg Arg Ala Lys Arg Ser Leu Leu Gln Leu
       290                 295                 300
   Phe Tyr Ala Ala Tyr Phe Gln Tyr Cys Arg Tyr Ile Asn Pro Asn Thr
   305                 310                 315                 320
   Gly Lys Ser Gly Thr Leu Phe Asp Val Ile Asp Tyr Leu Ile Gln Ala
                   325                 330                 335
   Lys Lys Val Thr Asn Gln Leu Ala Gly Asp Ile Tyr Cys Val Gly Met
               340                 345                 350
   Arg Phe Trp Lys Arg Lys Val Val Gln Pro Phe Phe Gln Phe Pro Arg
           355                 360                 365
   Cys Arg Leu His Phe Val Leu Asn Val His Glu Leu Lys Arg Cys Ile
       370                 375                 380
   His Glu Lys Ser Gln Ala Lys Ile Val Val Trp Gly His Ser His Ile
   385                 390                 395                 400
   Glu Val Val Glu Tyr Ala Lys Gln Gln Gln Leu Pro Leu Leu Arg Met
                   405                     410                 415
   Glu Asp Gly Phe Leu Arg Ser Val Gly Leu Gly Ser Asn Leu Thr Pro
               420                     425                 430
   Pro Ile Ser Leu Val Leu Asp Asp Val Gly Ile Tyr Phe Asp Ala Gln
           435                     440                 445
   Ser Arg Ser Arg Leu Glu Asp Ile Leu Gln His Gln Ser Phe Thr Leu
       450                     455                 460
   Lys Asp Leu Gln Arg Ala Glu Thr Leu Lys Lys Thr Leu Ile Glu Gln
   465                     470                 475                 480
   His Ile Gly Lys Tyr Asn Val Gly His Thr His Leu Cys Leu Thr His
                   485                     490                 495
   Ile Arg Gln Asn Lys Leu Leu Val Val Gly Gln Val Glu Asn Asp Ala
               500                     505                 510
   Ser Ile Gln Tyr Gly Ser Pro His Ile Arg Thr Asn Ala Glu Leu Leu
           515                     520                 525
   Cys Thr Val Arg Lys Asn Asn Pro Gln Ala Tyr Ile Ile Tyr Lys Pro
       530                     535                 540
   His Pro Asp Val Val Ala Gly Asn Arg Lys Asn Thr Asp Arg Leu Asp
   545                     550                 555                 560
   Asp Tyr Arg Gln Tyr Ala Asp Phe Val Val Glu Lys Ala Asn Ile Leu
                   565                     570                 575
   Asp Cys Ile Asn Gln Val Asp Glu Val His Thr Met Thr Ser Leu Ala
               580                     585                 590
   Gly Phe Glu Ala Leu Leu Arg Glu Lys Lys Val His Cys Tyr Gly Leu
           595                     600                 605
   Pro Phe Tyr Ser Asn Trp Gly Leu Thr Val Asp His Leu Ser Leu Asn
       610                     615                 620
   Arg Arg Ser Arg Lys Leu Ser Leu Leu Glu Leu Ile Ala Gly Val Leu
   625                     630                 635                 640
   Ile Tyr Tyr Pro Gln Tyr Ile Asp Pro Lys Thr Lys Thr Met Ile Asp
                   645                     650                 655
   Val Gln Arg Ala Val Asp Ile Leu Ile Glu Gln Arg Arg Lys Ile Lys
               660                     665                 670
   Asn Asn Lys Leu His Thr Asn Tyr Phe Met Asn Ile Phe Met Lys Leu
           675                     680                 685
   Lys Asn Val Tyr Ser Val Leu Arg
       690                     695
<210> 4
   <211> 226
   <212> DNA
   <213> Pasteurella multocida
<400> 4
   agcaaaagtt cgattactac cagagacagt aagaagtgcg gttaaaatac ctaacaagaa     60
   gagaaataac acaatattaa tgttatttga attaagtgcg ttatcactgt atgccaatga    120
   aataatatta tttttgagat acattagcgt atgcgaaata ttaaaatctg caagcattaa    180
   agcacctaca ataataccaa cactcaatga taaaataaca cggcgc                   226
<210> 5
   <211> 87
   <212> DNA
   <213> Pasteurella multocida
<400> 5
   cgacactatg cattcttatt gatcgtcaag tgagtttggc ggaatacggt aaatcctgga     60
   ttttaggcgt gaagtcaatg ctcggtg                                         87
<210> 6
   <211> 1524
   <212> DNA
   <213> Pasteurella multocida
<400> 6
   atggaactta ttgattattc gacgtctatt tggtctgtcg taccccctat tttagcatta     60
   ttattagcca ttgggacacg ccgtgttatt ttatcattga gtgttggtat tattgtaggc    120
   gctttaatgc ttgcagattt taatatttcg catacgctaa tgtatctcaa aaataatatt    180
   atttcattgg catacagtga taacacactt aattcaaata acattaatat tgtgttattt    240
   ctcttcttgt taggtatttt aaccgcactt cttactgtct caggcagtaa tcgagccttt    300
   gcagaatggg cacaaaaacg aattaaagat agaaaagggg ctaaattatt agccgcatcg    360
   ctcgtgtttg tgactttcat tgacgattat tttcatagct tagcggtggg agcgattgcc    420
   agcccagtta cagataaatt taaagtttca cgcccaaaac ttgcctatat tcttgattca    480
   accgctgcgc caatgtgtgt gttgatgcct gtatcaagtt ggggcgccta tattattaca    540
   cttattgcag gacttcttgc gacttattcg atcaccgagt attcccctat cggtgcattt    600
   atgacaatga gtgcaatgaa cttttatgct atttttctta ttttaatggt gttctttgta    660
   tcttattatt cgtttgatat tggttcaatg gcgcgtcacg aaagaatggc cctagcgcgt    720
   gtaacagaag aagaaaaact ggaaagtagt aataaagggc atgttctcta tttaatttta    780
   ccgattactg tcctgatttt agcaaccgtt ggtatgatga tgtacacggg ctatgaagca    840
   ttagcggcgg atggaaaacc ttttgatgtg ttaggcgcgt ttgagaatac tacagtaggg    900
   atttcattgg ttgtgggggg attaagtgcg gtcttgattt cgacactatg cattcttatt    960
   gatcgtcaag tgagtttggc tgaatacggt aaatcctgga ttttaggcgt gaagtcaatg   1020
   ctcggtgcgg tattgatttt attgtttgct tggactatta ataccatcgt tggagatgtc   1080
   aaaacaggga tttatttatc ttcattagta tcggatagtt taccgattgc tttgttgcct   1140
   gcgttattat ttattttaac tggaatcatg gcattctcga caggaacaag ctggggaact   1200
   tttgggatta tgttaccgat cgcggcagcg attgcagcga atactgcacc agaattgatg   1260
   ttaccttgtt tatccgcagt catggctggt gcagtttgtg gtgatcattg ctcaccgatt   1320
   tcggatacca cgattttatc ttctaccggg gcaaaatgta atcatatcga ccatgtaaca   1380
   acacagttac cttatgcgat gttaattgcg acagcgtcta ttgctggcta tttagtacta   1440
   gggttcagcc agtcaggcat actgggtttt gtgacaacgg gtgtggtttt atcagtactt   1500
   gtttttatat ttagaaaaaa ataa                                          1524
<210> 7
   <211> 507
   <212> PRT
   <213> Pasteurella multocida
<400> 7
   Met Glu Leu Ile Asp Tyr Ser Thr Ser Ile Trp Ser Val Val Pro Pro
   1               5                   10                  15
   Ile Leu Ala Leu Leu Leu Ala Ile Gly Thr Arg Arg Val Ile Leu Ser
               20                  25                  30
   Leu Ser Val Gly Ile Ile Val Gly Ala Leu Met Leu Ala Asp Phe Asn
           35                  40                  45
   Ile Ser His Thr Leu Met Tyr Leu Lys Asn Asn Ile Ile Ser Leu Ala
       50                  55                  60
   Tyr Ser Asp Asn Thr Leu Asn Ser Asn Asn Ile Asn Ile Val Leu Phe
   65                  70                  75                  80
   Leu Phe Leu Leu Gly Ile Leu Thr Ala Leu Leu Thr Val Ser Gly Ser
                   85                  90                  95
   Asn Arg Ala Phe Ala Glu Trp Ala Gln Lys Arg Ile Lys Asp Arg Lys
               100                 105                 110
   Gly Ala Lys Leu Leu Ala Ala Ser Leu Val Phe Val Thr Phe Ile Asp
           115                 120                 125
   Asp Tyr Phe His Ser Leu Ala Val Gly Ala Ile Ala Ser Pro Val Thr
       130                 135                 140
   Asp Lys Phe Lys Val Ser Arg Pro Lys Leu Ala Tyr Ile Leu Asp Ser
   145                 150                 155                 160
   Thr Ala Ala Pro Met Cys Val Leu Met Pro Val Ser Ser Trp Gly Ala
                   165                 170                 175
   Tyr Ile Ile Thr Leu Ile Ala Gly Leu Leu Ala Thr Tyr Ser Ile Thr
               180                 185                 190
   Glu Tyr Ser Pro Ile Gly Ala Phe Met Thr Met Ser Ala Met Asn Phe
           195                 200                 205
   Tyr Ala Ile Phe Ser Ile Leu Met Val Phe Phe Val Ser Tyr Tyr Ser
       210                 215                 220
   Phe Asp Ile Gly Ser Met Ala Arg His Glu Arg Met Ala Leu Ala Arg
   225                 230                 235                 240
   Val Thr Glu Glu Glu Lys Leu Glu Ser Ser Asn Lys Gly His Val Leu
                   245                 250                 255
   Tyr Leu Ile Leu Pro Ile Thr Val Leu Ile Leu Ala Thr Val Gly Met
               260                 265                 270
   Met Met Tyr Thr Gly Tyr Glu Ala Leu Ala Ala Asp Gly Lys Pro Phe
           275                 280                 285
   Asp Val Leu Gly Ala Phe Glu Asn Thr Thr Val Gly Ile Ser Leu Val
       290                 295                 300
   Val Gly Gly Leu Ser Ala Val Leu Ile Ser Thr Leu Cys Ile Leu Ile
   305                 310                 315                 320
   Asp Arg Gln Val Ser Leu Ala Glu Tyr Gly Lys Ser Trp Ile Leu Gly
                   325                 330                 335
   Val Lys Ser Met Leu Gly Ala Val Leu Ile Leu Leu Phe Ala Trp Thr
               340                 345                 350
   Ile Asn Thr Ile Val Gly Asp Val Lys Thr Gly Ile Tyr Leu Ser Ser
           355                 360                 365
   Leu Val Ser Asp Ser Leu Pro Ile Ala Leu Leu Pro Ala Leu Leu Phe
       370                 375                 380
   Ile Leu Thr Gly Ile Met Ala Phe Ser Thr Gly Thr Ser Trp Gly Thr
   385                 390                 395                 400
   Phe Gly Ile Met Leu Pro Ile Ala Ala Ala Ile Ala Ala Asn Thr Ala
                   405                 410                 415
   Pro Glu Leu Met Leu Pro Cys Leu Ser Ala Val Met Ala Gly Ala Val
               420                 425                 430
   Cys Gly Asp His Cys Ser Pro Ile Ser Asp Thr Thr Ile Leu Ser Ser
           435                 440                 445
   Thr Gly Ala Lys Cys Asn His Ile Asp His Val Thr Thr Gln Leu Pro
       450                 455                 460
   Tyr Ala Met Leu Ile Ala Thr Ala Ser Ile Ala Gly Tyr Leu Val Leu
   465                 470                 475                 480
   Gly Phe Ser Gln Ser Gly Ile Leu Gly Phe Val Thr Thr Gly Val Val
                   485                 490                 495
   Leu Ser Val Leu Val Phe Ile Phe Arg Lys Lys
               500                 505
<210> 8
   <211> 78
   <212> DNA
   <213> Pasteurella multocida
<400> 8
   gggaaaagca gcaaatatca aaaatactgt tttagtgaaa acaggaaaac cgattacagc      60
   agaaggcgta cacccacc                                                    78
<210> 9
   <211> 555
   <212> DNA
   <213> Pasteurella multocida
<400> 9
   atgaaaaaag caattttttt agatcgagat ggcacattaa atattgatca tggctatgtt      60
   catgaaattg atcagtttca atttattgac ggtagcattg aagcgttaca acaactgaaa     120
   gcgatgggct atttattggt acttgtaaca aatcagtcag gtattgcgcg tggatatttt     180
   agcgaagatc aatttttaca gctgacagaa tggatggatt ggtctcttgc agatcgtgga     240
   gtggatttag atggcatcta ttattgccca caccacacag aaggaaaagg tgagtattgc     300
   caagactgcg attgccgtaa gccaaaacct ggtatgttac tgcaggcaat taaggaactt     360
   aatatagatc ccaatacctc ttttatggtg ggtgataaag tggaagatat gttagcaggt     420
   aaaggtgcca aaattaaaaa tactgtttta gtgaaaacag gcaagcctat tacggaggat     480
   ggcaaaaaac aggcaaacta tgtattagag tccattgcgg atctactaaa actgataaaa     540
   ggattaaaaa gttaa                                                      555
<210> 10
   <211> 184
   <212> PRT
   <213> Pasteurella multocida
<400> 10
   Met Lys Lys Ala Ile Phe Leu Asp Arg Asp Gly Thr Leu Asn Ile Asp
   1               5                   10                  15
   His Gly Tyr Val His Glu Ile Asp Gln Phe Gln Phe Ile Asp Gly Ser
               20                  25                  30
   Ile Glu Ala Leu Gln Gln Leu Lys Ala Met Gly Tyr Leu Leu Val Leu
           35                  40                  45
   Val Thr Asn Gln Ser Gly Ile Ala Arg Gly Tyr Phe Ser Glu Asp Gln
       50                  55                  60
   Phe Leu Gln Leu Thr Glu Trp Met Asp Trp Ser Leu Ala Asp Arg Gly
   65                  70                  75                  80
   Val Asp Leu Asp Gly Ile Tyr Tyr Cys Pro His His Thr Glu Gly Lys
                   85                  90                  95
   Gly Glu Tyr Cys Gln Asp Cys Asp Cys Arg Lys Pro Lys Pro Gly Met
               100                 105                 110
   Leu Leu Gln Ala Ile Lys Glu Leu Asn Ile Asp Pro Asn Thr Ser Phe
           115                 120                 125
   Met Val Gly Asp Lys Val Glu Asp Met Leu Ala Gly Lys Gly Ala Lys
       130                 135                 140
   Ile Lys Asn Thr Val Leu Val Lys Thr Gly Lys Pro Ile Thr Glu Asp
   145                 150                 155                 160
   Gly Lys Lys Gln Ala Asn Tyr Val Leu Glu Ser Ile Ala Asp Leu Pro
                   165                 170                 175
   Lys Leu Ile Lys Gly Leu Lys Ser
               180
<210> 11
   <211> 467
   <212> DNA
   <213> Pasteurella multocida
<400> 11 .
   gagctctgca tttagtttaa gtggtgattt cttatttgac ttcaataaag attcattaac     60
   agcaaaaggt aaagaagttg ttgacagcgt tgcaacacaa ttaaaagcct ctgatgcaaa    120
   agaagtgaaa gtcgcaggct ttactgaccg tttaggttca gaagcgtata acttaaaact    180
   ttctcaacgt cgtgcagatc gtgttaaagc gcgtttaatt gagcaaggtg ttgccgcaaa    240
   tattcatgct gtaggctatg gtaaagcaca acaagtgaaa gcttgtgatg atgtacaagg    300
   tgcagcatta agagactgtt tacgtcctaa ccgtcgtgtt gaaattaccg cttctggtac    360
   tgtgttaaaa caaggttcac aaggtatgga agcagggaca acaggaccag caccacttta    420
   tagaaaataa tttttctcaa tgaaatagaa gggcgcttta atagcgc                  467
<210> 12
   <211> 819
   <212> DNA
   <213> Pasteurella multocida
<400> 12
   atgaaattat ctcgcgtttt attaacagtt gttgctgcga cgacattggc tgcctgcggt     60
   aatttaagta aagttactcc agaaggtaca tctgacaatt tagtgtggcc aaaaattgat    120
   gaatcagtct ttaatcatga tggtagccaa tttggttctt ggccaaactg ggataacgta    180
   cgcatggttg agcgtggtat gaataaagac caactttata atttgttagg tcgtccacac    240
   ttctctgaag gcttatacgg tgtgcgtgaa tgggactatg tgtttaacta tcgtgagaat    300
   ggtgtacata aagtatgtca atataaagtc ttatttgaca aaaatatgaa tgcacaaagt    360
   ttcttctggt atccaaatgg ctgtaacggt agctctgcat ttagtttaag tggtgatttc    420
   ttatttgact tcaataaaga ttcattaaca gcaaaaggta aagaagttgt tgacagcgtt    480
   gcaacacaat taaaagcctc tgatgcaaaa gaagtgaaag tcgcaggctt tactgaccgt    540
   ttaggttcag aagcgtataa cttaaaactt tctcaacgtc gtgcagatcg tgttaaagcg    600
   cgtttaattg agcaaggtgt tgccgcaaat attcatgctg taggctatgg taaagcacaa    660
   caagtgaaag cttgtgatga tgtacaaggt gcagcattaa gagattgttt acgtcctaac    720
   cgtcgtgttg aaattaccgc ttctggtact gtgttaaaac aaggttcaca aggtatggaa    780
   gcagggacaa caggaccagc accactttat agaaaataa                           819
<210> 13
   <211> 272
   <212> PRT
   <213> Pasteurella multocida
<400> 13
   Met Lys Leu Ser Arg Val Leu Leu Thr Val Val Ala Ala Thr Thr Leu
   1               5                   10                  15
   Ala Ala Cys Gly Asn Leu Ser Lys Val Thr Pro Glu Gly Thr Ser Asp
               20                  25                  30
   Asn Leu Val Trp Pro Lys Ile Asp Glu Ser Val Phe Asn His Asp Gly
           35                  40                  45
   Ser Gln Phe Gly Ser Trp Pro Asn Trp Asp Asn Val Arg Met Val Glu
       50                  55                  60
   Arg Gly Met Asn Lys Asp Gln Leu Tyr Asn Leu Leu Gly Arg Pro His
   65                  70                  75                  80
   Phe Ser Glu Gly Leu Tyr Gly Val Arg Glu Trp Asp Tyr Val Phe Asn
                   85                  90                  95
   Tyr Arg Glu Asn Gly Val His Lys Val Cys Gln Tyr Lys Val Leu Phe
                   100                     105                     110
   Asp Lys Asn Met Asn Ala Gln Ser Phe Phe Trp Tyr Pro Asn Gly Cys
               115                     120                     125
   Asn Gly Ser Ser Ala Phe Ser Leu Ser Gly Asp Phe Leu Phe Asp Phe
       130                     135                     140
   Asn Lys Asp Ser Leu Thr Ala Lys Gly Lys Glu Val Val Asp Ser Val
   145                     150                     155                 160
   Ala Thr Gln Leu Lys Ala Ser Asp Ala Lys Glu Val Lys Val Ala Gly
                       165                     170                 175
   Phe Thr Asp Arg Leu Gly Ser Glu Ala Tyr Asn Leu Lys Leu Ser Gln
                   180                     185                     190
   Arg Arg Ala Asp Arg Val Lys Ala Arg Leu Ile Glu Gln Gly Val Ala
               195                     200                     205
   Ala Asn Ile His Ala Val Gly Tyr Gly Lys Ala Gln Gln Val Lys Ala
       210                     215                     220
   Cys Asp Asp Val Gln Gly Ala Ala Leu Arg Asp Cys Leu Arg Pro Asn
   225                     230                     235                 240
   Arg Arg Val Glu Ile Thr Ala Ser Gly Thr Val Leu Lys Gln Gly Ser
                       245                     250                 255
   Gln Gly Met Glu Ala Gly Thr Thr Gly Pro Ala Pro Leu Tyr Arg Lys
                   260                     265                     270
<210> 14
   <211> 204
   <212> DNA
   <213> Pasteurella multocida
<400> 14
   tgccaattat agactttgtg tgaatgtacg agtaatcaca tcacgttgtt gttcaggtgt     60
   taatgagttg aaacgtacag cgtaacctga aacacggatt gttaattgtg ggtatttttc    120
   tgggttattg accgcatctt ctaaggtttc gcggcgtaat acgttaacgt ttaagtgttg    180
   accaccttct actttgactg ttgg                                           204
<210> 15
   <211> 35
   <212> DNA
   <213> Pasteurella multocida
<400> 15
   aggtgttttt ttaagaggta aatggatgcc aatta 35
<210> 16
   <211> 384
   <212> DNA
   <213> Pasteurella multocida
<400> 16
   atgattaaag gtattcaaat tacccaagcg gctaatgaca atttattaaa ctcattttgg 60
   ttattagata gcgaaaaagg tgaagcgcgt tgtttatgtg ctaaaggtga cttcgttgaa 120
   gatcaaatcg ttgcagtaag tgaattaggt caaatcgaat atcgcgaatt accagttgat 180
   atcgccccaa cagtcaaagt agaaggtggt caacacttaa acgttaacgt attacgccgc 240
   gaaaccttag aagatgcggt caataaccca gaaaaatacc cacaattaac aatccgtgtt 300
   tcaggttacg ctgtacgttt caactcatta acacctgaac aacaacgtga tgtgattact 360
   cgtacattca cacaaagtct ataa 384
<210> 17
   <211> 127
   <212> PRT
   <213> Pasteurella multocida
<400> 17
   Met Ile Lys Gly Ile Gln Ile Thr Gln Ala Ala Asn Asp Asn Leu Leu
   1 5 10 15
   Asn Ser Phe Trp Leu Leu Asp Ser Glu Lys Gly Glu Ala Arg Cys Leu
   20 25 30
   Cys Ala Lys Gly Asp Phe Val Glu Asp Gln Ile Val Ala Val Ser Glu
   35 40 45
   Leu Gly Gln Ile Glu Tyr Arg Glu Leu Pro Val Asp Ile Ala Pro Thr
   50 55 60
   Val Lys Val Glu Gly Gly Gln His Leu Asn Val Asn Val Leu Arg Arg
   65 70 75 80
   Glu Thr Leu Glu Asp Ala Val Asn Asn Pro Glu Lys Tyr Pro Gln Leu
   85 90 95
   Thr Ile Arg Val Ser Gly Tyr Ala Val Arg Phe Asn Ser Leu Thr Pro
   100 105 110
   Glu Gln Gln Arg Asp Val Ile Thr Arg Thr Phe Thr Gln Ser Leu
   115 120 125
<210> 18
   <211> 75
   <212> DNA
   <213> Pasteurella multocida
<400> 18
   acattcttga tgcaataagt cataacgttt tttgagaaac tggagcttat taaagaaaaa 60
   gcgtacatgc cctgt 75
<210> 19
   <211> 390
   <212> DNA
   <213> Pasteurella multocida
<400> 19
   atgacacgga ttaatctcat cgcccccgct gaactttgtg atcaacatct gttagcagaa      60
   cacagagaac tgacacgtat tcccaatgct gtggcaaaag ggaaatttag cctcctcggt     120
   cagccagaag attataaatt aggtacaggg catgtacgct ttttctttaa taagctccag      180
   tttctcaaaa aacgttatga cttattgcat caagaatgtc gagctcgagg ttttaatgtg     240
   caatatattt ggcccgacaa gttgccggag gacgataacc tctggttaga ctacatccct     300
   actgagcatg ccttagccgc caatagagcg cgtattttag aaagaatgcc tgtcaaagcc     360
   cgctttacac caagtaaagc tacaacttaa                                      390
<210> 20
   <211> 129
   <212> PRT
   <213> Pasteurella multocida
<400> 20
   Met Thr Arg Ile Asn Leu Ile Ala Pro Ala Glu Leu Cys Asp Gln His
   1               5                   10                  15
   Leu Leu Ala Glu His Arg Glu Leu Thr Arg Ile Pro Asn Ala Val Ala
               20                  25                  30
   Lys Gly Lys Phe Ser Leu Leu Gly Gln Pro Glu Asp Tyr Lys Leu Gly
           35                  40                  45
   Thr Gly His Val Arg Phe Phe Phe Asn Lys Leu Gln Phe Leu Lys Lys
       50                  55                  60
   Arg Tyr Asp Leu Leu His Gln Glu Cys Arg Ala Arg Gly Phe Asn Val
   65                  70                  75                  80
   Gln Tyr Ile Trp Pro Asp Lys Leu Pro Glu Asp Asp Asn Leu Trp Leu
                   85                  90                  95
   Asp Tyr Ile Pro Thr Glu His Ala Leu Ala Ala Asn Arg Ala Arg Ile
               100                 105                 110
   Leu Glu Arg Met Pro Val Lys Ala Arg Phe Thr Pro Ser Lys Ala Thr
           115                 120                 125
   Thr
<210> 21
   <211> 229
   <212> DNA
   <213> Pasteurella multocida
<400> 21
   cgatttcaat ccctttttgg cggagttgtt catcatcata aatatgttta tcgccgtaga      60
   gggcattgat tgtttcatgg ataccaatat agcctaatga aatagaggca cgcccatttt     120
   taaagatttg tgccacattg tcatcagcct ttaagcgtac accacaggca ccctccatat     180
   aaagaattgg ggcaacgcga gctttggtat gttctaaacg tgcaatgcg                 229
<210> 22
   <211> 2142
   <212> DNA
   <213> Pasteurella multocida
<400> 22
   atggcaactt tctttgtaat taaacgagat gggtcacgaa cggggtttga aatccaacgt 60
   atcattaatg caattaaaaa agcggcacaa gcggtgaata ttgaagatga acgttattgt 120
   catacaatgg gacagcaggt atgtaatgac atctttactc gttaccaaca agaaattgat 180
   atcagccaca ttcaaaaaat cgtagaaaat accttaatgg cgggaaaata ccctgaaata 240
   gcgcgagctt atatcgaata ccgccatgat cgggatctcg cgcgagaaaa acgcagtcaa 300
   ctcacaaaag aaatcgaagg attaattcag caaagtaatg ttgaactcct caatgaaaat 360
   gccaataaag atgcgaaagt tatcccaact caacgcgatc tcttagcggg tattgtggca 420
   aaacattacg ctaaacgtta tattctgcca cgcgatgtcg tagacgcaca tgaaaaaggg 480
   gaaattcatt atcacgattt agactatgcc ccatttttcc caatgtttaa ctgcatgctt 540
   gtcgatctca aagggatgct aagcaatggt ttcaaaatgg gtaatgccga aattgaacca 600
   ccgaaatcga tcacaacagc aaccgcagtc agtgcacaaa ttatcgcaca agtcgcgagc 660
   catatttacg gtggtaccac gattaaccgt atagatgaaa tccttgcccc ttatgtgcaa 720
   ttaagttatg aaaaacattt aaaaaatgca gcggaatgga aagttcccga accagaagcc 780
   tacgcgaaag cactcattga aaaagaatgt ttcgacgctt ttcaatcctt agaatatgaa 840
   gtcaatacgc tgcatacttc aaatgggcaa accccttttg tcacttttgg ctttggctta 900
   ggaacgacgt ggcaatcgag acttatccag cgctcaattc tgaaaaatcg tattcgtggt 960
   ttaggcaaaa atcacaaaac ccctgtcttc ccaaaactgg tgttcactat taaaaaaggc 1020
   attaaccata gcccgagtga tcctaactac gacattaaac aactggcttt agaatgtgcc 1080
   tccaaacgga tgtatcctga tattctcaat tatgatcagg tggtgaaagt cacgggttct 1140
   tttaaagcac caatgggatg ccgtagtttc ttaggtgctt atcaggagca aggacaggaa 1200
   atccatgatg gacgtaataa cttaggcgta gtgagtttga atttaccgcg tatagcaatt 1260
   gaagccaacg ccacgaattc agcccaaagt gcggtcgagt tttataaaat tttagatcaa 1320
   cgtcttgcga ttgccaaaaa agccttaatg acacgcattg cacgtttaga acataccaaa 1380
   gctcgcgttg ccccaattct ttatatggag ggtgcctgtg gtgtacgctt aaaggctgat 1440
   gacaatgtgg cacaaatctt taaaaatggg cgtgcctcta tttcgttagg ctatattggt 1500
   atccatgaaa caatcaatgc cctctacggc gataaacata tttatgatga tgaacaactc 1560
   cgccaaaaag ggattgaaat cgtcgaatat ttacacgaga ccgtgcaacg ttggaaacaa 1620
   gaaacaggtt atgctttcag cctatattcc acaccaagtg aaaacctttg tgaccgtttc 1680
   tgtcgcttgg atactaagca atttgggctt atcgaaggtg tcacagataa aggctactat 1740
   actaatagct accacttaga cgtagagaaa aaagtcaatc cttatgacaa gatagatttt 1800
   gaattgcctt atccaccgtt cgcaagcggc gggtttattt gctatggtga atacccaaat 1860
   gttcagcata accttaaagc attagaggac gtttgggatt atagctatga cagagtgcct 1920
   tactatggga ccaatacacc gattgatgaa tgctatgaat gtggtttcag tggtgaattt 1980
   gaatgtacca gtaaagggtt tacttgtccg aaatgtggta accatgacag tgagaaagtc 2040
   tccgtgaccc gacgtgtctg tggctatctt ggcagtccag atgccagacc atttaatgcc 2100
   ggtaaacaag aagaagtcaa gcgcagagta aaacatctct aa 2142
<210> 23
   <211> 713
   <212> PRT
   <213> Pasteurella multocida
<400> 23
   Met Ala Thr Phe Phe Val Ile Lys Arg Asp Gly Ser Arg Thr Gly Phe
   1               5                   10                  15
   Glu Ile Gln Arg Ile Ile Asn Ala Ile Lys Lys Ala Ala Gln Ala Val
               20                  25                  30
   Asn Ile Glu Asp Glu Arg Tyr Cys His Thr Met Gly Gln Gln Val Cys
           35                  40                  45
   Asn Asp Ile Phe Thr Arg Tyr Gln Gln Glu Ile Asp Ile Ser His Ile
       50                  55                  60
   Gln Lys Ile Val Glu Asn Thr Leu Met Ala Gly Lys Tyr Pro Glu Ile
   65                  70                  75                  80
   Ala Arg Ala Tyr Ile Glu Tyr Arg His Asp Arg Asp Leu Ala Arg Glu
                   85                  90                  95
   Lys Arg Ser Gln Leu Thr Lys Glu Ile Glu Gly Leu Ile Gln Gln Ser
               100                 105                 110
   Asn Val Glu Leu Leu Asn Glu Asn Ala Asn Lys Asp Ala Lys Val Ile
           115                 120                 125
   Pro Thr Gln Arg Asp Leu Leu Ala Gly Ile Val Ala Lys His Tyr Ala
       130                 135                 140
   Lys Arg Tyr Ile Leu Pro Arg Asp Val Val Asp Ala His Glu Lys Gly
   145                 150                 155                 160
   Glu Ile His Tyr His Asp Leu Asp Tyr Ala Pro Phe Phe Pro Met Phe
                   165                 170                 175
   Asn Cys Met Leu Val Asp Leu Lys Gly Met Leu Ser Asn Gly Phe Lys
               180                 185                 190
   Met Gly Asn Ala Glu Ile Glu Pro Pro Lys Ser Ile Thr Thr Ala Thr
           195                 200                 205
   Ala Val Ser Ala Gln Ile Ile Ala Gln Val Ala Ser His Ile Tyr Gly
       210                 215                 220
   Gly Thr Thr Ile Asn Arg Ile Asp Glu Ile Leu Ala Pro Tyr Val Gln
   225                 230                 235                 240
   Leu Ser Tyr Glu Lys His Leu Lys Asn Ala Ala Glu Trp Lys Val Pro
                   245                 250                 255
   Glu Pro Glu Ala Tyr Ala Lys Ala Leu Ile Glu Lys Glu Cys Phe Asp
           260                 265                 270
   Ala Phe Gln Ser Leu Glu Tyr Glu Val Asn Thr Leu His Thr Ser Asn
       275                 280                 285
   Gly Gln Thr Pro Phe Val Thr Phe Gly Phe Gly Leu Gly Thr Thr Trp
       290                 295                 300
   Gln Ser Arg Leu Ile Gln Arg Ser Ile Leu Lys Asn Arg Ile Arg Gly
   305                 310                 315                 320
   Leu Gly Lys Asn His Lys Thr Pro Val Phe Pro Lys Leu Val Phe Thr
                   325                 330                 335
   Ile Lys Lys Gly Ile Asn His Ser Pro Ser Asp Pro Asn Tyr Asp Ile
               340                 345                 350
   Lys Gln Leu Ala Leu Glu Cys Ala Ser Lys Arg Met Tyr Pro Asp Ile
           355                 360                 365
   Leu Asn Tyr Asp Gln Val Val Lys Val Thr Gly Ser Phe Lys Ala Pro
       370                 375                 380
   Met Gly Cys Arg Ser Phe Leu Gly Ala Tyr Gln Glu Gln Gly Gln Glu
   385                 390                 395                 400
   Ile His Asp Gly Arg Asn Asn Leu Gly Val Val Ser Leu Asn Leu Pro
                   405                 410                 415
   Arg Ile Ala Ile Glu Ala Asn Ala Thr Asn Ser Ala Gln Ser Ala Val
               420                 425                 430
   Glu Phe Tyr Lys Ile Leu Asp Gln Arg Leu Ala Ile Ala Lys Lys Ala
           435                 440                 445
   Leu Met Thr Arg Ile Ala Arg Leu Glu His Thr Lys Ala Arg Val Ala
       450                 455                 460
   Pro Ile Leu Tyr Met Glu Gly Ala Cys Gly Val Arg Leu Lys Ala Asp
   465                 470                 475                 480
   Asp Asn Val Ala Gln Ile Phe Lys Asn Gly Arg Ala Ser Ile Ser Leu
                   485                 490                 495
   Gly Tyr Ile Gly Ile His Glu Thr Ile Asn Ala Leu Tyr Gly Asp Lys
               500                 505                 510
   His Ile Tyr Asp Asp Glu Gln Leu Arg Gln Lys Gly Ile Glu Ile Val
           515                 520                 525
   Glu Tyr Leu His Glu Thr Val Gln Arg Trp Lys Gln Glu Thr Gly Tyr
       530                 535                 540
   Ala Phe Ser Leu Tyr Ser Thr Pro Ser Glu Asn Leu Cys Asp Arg Phe
   545                 550                 555                 560
   Cys Arg Leu Asp Thr Lys Gln Phe Gly Leu Ile Glu Gly Val Thr Asp
                   565                 570                 575
   Lys Gly Tyr Tyr Thr Asn Ser Tyr His Leu Asp Val Glu Lys Lys Val
               580                 585                 590
   Asn Pro Tyr Asp Lys Ile Asp Phe Glu Leu Pro Tyr Pro Pro Phe Ala
           595                 600                 605
   Ser Gly Gly Phe Ile Cys Tyr Gly Glu Tyr Pro Asn Val Gln His Asn
       610                 615                 620
   Leu Lys Ala Leu Glu Asp Val Trp Asp Tyr Ser Tyr Asp Arg Val Pro
   625                 630                 635                 640
   Tyr Tyr Gly Thr Asn Thr Pro Ile Asp Glu Cys Tyr Glu Cys Gly Phe
                   645                 650                 655
   Ser Gly Glu Phe Glu Cys Thr Ser Lys Gly Phe Thr Cys Pro Lys Cys
               660                 665                 670
   Gly Asn His Asp Ser Glu Lys Val Ser Val Thr Arg Arg Val Cys Gly
           675                 680                 685
   Tyr Leu Gly Ser Pro Asp Ala Arg Pro Phe Asn Ala Gly Lys Gln Glu
       690                 695                 700
   Glu Val Lys Arg Arg Val Lys His Leu
   705 710
<210> 24
   <211> 58
   <212> DNA
   <213> Pasteurella multocida
<400> 24
   attgtgatta cgggattatc gggatcaggt aaatcttctt tagcctttga taccctgt 58
<210> 25
   <211> 2832
   <212> DNA
   <213> Pasteurella multocida
<400> 25
   atggataaga ttgaggtacg tggggcgaga acccataatt taaaaaatat taatttaact 60
   attcctcgtg ataaattaat tgtgattacg ggattatcgg gatcaggtaa atcttcttta 120
   gcctttgata ccctgtatgc tgagggacaa cgccgttatg tagaatcctt gtcggcatat 180
   gcacgtcaat tcttatccct aatggaaaag ccggatgtgg atcatattga aggattatcg 240
   ccggcgattt ctattgaaca aaaatctacc tcacataatc cgcgttcaac ggtgggtacg 300
   gtcaccgaaa ttcatgatta cttacgtctt ctttttgctc gagtagggga accgcgttgt 360
   ccgaatcatg atgtgccttt agcggcacaa accattagcc agatggtgga taaagtattg 420
   agtttgcctg aagaaagcaa aatgatgttg ctggcgccag tggtgaaaga acgtaagggt 480
   gaacatgtta aattattaga gcaaattgcc gcccaaggtt atattcgagc cagaattgac 540
   ggtgaaattt gtgatttatc tgatgcacct aaattagaat tacataaaaa acatactatt 600
   gaagtggttg tggaccgttt taaggtgcgg tctgatttag ccacaagatt agcagaatcc 660
   tttgaaacag cattggaatt atctggtggc accgctgtag ttgcttccat ggatgagcct 720
   gaaacggaag aattggtctt ttcggctaat tttgcttgtc cacattgtgg gtattctgtc 780
   ccagaattag aacctcgttt attttctttt aataatcccg ccggggcgtg tccgacttgc 840
   gatggcttag gtgtacaaca atattttgat gaaaaacgcg tggtgcaaaa cccgagtatt 900
   tcgttagcca gtggcgcagt aaaaggctgg gatcgtcgta acttctatta ctaccaaatg 960
   ctcacctcct tagcgaagca ttatgaattt gatattgaat caccttttga ggcactgccg 1020
   aaaaaaatcc agcagattat tttaaatggt tcaggtaagg aagaaattga gtttcaatac 1080
   atgaatgatc gcggcgatgt agtcgtgcgt catcatgcat ttgaaggcat tctaaataat 1140
   atggcgcgcc gttataaaga aacggaatcg ctgtctgtgc gtgaagaatt agcgaaaaat 1200
   atcagtacct gtccttgcca tgattgtggg ggttcacgtt tacgtcaaga ggcacgtcat 1260
   gtgtatattg gcaccaccac tttacctgat gtggcagaaa agagtattgg cgaaaccttg 1320
   catttcttta gtgaattgca tttaagcggg caaagagctc aaattgccga gaaaatctta 1380
   aaagaaatta aagagcgctt acaattttta gtcaatgtag ggttggatta tctttccctt 1440
   tctcgttcag cagaaacctt gtctggtggg gaggcacagc gaattcgttt agccagtcaa 1500
   attggtgcgg gtttagtggg ggtgatgtat gtgctagatg agccgtctat tggtttgcat 1560
   caacgtgata atgagcgatt actgaataca ttgcttcact tacgtaactt agggaacacc 1620
   gtgattgtgg tagaacatga tgaagatgcc attatggcgg cagatcatat tattgatatt 1680
   ggtcccgggg caggagttca tggtgggcaa attgtggcag aaggttcggc aaaggcgatt 1740
   atggctaatc cacactcaat tacggggaaa tttttatctg ggtcgagaaa aatcgaaatt 1800
   cccgcaaaac ggaccgcact tgataagaaa aaaatgttga aattagaagg ggcaacgggg 1860
   aataatctga aatcagtgaa tttagccatt ccagtaggat tgtttacctg tgtgacaggt 1920
   gtttcggggt cagggaaatc gaccttgatt aatgatacgt tgttcccatt agcacaaaat 1980
   gccttgaatc gtgcggaaaa tacgcaattt gcgccttatc aatccatttc gggtttggaa 2040
   ttttttgata aagtaattga tattgaccaa agtccaattg gtcgtacacc gcgttcgaat 2100
   cctgccactt atactggctt atttacgccg attcgagaat tatttgcggg cgtgcctgag 2160
   tcgagagccc ggggttataa tcccggacgt tttagtttta atgtacgcgg tggacgctgt 2220
   gaggcctgtc aaggcgatgg tgtgattaaa gtagagatgc actttttgcc cgatgtgtat 2280
   gtgccttgtg agcaatgtaa gggaaaacgt tataatcgag agaccttaga gatccgttac 2340
   aaaggtaaaa cgattcatca agtgttagaa atgacggtag aagaagcgcg cgagtttttt   2400
   gatgcgattc cgcagatcgc ccgtaaatta caaactttaa tggatgttgg tttatcctat   2460
   attcgtttag gacaatcttc gaccacgtta tcgggtgggg aagcgcaacg agtgaaatta   2520
   gcaacggagc tttcaaaacg tgatacaggg aaaactttgt atgtattaga tgaaccgacg   2580
   acaggtttac attttgctga tattaaacag ctattaacag tcttgcatcg tttacgtgat   2640
   caaggcaata cgatagtggt gattgagcac aatttagatg tgatcaaaac agccgattgg   2700
   attattgatt taggtcctga aggggggaat ggcggtggac aaattattgc cacaggcaca   2760
   ccagaacagg tcgctgaagt gaaaggttca cataccgcac gcttcttaaa aacgctttta   2820
   caaaagcgct aa                                                        2832
<210> 26
   <211> 943
   <212> PRT
   <213> Pasteurella multocida
<400> 26
   Met Asp Lys Ile Glu Val Arg Gly Ala Arg Thr His Asn Leu Lys Asn
   1               5                   10                  15
   Ile Asn Leu Thr Ile Pro Arg Asp Lys Leu Ile Val Ile Thr Gly Leu
               20                  25                  30
   Ser Gly Ser Gly Lys Ser Ser Leu Ala Phe Asp Thr Leu Tyr Ala Glu
           35                  40                  45
   Gly Gln Arg Arg Tyr Val Glu Ser Leu Ser Ala Tyr Ala Arg Gln Phe
       50                  55                  60
   Leu Ser Leu Met Glu Lys Pro Asp Val Asp His Ile Glu Gly Leu Ser
   65                  70                  75                  80
   Pro Ala Ile Ser Ile Glu Gln Lys Ser Thr Ser His Asn Pro Arg Ser
                   85                  90                  95
   Thr Val Gly Thr Val Thr Glu Ile His Asp Tyr Leu Arg Leu Leu Phe
               100                 105                 110
   Ala Arg Val Gly Glu Pro Arg Cys Pro Asn His Asp Val Pro Leu Ala
           115                 120                 125
   Ala Gln Thr Ile Ser Gln Met Val Asp Lys Val Leu Ser Leu Pro Glu
       130                 135                 140
   Glu Ser Lys Met Met Leu Leu Ala Pro Val Val Lys Glu Arg Lys Gly
   145                 150                 155                 160
   Glu His Val Lys Leu Leu Glu Gln Ile Ala Ala Gln Gly Tyr Ile Arg
                   165                 170                 175
   Ala Arg Ile Asp Gly Glu Ile Cys Asp Leu Ser Asp Ala Pro Lys Leu
               180                 185                 190
   Glu Leu His Lys Lys His Thr Ile Glu Val Val Val Asp Arg Phe Lys
           195                 200                 205
   Val Arg Ser Asp Leu Ala Thr Arg Leu Ala Glu Ser Phe Glu Thr Ala
       210                 215                 220
   Leu Glu Leu Ser Gly Gly Thr Ala Val Val Ala Ser Met Asp Glu Pro
   225                 230                 235                 240
   Glu Thr Glu Glu Leu Val Phe Ser Ala Asn Phe Ala Cys Pro His Cys
                   245                 250                 255
   Gly Tyr Ser Val Pro Glu Leu Glu Pro Arg Leu Phe Ser Phe Asn Asn
               260                 265                 270
   Pro Ala Gly Ala Cys Pro Thr Cys Asp Gly Leu Gly Val Gln Gln Tyr
           275                 280                 285
   Phe Asp Glu Lys Arg Val Val Gln Asn Pro Ser Ile Ser Leu Ala Ser
       290                 295                 300
   Gly Ala Val Lys Gly Trp Asp Arg Arg Asn Phe Tyr Tyr Tyr Gln Met
   305                 310                 315                 320
   Leu Thr Ser Leu Ala Lys His Tyr Glu Phe Asp Ile Glu Ser Pro Phe
                   325                 330                 335
   Glu Ala Leu Pro Lys Lys Ile Gln Gln Ile Ile Leu Asn Gly Ser Gly
               340                 345                 350
   Lys Glu Glu Ile Glu Phe Gln Tyr Met Asn Asp Arg Gly Asp Val Val
           355                 360                 365
   Val Arg His His Ala Phe Glu Gly Ile Leu Asn Asn Met Ala Arg Arg
       370                 375                 380
   Tyr Lys Glu Thr Glu Ser Leu Ser Val Arg Glu Glu Leu Ala Lys Asn
   385                 390                 395                 400
   Ile Ser Thr Cys Pro Cys His Asp Cys Gly Gly Ser Arg Leu Arg Gln
                   405                 410                 415
   Glu Ala Arg His Val Tyr Ile Gly Thr Thr Thr Leu Pro Asp Val Ala
               420                 425                 430
   Glu Lys Ser Ile Gly Glu Thr Leu His Phe Phe Ser Glu Leu His Leu
           435                 440                 445
   Ser Gly Gln Arg Ala Gln Ile Ala Glu Lys Ile Leu Lys Glu Ile Lys
       450                 455                 460
   Glu Arg Leu Gln Phe Leu Val Asn Val Gly Leu Asp Tyr Leu Ser Leu
   465                 470                 475                 480
   Ser Arg Ser Ala Glu Thr Leu Ser Gly Gly Glu Ala Gln Arg Ile Arg
                   485                 490                 495
   Leu Ala Ser Gln Ile Gly Ala Gly Leu Val Gly Val Met Tyr Val Leu
               500                 505                 510
   Asp Glu Pro Ser Ile Gly Leu His Gln Arg Asp Asn Glu Arg Leu Leu
           515                 520                 525
   Asn Thr Leu Leu His Leu Arg Asn Leu Gly Asn Thr Val Ile Val Val
       530                 535                 540
   Glu His Asp Glu Asp Ala Ile Met Ala Ala Asp His Ile Ile Asp Ile
   545                 550                 555                 560
   Gly Pro Gly Ala Gly Val His Gly Gly Gln Ile Val Ala Glu Gly Ser
                   565                 570                 575
   Ala Lys Ala Ile Met Ala Asn Pro His Ser Ile Thr Gly Lys Phe Leu
               580                 585                 590
   Ser Gly Val Glu Lys Ile Glu Ile Pro Ala Lys Arg Thr Ala Leu Asp
           595                 600                 605
   Lys Lys Lys Met Leu Lys Leu Glu Gly Ala Thr Gly Asn Asn Leu Lys
       610                 615                 620
   Ser Val Asn Leu Ala Ile Pro Val Gly Leu Phe Thr Cys Val Thr Gly
   625                 630                 635                 640
   Val Ser Gly Ser Gly Lys Ser Thr Leu Ile Asn Asp Thr Leu Phe Pro
                   645                 650                 655
   Leu Ala Gln Asn Ala Leu Asn Arg Ala Glu Asn Thr Gln Phe Ala Pro
               660                 665                 670
   Tyr Gln Ser Ile Ser Gly Leu Glu Phe Phe Asp Lys Val Ile Asp Ile
           675                 680                 685
   Asp Gln Ser Pro Ile Gly Arg Thr Pro Arg Ser Asn Pro Ala Thr Tyr
       690                 695                 700
   Thr Gly Leu Phe Thr Pro Ile Arg Glu Leu Phe Ala Gly Val Pro Glu
   705                 710                 715                 720
   Ser Arg Ala Arg Gly Tyr Asn Pro Gly Arg Phe Ser Phe Asn Val Arg
                   725                 730                 735
   Gly Gly Arg Cys Glu Ala Cys Gln Gly Asp Gly Val Ile Lys Val Glu
               740                 745                 750
   Met His Phe Leu Pro Asp Val Tyr Val Pro Cys Glu Gln Cys Lys Gly
           755                 760                 765
   Lys Arg Tyr Asn Arg Glu Thr Leu Glu Ile Arg Tyr Lys Gly Lys Thr
   770                 775                 780
   Ile His Gln Val Leu Glu Met Thr Val Glu Glu Ala Arg Glu Phe Phe
   785                 790                 795                 800
   Asp Ala Ile Pro Gln Ile Ala Arg Lys Leu Gln Thr Leu Met Asp Val
                   805                 810                 815
   Gly Leu Ser Tyr Ile Arg Leu Gly Gln Ser Ser Thr Thr Leu Ser Gly
               820                 825                 830
   Gly Glu Ala Gln Arg Val Lys Leu Ala Thr Glu Leu Ser Lys Arg Asp
           835                 840                 845
   Thr Gly Lys Thr Leu Tyr Val Leu Asp Glu Pro Thr Thr Gly Leu His
       850                 855                 860
   Phe Ala Asp Ile Lys Gln Leu Leu Thr Val Leu His Arg Leu Arg Asp
   865                 870                 875                 880
   Gln Gly Asn Thr Ile Val Val Ile Glu His Asn Leu Asp Val Ile Lys
                   885                 890                 895
   Thr Ala Asp Trp Ile Ile Asp Leu Gly Pro Glu Gly Gly Asn Gly Gly
               900                 905                 910
   Gly Gln Ile Ile Ala Thr Gly Thr Pro Glu Gln Val Ala Glu Val Lys
           915                 920                 925
   Gly Ser His Thr Ala Arg Phe Leu Lys Thr Leu Leu Gln Lys Arg
       930                 935                 940
<210> 27
   <211> 54
   <212> DNA
   <213> Pasteurella multocida
<400> 27
   caggacttag tgggtaacaa cacaccagtc ttctttatcc gtgatccatt gaaa 54
<210> 28
   <211> 1455
   <212> DNA
   <213> Pasteurella multocida
<400> 28
   atgtctaaat gcccatttga ccatggttca aaaaccttaa cgaatgcagc tggtgcacct   60
   attgttgaaa acgacaacac catgtctgcg ggtcctaaag gtccattact tttacaagat  120
   gtttggttcc aagagaaatt agcacacttt gcacgtgagc gtattcctga gcgtgttgtt  180
   catgcaaaag gttcagcagc gtacggtaca ttcactgtga cacacgatat cagcaaatac  240
   accaaagcgg atttattcaa tggtatcggt aaacaaacac aagtcttatt acgtttctca  300
   acagtagcgg gtgagcgcgg tgcggcagac gctgagcgtg atgtgcgtgg tttcgcatta  360
   aaattctaca ctgaacaagg taactgggac ttagtgggta acaacacacc cgtattcttt  420
   atccgtgatc cattgaaatt cccagatttc attcatactc aaaaacgtaa tccacaaact  480
   aacttgcgtg atgcaaacgc ggcatgggat ttctggtcac gtcatcctga atcattacac  540
   caaatcatga ttcttttcag tgatcgtggt attccaactg acttacgtca tatgaacggt  600
   tacggtagcc atacatatag ctttattaac gcacaaaatg agcgtttctg ggtgaaattc  660
   cacttcaaaa cacaacaagg tcacaaattc tatactaatg aagaagcggc taaagtggtg  720
   ggtgaaaacc gtgagtcaag ccaacaagat ttatacgaag cgattgagcg tggcgaattc  780
   ccacgttgga atgttcaagt gcaaatcatg ccagaagcag atgcacacaa acataactat  840
   gcgtttgact taactaaagt atggccacac aaagattatc cgatgatcga agtgggtgta  900
   ttagagttaa accaaaaccc aattaactac ttcgcagaag tggaacaagc tgcgtttgca  960
   ccttctaaca tcgtaccggg aattggtttc tcaccagacc gtatgttaca aggtcgtctt 1020
   ttctcatacc aagacgcgca acgttatcgt ttaggggtta accatcacca aatcccagtg 1080
   aacgcaccaa aatgcccata ccacaccact caccgtgatg gcgcaatgcg tgtagataac 1140
   aatggtggta cacaccctaa ctatgcaccg aaccgttttg atacttatgt gccgactcac   1200
   caacaagagc ctgcattaga gttagagcgt tcagcagcac actttaactt ccgtgagtat   1260
   gatgaagact actacacaca acctgccgca ctttacaact tattcgatgt ggatcaaaaa   1320
   gcacgtgtgg cagccaactt cgcagcgggc ttagcaggtg ttacagaacc tgcgattgtt   1380
   gaaagacaat tagcccactt cgacaaagta agcaaagaat tagctgatgc aattcgtgcg   1440
   aacttagcga aataa                                                    1455
<210> 29
   <211> 484
   <212> PRT
   <213> Pasteurella multocida
<400> 29
   Met Ser Lys Cys Pro Phe Asp His Gly Ser Lys Thr Leu Thr Asn Ala
   1               5                   10                  15
   Ala Gly Ala Pro Ile Val Glu Asn Asp Asn Thr Met Ser Ala Gly Pro
               20                  25                  30
   Lys Gly Pro Leu Leu Leu Gln Asp Val Trp Phe Gln Glu Lys Leu Ala
           35                  40                  45
   His Phe Ala Arg Glu Arg Ile Pro Glu Arg Val Val His Ala Lys Gly
       50                  55                  60
   Ser Ala Ala Tyr Gly Thr Phe Thr Val Thr His Asp Ile Ser Lys Tyr
   65                  70                  75                  80
   Thr Lys Ala Asp Leu Phe Asn Gly Ile Gly Lys Gln Thr Gln Val Leu
                   85                  90                  95
   Leu Arg Phe Ser Thr Val Ala Gly Glu Arg Gly Ala Ala Asp Ala Glu
               100                 105                 110
   Arg Asp Val Arg Gly Phe Ala Leu Lys Phe Tyr Thr Glu Gln Gly Asn
           115                 120                 125
   Trp Asp Leu Val Gly Asn Asn Thr Pro Val Phe Phe Ile Arg Asp Pro
       130                 135                 140
   Leu Lys Phe Pro Asp Phe Ile His Thr Gln Lys Arg Asn Pro Gln Thr
   145                 150                 155                 160
   Asn Leu Arg Asp Ala Asn Ala Ala Trp Asp Phe Trp Ser Arg His Pro
                   165                 170                 175
   Glu Ser Leu His Gln Ile Met Ile Leu Phe Ser Asp Arg Gly Ile Pro
               180                 185                 190
   Thr Asp Leu Arg His Met Asn Gly Tyr Gly Ser His Thr Tyr Ser Phe
           195                 200                 205
   Ile Asn Ala Gln Asn Glu Arg Phe Trp Val Lys Phe His Phe Lys Thr
       210                 215                 220
   Gln Gln Gly His Lys Phe Tyr Thr Asn Glu Glu Ala Ala Lys Val Val
   225                 230                 235                 240
   Gly Glu Asn Arg Glu Ser Ser Gln Gln Asp Leu Tyr Glu Ala Ile Glu
                   245                 250                 255
   Arg Gly Glu Phe Pro Arg Trp Asn Val Gln Val Gln Ile Met Pro Glu
               260                 265                 270
   Ala Asp Ala His Lys His Asn Tyr Ala Phe Asp Leu Thr Lys Val Trp
           275                 280                 285
   Pro His Lys Asp Tyr Pro Met Ile Glu Val Gly Val Leu Glu Leu Asn
       290                 295                 300
   Gln Asn Pro Ile Asn Tyr Phe Ala Glu Val Glu Gln Ala Ala Phe Ala
   305                 310                 315                 320
   Pro Ser Asn Ile Val Pro Gly Ile Gly Phe Ser Pro Asp Arg Met Leu
                   325                 330                 335
   Gln Gly Arg Leu Phe Ser Tyr Gln Asp Ala Gln Arg Tyr Arg Leu Gly
               340                 345                 350
   Val Asn His His Gln Ile Pro Val Asn Ala Pro Lys Cys Pro Tyr His
           355                 360                 365
   Thr Thr His Arg Asp Gly Ala Met Arg Val Asp Asn Asn Gly Gly Thr
       370                 375                 380
   His Pro Asn Tyr Ala Pro Asn Arg Phe Asp Thr Tyr Val Pro Thr His
   385                 390                 395                 400
   Gln Gln Glu Pro Ala Leu Glu Leu Glu Arg Ser Ala Ala His Phe Asn
                   405                 410                 415
   Phe Arg Glu Tyr Asp Glu Asp Tyr Tyr Thr Gln Pro Ala Ala Leu Tyr
               420                 425                 430
   Asn Leu Phe Asp Val Asp Gln Lys Ala Arg Val Ala Ala Asn Phe Ala
           435                 440                 445
   Ala Gly Leu Ala Gly Val Thr Glu Pro Ala Ile Val Glu Arg Gln Leu
       450                 455                 460
   Ala His Phe Asp Lys Val Ser Lys Glu Leu Ala Asp Ala Ile Arg Ala
   465                 470                 475                 480
   Asn Leu Ala Lys
<210> 30
   <211> 172
   <212> DNA
   <213> Pasteurella multocida
<400> 30
   atttctcacg cattttttcg gtaaaaccaa cgggaatcgt tgattttata ataatcgttg    60
   cttgtggatt gattgagagg gtttgttcaa tgacagcttc aacagtggat gtattaaaat   120
   aacctgtttc ggtattatag tctgttggcg ttgcgatgat gacaaagtcc tg           172
<210> 31
   <211> 1173
   <212> DNA
   <213> Pasteurella multocida
<400> 31
   atgaagaaaa ttacaattgc tggggctggc tatgttggtt tatccaatgc agtattatta    60
   gctcaacacc acaatgtgat cttattagat attgatcaaa ataaagttga tttaattaat   120
   aataaaaaat cgcccatcac agataaagaa atcgaagatt tcttacaaaa taaatcactg   180
   acaatgatgg caacaacaga taaagaagtg gcattaaaaa acgcagactt tgtcatcatc   240
   gcaacgccaa cagactataa taccgaaaca ggttatttta atacatccac tgttgaagct   300
   gtcattgaac aaaccctttc aatcaatcca caagcaacga ttattataaa atcaacaatt   360
   cccgttggtt ttaccgaaaa catgcgtgaa aaatttaata ccccaaatct tatcttttca   420
   cctgaatttc taagagaggg aaaagccctt tacgataatt tgtatccaag cagaattatt   480
   gttggcagta cttcttatca agcaaaagta tttgccgata tgttgacaca gtgtgccaga   540
   aaaaaagatg taactgtttt atttacacac aatactgagg ccgaagctgt taaattattt   600
   gcaaatacgt atctcgcaat gcgagttgcc ttttttaatg aattagatac ttatgcgagt    660
   cttcaccatt taaatacaaa agacattatc aatggtattt ctactgatcc tcgcattggt   720
   acacactaca ataacccaag tttcggctat ggcggttatt gtttacccaa agacactaaa   780
   cagttactgg ctaactatgc tgacgtgcct caaaatctca ttgaagccat tgtcaaatct   840
   aatgaaacca gaaaacgttt cattactcat gatgtattaa ataagaaacc taaaactgtt   900
   ggtatttatc gtttaatcat gaagtcaggt tctgataact tcagagcttc tgctattctc   960
   gatattatgc cgcatctcaa agaaaacggt gttgagattg tgatttatga gccaacctta  1020
   aatcaacagg catttgagga ctaccccgtt attaatcaac tctctgaatt tattaatcgc  1080
   tctgatgtca ttctcgctaa tcgttctgag ccagatttaa atcaatgttc ccataaaatc  1140
   tatacaagag atatttttgg cggtgatgct taa 1173
<210> 32
   <211> 390
   <212> PRT
   <213> Pasteurella multocida
<400> 32
   Met Lys Lys Ile Thr Ile Ala Gly Ala Gly Tyr Val Gly Leu Ser Asn
   1               5                   10                  15
   Ala Val Leu Leu Ala Gln His His Asn Val Ile Leu Leu Asp Ile Asp
               20                  25                  30
   Gln Asn Lys Val Asp Leu Ile Asn Asn Lys Lys Ser Pro Ile Thr Asp
           35                  40                  45
   Lys Glu Ile Glu Asp Phe Leu Gln Asn Lys Ser Leu Thr Met Met Ala
       50                  55                  60
   Thr Thr Asp Lys Glu Val Ala Leu Lys Asn Ala Asp Phe Val Ile Ile
   65                  70                  75                  80
   Ala Thr Pro Thr Asp Tyr Asn Thr Glu Thr Gly Tyr Phe Asn Thr Ser
                   85                  90                  95
   Thr Val Glu Ala Val Ile Glu Gln Thr Leu Ser Ile Asn Pro Gln Ala
               100                 105                 110
   Thr Ile Ile Ile Lys Ser Thr Ile Pro Val Gly Phe Thr Glu Asn Met
           115                 120                 125
   Arg Glu Lys Phe Asn Thr Pro Asn Leu Ile Phe Ser Pro Glu Phe Leu
       130                 135                 140
   Arg Glu Gly Lys Ala Leu Tyr Asp Asn Leu Tyr Pro Ser Arg Ile Ile
   145                 150                 155                 160
   Val Gly Ser Thr Ser Tyr Gln Ala Lys Val Phe Ala Asp Met Leu Thr
                   165                 170                 175
   Gln Cys Ala Arg Lys Lys Asp Val Thr Val Leu Phe Thr His Asn Thr
               180                 185                 190
   Glu Ala Glu Ala Val Lys Leu Phe Ala Asn Thr Tyr Leu Ala Met Arg
           195                 200                 205
   Val Ala Phe Phe Asn Glu Leu Asp Thr Tyr Ala Ser Leu His His Leu
       210                 215                 220
   Asn Thr Lys Asp Ile Ile Asn Gly Ile Ser Thr Asp Pro Arg Ile Gly
   225                 230                 235                 240
   Thr His Tyr Asn Asn Pro Ser Phe Gly Tyr Gly Gly Tyr Cys Leu Pro
                   245                 250                 255
   Lys Asp Thr Lys Gln Leu Leu Ala Asn Tyr Ala Asp Val Pro Gln Asn
               260                 265                 270
   Leu Ile Glu Ala Ile Val Lys Ser Asn Glu Thr Arg Lys Arg Phe Ile
           275                 280                 285
   Thr His Asp Val Leu Asn Lys Lys Pro Lys Thr Val Gly Ile Tyr Arg
       290                 295                 300
   Leu Ile Met Lys Ser Gly Ser Asp Asn Phe Arg Ala Ser Ala Ile Leu
   305                 310                 315                 320
   Asp Ile Met Pro His Leu Lys Glu Asn Gly Val Glu Ile Val Ile Tyr
                   325                 330                 335
   Glu Pro Thr Leu Asn Gln Gln Ala Phe Glu Asp Tyr Pro Val Ile Asn
               340                 345                 350
   Gln Leu Ser Glu Phe Ile Asn Arg Ser Asp Val Ile Leu Ala Asn Arg
           355                 360                 365
   Ser Glu Pro Asp Leu Asn Gln Cys Ser His Lys Ile Tyr Thr Arg Asp
       370                 375                 380
   Ile Phe Gly Gly Asp Ala
   385                 390
<210> 33
   <211> 226
   <212> DNA
   <213> Pasteurella multocida
<400> 33
   gcaccaaagt gaataatatt tgggaaacct cgggcttgaa tattttagag gtattagtac    60
   gtttagatag caccaagtta cctagtttca tttctaacat cctgtccgcg cgaaccaata   120
   tttcggcaat ctatattcaa aaagccttca aagtagaacc acagaaatca ctcgaagcgt   180
   ttaaggatct tgatactcta gcagatacag cagaagctta tactaa                  226
<210> 34
   <211> 726
   <212> DNA
   <213> Pasteurella multocida
<400> 34
   atgaataatg atcaacctct tttaaaagca cagagtccgg ctggtttagc ggaagaatat    60
   attgtcagaa gtatctggaa taatcatttc ccaccaggct ccgatttgcc ggctgaacgt   120
   gagttggcag agaaaattgg ggtgacgcgt accacgttac gtgaagtgct acaacgcctg   180
   gcgcgtgacg gttggttgaa tattcaacat gggaaaccaa ccaaagtgaa taatatttgg   240
   gaaacttcgg gcttgaatat tttagaggta ttagtacgtt tagatagcac caagttacct   300
   agtttcattt ctaacatcct gtccgcgcga accaatattt cggcaatcta tattcaaaaa   360
   gccttcaaag tagaaccaca gaaatcactc gaagcgttta aggatcttga tactctagca   420
   gatacagcag aagcttatac taattttgat tatgatcttt tccgtaaatt agcatttgca   480
   tctgataatc ctgtgtatgg tttgatttta aatagtttga aagggttata tacacgtgta   540
   gggttgtttt attttgccaa tccatcggcg cgtgagttag cgaaacgctt ttatctttcg   600
   ctaaagacgt tgtgtcaaac acagcaagtg aacgatgtca aagagtgtat ccgtcaatat   660
   ggtaaagaca gtggggtgat ttgggcaaat atgcaggcat atttaccggc taattttaat   720
   gaatag                                                              726
<210> 35
   <211> 241
   <212> PRT
   <213> Pasteurella multocida
<400> 35
   Met Asn Asn Asp Gln Pro Leu Leu Lys Ala Gln Ser Pro Ala Gly Leu
   1               5                   10                  15
   Ala Glu Glu Tyr Ile Val Arg Ser Ile Trp Asn Asn His Phe Pro Pro
               20                  25                  30
   Gly Ser Asp Leu Pro Ala Glu Arg Glu Leu Ala Glu Lys Ile Gly Val
           35                  40                  45
   Thr Arg Thr Thr Leu Arg Glu Val Leu Gln Arg Leu Ala Arg Asp Gly
       50                  55                  60
   Trp Leu Asn Ile Gln His Gly Lys Pro Thr Lys Val Asn Asn Ile Trp
   65                  70                  75                  80
   Glu Thr Ser Gly Leu Asn Ile Leu Glu Val Leu Val Arg Leu Asp Ser
                   85                  90                  95
   Thr Lys Leu Pro Ser Phe Ile Ser Asn Ile Leu Ser Ala Arg Thr Asn
               100                 105                 110
   Ile Ser Ala Ile Tyr Ile Gln Lys Ala Phe Lys Val Glu Pro Gln Lys
           115                 120                 125
   Ser Leu Glu Ala Phe Lys Asp Leu Asp Thr Leu Ala Asp Thr Ala Glu
       130                 135                 140
   Ala Tyr Thr Asn Phe Asp Tyr Asp Leu Phe Arg Lys Leu Ala Phe Ala
   145                 150                 155                 160
   Ser Asp Asn Pro Val Tyr Gly Leu Ile Leu Asn Ser Leu Lys Gly Leu
                   165                 170                 175
   Tyr Thr Arg Val Gly Leu Phe Tyr Phe Ala Asn Pro Ser Ala Arg Glu
               180                 185                 190
   Leu Ala Lys Arg Phe Tyr Leu Ser Leu Lys Thr Leu Cys Gln Thr Gln
           195                 200                 205
   Gln Val Asn Asp Val Lys Glu Cys Ile Arg Gln Tyr Gly Lys Asp Ser
       210                 215                 220
   Gly Val Ile Trp Ala Asn Met Gln Ala Tyr Leu Pro Ala Asn Phe Asn
   225                 230                 235                 240
   Glu
<210> 36
   <211> 214
   <212> DNA
   <213> Pasteurella multocida
<400> 36
   acaaccgctg gcgtatccgt taaacggtgg gttaagcgca cttctttcac gcgctcacca 60
   agcaaggttt tcacacgttc cacaaaagaa gcatattgct catcttgtgc tttttgactg 120
   tcttcctctt tatccgctaa atcacctaga tctaaatccg ctttactgat ggtttgcagt 180
   ggcttaccgt caaattccgt taagtaactt aaca 214
<210> 37
   <211> 1896
   <212> DNA
   <213> Pasteurella multocida
<400> 37
   atgtcgacga atcaagaaac gcgtggtttt caatcagaag tcaaacaact tcttcaacta      60
   atgatccatt ctctctattc caataaagaa attttcttac gtgaattaat ttccaatgcc     120
   tctgatgcgg cagataaatt gcgttttaaa gccttgtctg tgccagagct ttatgaaggt     180
   gatggggatt taaaagtgcg tattcgtttt gatgaagaga aaggtacctt aaccattagt     240
   gataatggca ttgggatgac gcgtgatgaa gtaatcgatc atttaggtac cattgccaaa     300
   tcgggtacca aagaattttt aagtgcatta ggacaagatc aagccaaaga tagccaatta     360
   attggtcagt ttggggtcgg tttttattcc gcctttattg tggcagataa agtcactgtg     420
   aaaacgcgtg cagcaggcgt aagtgcagat aaagcggtgc tttgggaatc ggcaggcgaa     480
   ggtgagtatt ctgtggcgga tattgacaaa aaagaacgcg gtaccgaaat tacccttcac     540
   ttacgtgaag atgaaaaagc ctttttaaat gattggcgct tacgtgaaat tatcggcaaa     600
   tattcggatc atattggttt gccagtagaa attctagcca aagaatatga cgatgaaggc     660
   aaagaaaccg gcattaaatg ggaaaaaatc aataaagcgc aagccttgtg gacacgtgca     720
   aaaaatgaga tttcggagga agaatatcaa gagttctata agcatttaag tcatgatttt     780
   accgatccgt tactttgggc acacaataaa gtagaaggaa atcaagaata taccagttta     840
   ctttatgtgc cagcaaaagc cccttgggat ttatttaatc gcgaacataa acacggctta     900
   aagctgtatg tgcaacgtgt ctttattatg gatgatgcgc aagtctttat gccaaattat     960
   ctgcgtttta tgcgtggttt attagattcc aatgatttgc cactgaatgt atcgcgcgaa    1020
   attttacaag ataacaaagt cacgagtgct ttacgtaaag ccctaacgaa acgtgcattg    1080
   caaatgctcg aaaaattagc caaagacgat gcagagaaat accaacgctt ttggcaagag    1140
   tttggtttgg tgttaaaaga aggtccagca gaagattttg caaataaaga aacgattgca    1200
   aaattattac gttttgcttc aacacacaat gacagcagcc aacaaagcgt gtcgttagaa    1260
   gactatgtgg cacgtatgaa agaaggacaa aaggcgattt attatattac ggcagatact    1320
   tatgtcgccg cgaaaaactc accgcactta gaattgttca ataagaaagg cattgaagta    1380
   ttattgttgt ccgatcgtat tgatgaatgg atgttaagct acttaacgga atttgatggt    1440
   aagccactgc aaaccatcag taaagcggat ttagatctag gtgatttagc ggataaagag    1500
   gaagacagtc aaaaagcaca agatgagcaa tatgcttctt ttgtggaacg tgtgaaaacc    1560
   ttgcttggcg agcgcgtgaa agaagtgcgc ttaactcacc gtttaacgga tacgccagcg    1620
   gttgtttcga cgggtgatga ccagatgacc acccaaatgg cgaaattgtt cgctgcggcg    1680
   ggtcaagcga tgccagaggt taaatacacc ttcgaattaa atccagaaca tggtttagta    1740
   caaaaagtag cagaaattgc cgatgagcag caatttgccg attggattga attgctactt    1800
   gaacaagcaa tgttggctga gcgtggtagc cttgaaaatc cagttgcctt tattaaacgc    1860
   atgaacacct tgttaagtaa actcacaagt cattaa                              1896
<210> 38
   <211> 631
   <212> PRT
   <213> Pasteurella multocida
<400> 38
   Met Ser Thr Asn Gln Glu Thr Arg Gly Phe Gln Ser Glu Val Lys Gln
   1 5 10 15
   Leu Leu Gln Leu Met Ile His Ser Leu Tyr Ser Asn Lys Glu Ile Phe
   20 25 30
   Leu Arg Glu Leu Ile Ser Asn Ala Ser Asp Ala Ala Asp Lys Leu Arg
   35 40 45
   Phe Lys Ala Leu Ser Val Pro Glu Leu Tyr Glu Gly Asp Gly Asp Leu
   50 55 60
   Lys Val Arg Ile Arg Phe Asp Glu Glu Lys Gly Thr Leu Thr Ile Ser
   65 70 75 80
   Asp Asn Gly Ile Gly Met Thr Arg Asp Glu Val Ile Asp His Leu Gly
   85 90 95
   Thr Ile Ala Lys Ser Gly Thr Lys Glu Phe Leu Ser Ala Leu Gly Gln
   100 105 110
   Asp Gln Ala Lys Asp Ser Gln Leu Ile Gly Gln Phe Gly Val Gly Phe
   115 120 125
   Tyr Ser Ala Phe Ile Val Ala Asp Lys Val Thr Val Lys Thr Arg Ala
   130 135 140
   Ala Gly Val Ser Ala Asp Lys Ala Val Leu Trp Glu Ser Ala Gly Glu
   145 150 155 160
   Gly Glu Tyr Ser Val Ala Asp Ile Asp Lys Lys Glu Arg Gly Thr Glu
   165 170 175
   Ile Thr Leu His Leu Arg Glu Asp Glu Lys Ala Phe Leu Asn Asp Trp
   180 185 190
   Arg Leu Arg Glu Ile Ile Gly Lys Tyr Ser Asp His Ile Gly Leu Pro
   195 200 205
   Val Glu Ile Leu Ala Lys Glu Tyr Asp Asp Glu Gly Lys Glu Thr Gly
   210 215 220
   Ile Lys Trp Glu Lys Ile Asn Lys Ala Gln Ala Leu Trp Thr Arg Ala
   225 230 235 240
   Lys Asn Glu Ile Ser Glu Glu Glu Tyr Gln Glu Phe Tyr Lys His Leu
   245 250 255
   Ser His Asp Phe Thr Asp Pro Leu Leu Trp Ala His Asn Lys Val Glu
   260 265 270
   Gly Asn Gln Glu Tyr Thr Ser Leu Leu Tyr Val Pro Ala Lys Ala Pro
   275 280 285
   Trp Asp Leu Phe Asn Arg Glu His Lys His Gly Leu Lys Leu Tyr Val
       290                 295                 300
   Gln Arg Val Phe Ile Met Asp Asp Ala Gln Val Phe Met Pro Asn Tyr
   305                 310                 315                 320
   Leu Arg Phe Met Arg Gly Leu Leu Asp Ser Asn Asp Leu Pro Leu Asn
                   325                 330                 335
   Val Ser Arg Glu Ile Leu Gln Asp Asn Lys Val Thr Ser Ala Leu Arg
               340                 345                 350
   Lys Ala Leu Thr Lys Arg Ala Leu Gln Met Leu Glu Lys Leu Ala Lys
           355                 360                 365
   Asp Asp Ala Glu Lys Tyr Gln Arg Phe Trp Gln Glu Phe Gly Leu Val
       370                 375                 380
   Leu Lys Glu Gly Pro Ala Glu Asp Phe Ala Asn Lys Glu Thr Ile Ala
   385                 390                 395                 400
   Lys Leu Leu Arg Phe Ala Ser Thr His Asn Asp Ser Ser Gln Gln Ser
                   405                 410                 415
   Val Ser Leu Glu Asp Tyr Val Ala Arg Met Lys Glu Gly Gln Lys Ala
               420                 425                 430
   Ile Tyr Tyr Ile Thr Ala Asp Thr Tyr Val Ala Ala Lys Asn Ser Pro
           435                 440                 445
   His Leu Glu Leu Phe Asn Lys Lys Gly Ile Glu Val Leu Leu Leu Ser
       450                 455                 460
   Asp Arg Ile Asp Glu Trp Met Leu Ser Tyr Leu Thr Glu Phe Asp Gly
   465                 470                 475                 480
   Lys Pro Leu Gln Thr Ile Ser Lys Ala Asp Leu Asp Leu Gly Asp Leu
                   485                 490                 495
   Ala Asp Lys Glu Glu Asp Ser Gln Lys Ala Gln Asp Glu Gln Tyr Ala
               500                 505                 510
   Ser Phe Val Glu Arg Val Lys Thr Leu Leu Gly Glu Arg Val Lys Glu
           515                 520                 525
   Val Arg Leu Thr His Arg Leu Thr Asp Thr Pro Ala Val Val Ser Thr
       530                 535                 540
   Gly Asp Asp Gln Met Thr Thr Gln Met Ala Lys Leu Phe Ala Ala Ala
   545                 550                 555                 560
   Gly Gln Ala Met Pro Glu Val Lys Tyr Thr Phe Glu Leu Asn Pro Glu
                   565                 570                 575
   His Gly Leu Val Gln Lys Val Ala Glu Ile Ala Asp Glu Gln Gln Phe
               580                 585                 590
   Ala Asp Trp Ile Glu Leu Leu Leu Glu Gln Ala Met Leu Ala Glu Arg
           595                 600                 605
   Gly Ser Leu Glu Asn Pro Val Ala Phe Ile Lys Arg Met Asn Thr Leu
       610                 615                 620
   Leu Ser Lys Leu Thr Ser His
   625                 630
<210> 39
   <211> 252
   <212> DNA
   <213> Pasteurella multocida
<400> 39
   gagttggttc agaatatatt gctcagtatg gcaatgtgag tcttactata caaaatggta     60
   aaattcatgg tgagatttat aggcataacc gagggtacga tgatctattt aagctctctg    120
   gagaaggccg gaatttaata ttaacgccac ataaaaataa ccctcatgat ctttccccaa    180
   caggacccga caacatgaca atggagctga attttatcaa cgcagaaaag actgataaaa    240
   aatacgttgt tc                                                        252
<210> 40
   <211> 1008
   <212> DNA
   <213> Pasteurella multocida
<400> 40
   atgaaacaaa tcgttttaaa aacaagctta ttgatgaccc tctcttcatt attagttgca      60
   tgtagcggcg gtggcggtag cgctggaaat cgtgctgacc gtgtagagga aaaagcacaa     120
   ccggttcaat caaatagtga gccttcttcc gctccaatca aaaatcctac taataccgct     180
   acgaatgatt ctcttcatga caaactttca atgtcttctc atgacacatc caaagaaaat     240
   agtcaacaat cctcctttaa agcccctcta gaacaagaaa aaaaccaacc tgcacaagaa     300
   aatctcactt ggacaggtta tcatgtttca gaagtgggaa atgcgagtaa taatgtagat     360
   aaagataacg ttacggtatt cactttcgta aaatataatt ctcaatacaa tgatgatcca     420
   gtttttgata aaacaaaaac acaaagtaaa acaatatcat tagttgacgg aaaaaatgag     480
   aataaagagg attattataa ctttacgtta aaagacgctt tattttatta tggaagttat     540
   ggacaacctt cagcagatta caaaaaagta gaaaaaaatt atatttatgc aattaaacca     600
   gatgcaataa ataatgagaa cctcaatgca ctaactgcaa cttattatca agaagatggt     660
   tttatatatt ccgtattaag tgatgtaaat cgagttggtt cagaatatat tcctcagtat     720
   ggcaatgtga ctcttacttt ccgaaatggc aagatttatg gtgaaatcta cagatataat     780
   agaggacgtg atgatttgtt tcagctctca ggagaaggac aaaacttaac tataacacca     840
   cacaaggaca atccccataa actatcccct acaggacccg acaacatggc aatggagctg     900
   aattttatca acgcagaaaa aactgataaa aaatacgttg ttggtgtagg aaaagctgaa     960
   aaatattatg ggttattatt tgctgaaaaa agtcaccaag cacaataa                 1008
<210> 41
   <211> 335
   <212> PRT
   <213> Pasteurella multocida
<400> 41
   Met Lys Gln Ile Val Leu Lys Thr Ser Leu Leu Met Thr Leu Ser Ser
   1               5                   10                  15
   Leu Leu Val Ala Cys Ser Gly Gly Gly Gly Ser Ala Gly Asn Arg Ala
               20                  25                  30
   Asp Arg Val Glu Glu Lys Ala Gln Pro Val Gln Ser Asn Ser Glu Pro
           35                  40                  45
   Ser Ser Ala Pro Ile Lys Asn Pro Thr Asn Thr Ala Thr Asn Asp Ser
       50                  55                  60
   Leu His Asp Lys Leu Ser Met Ser Ser His Asp Thr Ser Lys Glu Asn
   65                  70                  75                  80
   Ser Gln Gln Ser Ser Phe Lys Ala Pro Leu Glu Gln Glu Lys Asn Gln
                   85                  90                  95
   Pro Ala Gln Glu Asn Leu Thr Trp Thr Gly Tyr His Val Ser Glu Val
               100                 105                 110
   Gly Asn Ala Ser Asn Asn Val Asp Lys Asp Asn Val Thr Val Phe Thr
           115                 120                 125
   Phe Val Lys Tyr Asn Ser Gln Tyr Asn Asp Asp Pro Val Phe Asp Lys
       130                 135                 140
   Thr Lys Thr Gln Ser Lys Thr Ile Ser Leu Val Asp Gly Lys Asn Glu
   145                 150                 155                 160
   Asn Lys Glu Asp Tyr Tyr Asn Phe Thr Leu Lys Asp Ala Leu Phe Tyr
                   165                 170                 175
   Tyr Gly Ser Tyr Gly Gln Pro Ser Ala Asp Tyr Lys Lys Val Glu Lys
               180                 185                 190
   Asn Tyr Ile Tyr Ala Ile Lys Pro Asp Ala Ile Asn Asn Glu Asn Leu
           195                 200                     205
   Asn Ala Leu Thr Ala Thr Tyr Tyr Gln Glu Asp Gly Phe Ile Tyr Ser
       210                 215                 220
   Val Leu Ser Asp Val Asn Arg Val Gly Ser Glu Tyr Ile Pro Gln Tyr
   225                 230                 235                 240
   Gly Asn Val Thr Leu Thr Phe Arg Asn Gly Lys Ile Tyr Gly Glu Ile
                   245                 250                 255
   Tyr Arg Tyr Asn Arg Gly Arg Asp Asp Leu Phe Gln Leu Ser Gly Glu
               260                 265                 270
   Gly Gln Asn Leu Thr Ile Thr Pro His Lys Asp Asn Pro His Lys Leu
           275                 280                 285
   Ser Pro Thr Gly Pro Asp Asn Met Ala Met Glu Leu Asn Phe Ile Asn
       290                 295                 300
   Ala Glu Lys Thr Asp Lys Lys Tyr Val Val Gly Val Gly Lys Ala Glu
   305                 310                 315                 320
   Lys Tyr Tyr Gly Leu Leu Phe Ala Glu Lys Ser His Gln Ala Gln
                   325                 330                 335
<210> 42
   <211> 546
   <212> DNA
   <213> Pasteurella multocida
<220>
   <221> misc_feature
   <222> (428)..(428)
   <223> A or C or G or T
<220>
   <221> misc_feature
   <222> (470) .. (470)
   <223> A or C or G or T
<220>
   <221> misc_feature
   <222> (497)..(497)
   <223> A or C or G or T
<220>
   <221> misc_feature
   <222> (499)..(499)
   <223> A or C or G or T
<220>
   <221> misc_feature
   <222> (504)..(504)
   <223> A or C or G or T
<220>
   <221> misc_feature
   <222> (507)..(507)
   <223> A or C or G or T
<220>
   <221> misc_feature .
   <222> (512)..(512)
   <223> A or C or G or T
<220>
   <221> misc_feature
   <222> (519)..(519)
   <223> A or C or G or T
<400> 42
   gcttggtatt tacagggaat ccaacctaat cccgttttta gacaggcttt taatgcaatt 60
   actgatccca aagaacaatt aattgcttta gaaggttttt ttaatttgat tctgatggat 120
   aaagaaaaaa atgttagaac aacaacgtaa tcctgctgat gcactaactg tatcagtgtt 180
   aaattcacaa tctcaagtca caaataaacc attgcgtgat tctgtgaaac aagcattgag 240
   aaattatttg tcgcagttag atggccaaga tgtcaatgag ctttatgaat tagtattagc 300
   agaagttgag catcctatgt tagatatggt tatgcaatat acacgtggaa atcaaactcg 360
   tgcagcgaca atgttaggga ttaaccgtgg cactttacgt aagaaattaa aaaagtacgg 420
   tatgggtnaa cggaccattg tagtatttaa actagttttg gttatagaan ggcggactta 480
   ggtccgcctt tttaatntnc attnccnttt cntttttcna aacaatgatt tttacgccct 540
   caaatg 546
<210> 43
   <211> 1005
   <212> DNA
   <213> Pasteurella multocida
<400> 43
   atgacagtgc ggataggttc ttatcagctt aaaaatcgca tttttcttgc tcctatggct 60
   ggcatcactg accaaccatt tcggcgaatc tgcactcatt atggggcagg tttaactttt 120
   tctgaaatga tgtcaacgaa tccgcaagtc tggcataccg aaaaatcgaa actgcgcttg 180
   gctcatcatc aagaagcagg aattaatgct gtgcaaatag ctggttgtga tcccgatgag 240
   atggcgaaag ctgctcaaat caatgtagaa tatggggcag aaattattga tatcaatatg 300
   ggctgcccag ccaaaaaagt gaatcgtaaa atggcgggct ctgcgctgtt acaatatcct 360
   gatttggtca aacaaattct taataaagtt gtgaaatctg ttactgtacc agtgacatta 420
   aagataagaa caggctggga taaagacaac cgaaattgtt tagaaatcgc taaaattgca 480
   gagcaatgtg gtattcaagc actgaccatc cacggacgaa caaggagttg tatgtttgag 540
   ggggaggctg aatatgacaa tatcaaggcg gtcaaagagc aactttctat tccgattatt 600
   gccaatggcg atattacttc cgctgaaaaa gcaaagtatg ttcttgatta taccaacgca 660
   gatgcaataa tgatcggacg tggttcatta ggcaatccgt ggcttttccg agttatggaa 720
   agcttaattg aaaaagactc gattgtttta gagccaagtt taaacgagaa atgtaatgtg 780
   attttacagc atatccaaga actgcatcaa ttttatggtg tggagaaagg atgtcgtatt 840
   gcacgtaaac acgttgcttg gtatttacag ggaatccaac ctaatcccgt ttttagacag 900
   gcttttaatg caattactga tcccaaagaa caattaattg ctttagaagg tttttttaat 960
   ttgattctga tggataaaga aaaaaatgtt agaacaacaa cgtaa 1005
<210> 44
   <211> 334
   <212> PRT
   <213> Pasteurella multocida
<400> 44
   Met Thr Val Arg Ile Gly Ser Tyr Gln Leu Lys Asn Arg Ile Phe Leu
   1               5                   10                  15
   Ala Pro Met Ala Gly Ile Thr Asp Gln Pro Phe Arg Arg Ile Cys Thr
               20                  25                  30
   His Tyr Gly Ala Gly Leu Thr Phe Ser Glu Met Met Ser Thr Asn Pro
           35                  40                  45
   Gln Val Trp His Thr Glu Lys Ser Lys Leu Arg Leu Ala His His Gln
       50                  55                  60
   Glu Ala Gly Ile Asn Ala Val Gln Ile Ala Gly Cys Asp Pro Asp Glu
   65                  70                  75                  80
   Met Ala Lys Ala Ala Gln Ile Asn Val Glu Tyr Gly Ala Glu Ile Ile
                   85                  90                  95
   Asp Ile Asn Met Gly Cys Pro Ala Lys Lys Val Asn Arg Lys Met Ala
               100                 105                 110
   Gly Ser Ala Leu Leu Gln Tyr Pro Asp Leu Val Lys Gln Ile Leu Asn
           115                 120                 125
   Lys Val Val Lys Ser Val Thr Val Pro Val Thr Leu Lys Ile Arg Thr
       130                 135                 140
   Gly Trp Asp Lys Asp Asn Arg Asn Cys Leu Glu Ile Ala Lys Ile Ala
   145                 150                 155                 160
   Glu Gln Cys Gly Ile Gln Ala Leu Thr Ile His Gly Arg Thr Arg Ser
                   165                 170                 175
   Cys Met Phe Glu Gly Glu Ala Glu Tyr Asp Asn Ile Lys Ala Val Lys
               180                 185                 190
   Glu Gln Leu Ser Ile Pro Ile Ile Ala Asn Gly Asp Ile Thr Ser Ala
           195                 200                 205
   Glu Lys Ala Lys Tyr Val Leu Asp Tyr Thr Asn Ala Asp Ala Ile Met
       210                 215                 220
   Ile Gly Arg Gly Ser Leu Gly Asn Pro Trp Leu Phe Arg Val Met Glu
   225                 230                 235                 240
   Ser Leu Ile Glu Lys Asp Ser Ile Val Leu Glu Pro Ser Leu Asn Glu
                   245                 250                 255
   Lys Cys Asn Val Ile Leu Gln His Ile Gln Glu Leu His Gln Phe Tyr
               260                 265                 270
   Gly Val Glu Lys Gly Cys Arg Ile Ala Arg Lys His Val Ala Trp Tyr
           275                 280                 285
   Leu Gln Gly Ile Gln Pro Asn Pro Val Phe Arg Gln Ala Phe Asn Ala
       290                 295                 300
   Ile Thr Asp Pro Lys Glu Gln Leu Ile Ala Leu Glu Gly Phe Phe Asn
   305                 310                 315                 320
   Leu Ile Leu Met Asp Lys Glu Lys Asn Val Arg Thr Thr Thr
                   325                 330
<210> 45
   <211> 43
   <212> DNA
   <213> Pasteurella multocida
<400> 45
   gcaaaatttt tggggatggt ctgatcctaa tgcaattcaa ata 43
<210> 46
   <211> 1869
   <212> DNA
   <213> Pasteurella multocida
<400> 46
   atgaaaaagg ttattatcat tggacataaa cagtctaact atcaagatgt tgaaaaggtt     60
   tttcaatgtt atgggatgaa tcccccgctt ccatcaaaac gtgaaaaaat gtcccccatc    120
   gaaattggcc atgtacttaa taaagtatta ccaagtcttg agcacacacc taaaaatgta    180
   tctttacttt ctaataagaa aagcaaaata aaaaaaggga attcagccaa aaataaatct    240
   cataagcacg ctaaaacgaa cacaatacaa acgacttcga gcatctggga taacttatct    300
   ctcgatttga tgctcgcgaa tatcgagcaa aatttttggg gatggtctga tcctaatgca    360
   attcaaatat tagattattg ggctaacctt gacccaaaca ttcatttcgt ctttgtttat    420
   gataagccag aaaatttatt ccaatatcat agcttagaag aggctctcaa attagataaa    480
   cacaccgtac aagaaaaatt tgaagagtgg caaacctaca atgaaaaaat cctaacttac    540
   tttaataaat ataaagatcg tagtgtatta ctgaatacac aacaactcca aaatacgaaa    600
   aaaacatcac tgtctgaaat ttataaacat atttctgcac ctgatgcatt agtcaaaaaa    660
   ctgaatgaac cttctctaaa taaagagatg gaaattattg aagtaaacca agatttatct    720
   caccaagaag aatgtccact gtctaacttt attgttagcc aaattataaa aaattctcct    780
   actgttacgc aggtatatga agaattacag tcgcatgctg atctgcctta tatttcagaa    840
   caaaaattag taaatgatgc cgattttgct ctccttgcat ggaaagatat gattcaaaaa    900
   aaagtcgatg taaatcaata tcaacatgaa aaagaattag aacttagcac aataaaagaa    960
   cgtcaattag aggtcacaga gagatatcaa ttgacggaac aaaaactgtc agaaacacaa   1020
   aaagaaatcg aacaaattaa agatgaaaat agaaaagtaa aatctgaaaa agcaaaactc   1080
   actgcatctg ttcaatcaac gagcaaaata ctttctgaga aagaaaaaga gatttcttgc   1140
   ataaaaagtg aaaatacaaa gattaaagaa gaaaaaatta aaattgatga agcataccac   1200
   ttaaccaaga aaaccttgtc ggataaagaa aaagccctca aaacgcatca agatgaaatt   1260
   gaagcgctca agataatttt taatgaaaat atttccgtac aagaagatat gcaagaaaaa   1320
   tttcaggaag ccaataaaag aaaacaagaa cttgaacaag agctaaaagc catatcggat   1380
   aagaaagcat tattagaaac agaaaacagc caaaaaaccc aagtatctga gtctttagaa   1440
   aatgaaaata aagtgttatt agctcaactc caactcattc aagaagaatt agaaaaactt   1500
   tatattgaca atcaagtatt aaaagctaaa ccacgccttt acggtgcagc tgatcgcata   1560
   aaaaaccaat taacttatcg actaggttac aaaatacaaa gacatggaag aagtctattt   1620
   ggtctcattt ttcttccttt catcttattt ttcacctatc tgggctttaa aagagagatg   1680
   aaaaagtacg agtggaatac gctcccacca attcatgaat atgaagatgc gcatgaagcc   1740
   aatcgcatta aaagccattt atcttataaa ttgggcgtcc tctttttgca agaaatcaac   1800
   aatccgttta agtggcttac tctcccttat aaactgatta aagaaggtaa acgattcaag   1860
   caaggttaa                                                          1869
<210> 47
   <211> 622
   <212> PRT
   <213> Pasteurella multocida
<400> 47
   Met Lys Lys Val Ile Ile Ile Gly His Lys Gln Ser Asn Tyr Gln Asp
   1               5                   10                  15
   Val Glu Lys Val Phe Gln Cys Tyr Gly Met Asn Pro Pro Leu Pro Ser
               20                  25                  30
   Lys Arg Glu Lys Met Ser Pro Ile Glu Ile Gly His Val Leu Asn Lys
           35                  40                  45
   Val Leu Pro Ser Leu Glu His Thr Pro Lys Asn Val Ser Leu Leu Ser
       50                  55                  60
   Asn Lys Lys Ser Lys Ile Lys Lys Gly Asn Ser Ala Lys Asn Lys Ser
   65                  70                  75                  80
   His Lys His Ala Lys Thr Asn Thr Ile Gln Thr Thr Ser Ser Ile Trp
                   85                  90                  95
   Asp Asn Leu Ser Leu Asp Leu Met Leu Ala Asn Ile Glu Gln Asn Phe
               100                 105                 110
   Trp Gly Trp Ser Asp Pro Asn Ala Ile Gln Ile Leu Asp Tyr Trp Ala
           115                 120                 125
   Asn Leu Asp Pro Asn Ile His Phe Val Phe Val Tyr Asp Lys Pro Glu
       130                 135                 140
   Asn Leu Phe Gln Tyr His Ser Leu Glu Glu Ala Leu Lys Leu Asp Lys
   145                 150                 155                 160
   His Thr Val Gln Glu Lys Phe Glu Glu Trp Gln Thr Tyr Asn Glu Lys
   165 170 175
   Ile Leu Thr Tyr Phe Asn Lys Tyr Lys Asp Arg Ser Val Leu Leu Asn
   180 185 190
   Thr Gln Gln Leu Gln Asn Thr Lys Lys Thr Ser Leu Ser Glu Ile Tyr
   195 200 205
   Lys His Ile Ser Ala Pro Asp Ala Leu Val Lys Lys Leu Asn Glu Pro
   210 215 220
   Ser Leu Asn Lys Glu Met Glu Ile Ile Glu Val Asn Gln Asp Leu Ser
   225 230 235 240
   His Gln Glu Glu Cys Pro Leu Ser Asn Phe Ile Val Ser Gln Ile Ile
   245 250 255
   Lys Asn Ser Pro Thr Val Thr Gln Val Tyr Glu Glu Leu Gln Ser His
   260 265 270
   Ala Asp Leu Pro Tyr Ile Ser Glu Gln Lys Leu Val Asn Asp Ala Asp
   275 280 285
   Phe Ala Leu Leu Ala Trp Lys Asp Met Ile Gln Lys Lys Val Asp Val
   290 295 300
   Asn Gln Tyr Gln His Glu Lys Glu Leu Glu Leu Ser Thr Ile Lys Glu
   305 310 315 320
   Arg Gln Leu Glu Val Thr Glu Arg Tyr Gln Leu Thr Glu Gln Lys Leu
   325 330 335
   Ser Glu Thr Gln Lys Glu Ile Glu Gln Ile Lys Asp Glu Asn Arg Lys
   340 345 350
   Val Lys Ser Glu Lys Ala Lys Leu Thr Ala Ser Val Gln Ser Thr Ser
   355 360 365
   Lys Ile Leu Ser Glu Lys Glu Lys Glu Ile Ser Cys Ile Lys Ser Glu
   370 375 380
   Asn Thr Lys Ile Lys Glu Glu Lys Ile Lys Ile Asp Glu Ala Tyr His
   385 390 395 400
   Leu Thr Lys Lys Thr Leu Ser Asp Lys Glu Lys Ala Leu Lys Thr His
   405 410 415
   Gln Asp Glu Ile Glu Ala Leu Lys Ile Ile Phe Asn Glu Asn Ile Ser
   420 425 430
   Val Gln Glu Asp Met Gln Glu Lys Phe Gln Glu Ala Asn Lys Arg Lys
   435 440 445
   Gln Glu Leu Glu Gln Glu Leu Lys Ala Ile Ser Asp Lys Lys Ala Leu
   450 455 460
   Leu Glu Thr Glu Asn Ser Gln Lys Thr Gln Val Ser Glu Ser Leu Glu
   465 470 475 480
   Asn Glu Asn Lys Val Leu Leu Ala Gln Leu Gln Leu Ile Gln Glu Glu
   485 490 495
   Leu Glu Lys Leu Tyr Ile Asp Asn Gln Val Leu Lys Ala Lys Pro Arg
   500 505 510
   Leu Tyr Gly Ala Ala Asp Arg Ile Lys Asn Gln Leu Thr Tyr Arg Leu
   515 520 525
   Gly Tyr Lys Ile Gln Arg His Gly Arg Ser Leu Phe Gly Leu Ile Phe
   530 535 540
   Leu Pro Phe Ile Leu Phe Phe Thr Tyr Leu Gly Phe Lys Arg Glu Met
   545 550 555 560
   Lys Lys Tyr Glu Trp Asn Thr Leu Pro Pro Ile His Glu Tyr Glu Asp
   565 570 575
   Ala His Glu Ala Asn Arg Ile Lys Ser His Leu Ser Tyr Lys Leu Gly
   580 585 590
   Val Leu Phe Leu Gln Glu Ile Asn Asn Pro Phe Lys Trp Leu Thr Leu
   595 600 605
   Pro Tyr Lys Leu Ile Lys Glu Gly Lys Arg Phe Lys Gln Gly
   610 615 620
<210> 48
   <211> 279
   <212> DNA
   <213> Pasteurella multocida
<400> 48
   accgcttcct gagctacgcc aaattctcac tgccttacca gtatccgcag aacaagcaga     60
   aaatgatgat tacttaaccc attttaatcg cagccaagaa ttacttaatt ggcaacattt    120
   ttttattgcc cagcaacttg ctttcgttaa cgcattggaa aatcaagaat gaaaaaatgg    180
   ttgaaacatt tagatttgag cactggctta caactgtctt ttctgatcag tgggctactt    240
   tgtctgtttg tcggtggcgt cgggctttat acttggcac                          279
<210> 49
   <211> 1983
   <212> DNA
   <213> Pasteurella multocida
<400> 49
   atgaaaaaat ggttgaaaca tttagatttg agcactggct tacaactgtc ttttctgatc 60
   agtgggctac tttgtctgtt tgtcggtggc gtcgggcttt atacttggca gcaacaacgc 120
   acggaaatca atttcgcact cgataaagat tttcctaaag tgcaagctgc gtttcaaaca 180
   gaagaacaaa ttaatatcct ccatcatgcc tttatccatt tggtcaatgt caaaaacacc 240
   aatgagaaag tcgaacgtta caaccatgca aagcaacagc tttcgacgtt aaaagaactg 300
   atcattgaat tagacgaaaa tttagatgag gatttgatgg cattattaca acaacaagcc 360
   tcacttttag aacaaatatc acaaaatatc acaggtacgc ttacgttaaa cgatgaactg 420
   aataaaacca tttctcaaat caactggtta cataatgatt ttcacaatga attcaccgca 480
   cttttgcaag aaatgagctg gcaacaatct actctggcta acaatattgt tcaacagcca 540
   cacaacaaac aaaaaatcga acaattaaaa aaactacaac aagaattatt gttagtttac 600
   gatttcacta cttatgaaga gcaaattatc acggaattac gcacccagat aacagagcca 660
   actgaaagca atgtcattcg actacacaat tatttgagct atttatcgtt attaattact 720
   aaccgaattc agttgcttgg tcttcattcc tccacgtcaa ccattaaaca aattttagat 780
   gaactgatta actttggctt aaacccacaa gcactccccg ccctatttgc aatccgtacc 840
   gaactgaacc aacaacgaga acagctgatt caacaaagtg ataagatatt cgaggcattt 900
   cgcgagcaaa tcagtactca aattggtaac agtaaacaac aattacattt actgcataat 960
   attgtcgaaa aaagtactac attcaacggc gcattaattt tattggtgat gctatttgcg 1020
   ggaatttttg tcatcggtat taacttcttt tatattcgtt tacgtctctt aaaacgtttt 1080
   caacaactta accacgccgt agttcaatta accaatggcg agcccaacgt caaaatcgcc 1140
   atttatggca atgatgaatt agggcggatt gctaaattat tgcgcttatt tctgttcgaa 1200
   atgaatcaca aaacagaaga gttaaaatcg cgtaatcaag ttctcttaga ggaaatcgaa 1260
   caccgtattg aagtacaaac cgcattagaa aatgcccaaa atgaactaac ccaagccgca 1320
   aaactggctg ctgtcggtaa aaccttgact tcgattagcc atgaaattac acaaccactt 1380
   aatgccatga acgcttattt gtttagtgcg aaaaaagccg tgagtaaaca aaacagtgag 1440
   gcagcacttg aatacttaaa taaaatcaat catttagttg aacgcacggc gctgattgtc 1500
   aaacgcttac ggcaattctc acgccaaggg agcggcaaaa tacaagctgt caatttaatg 1560
   gattgtattc aaagcgcgtg ggaattattg gaatcacaac ataaaccgcg tcaaagtcag 1620
   ctcatcacgc ccacagattt accactcgta ttaggtgaag atgtccttat cgaacaagtg 1680
   tttgtcaatc tcttcctcaa tgctttagaa gccattgaac acacaccgcc ccaaattcat 1740
   attgacgttg acagcgataa tgcggaagac ctctgtttat ggatcaccga caatggtcaa 1800
   ggttggccct taactgacaa gttattgcaa cctttttcga gcagtaaatc gatcaattta 1860
   ggtttaggac tgtccattag tcaatccatc atggagcaat gtcaaggatc attgaccatt 1920
   gcctctactc tcacccataa tgcattagtg atattaaaat ttaaggtggc tcaacatgtt 1980
   taa 1983
<210> 50
   <211> 660
   <212> PRT
   <213> Pasteurella multocida
<400> 50
   Met Lys Lys Trp Leu Lys His Leu Asp Leu Ser Thr Gly Leu Gln Leu
   1               5                   10                  15
   Ser Phe Leu Ile Ser Gly Leu Leu Cys Leu Phe Val Gly Gly Val Gly
               20                  25                  30
   Leu Tyr Thr Trp Gln Gln Gln Arg Thr Glu Ile Asn Phe Ala Leu Asp
           35                  40                  45
   Lys Asp Phe Pro Lys Val Gln Ala Ala Phe Gln Thr Glu Glu Gln Ile
       50                  55                  60
   Asn Ile Leu His His Ala Phe Ile His Leu Val Asn Val Lys Asn Thr
   65                  70                  75                  80
   Asn Glu Lys Val Glu Arg Tyr Asn His Ala Lys Gln Gln Leu Ser Thr
                   85                  90                  95
   Leu Lys Glu Leu Ile Ile Glu Leu Asp Glu Asn Leu Asp Glu Asp Leu
               100                 105                 110
   Met Ala Leu Leu Gln Gln Gln Ala Ser Leu Leu Glu Gln Ile Ser Gln
           115                 120                 125
   Asn Ile Thr Gly Thr Leu Thr Leu Asn Asp Glu Leu Asn Lys Thr Ile
       130                 135                 140
   Ser Gln Ile Asn Trp Leu His Asn Asp Phe His Asn Glu Phe Thr Ala
   145                 150                 155                 160
   Leu Leu Gln Glu Met Ser Trp Gln Gln Ser Thr Leu Ala Asn Asn Ile
                   165                 170                 175
   Val Gln Gln Pro His Asn Lys Gln Lys Ile Glu Gln Leu Lys Lys Leu
               180                 185                 190
   Gln Gln Glu Leu Leu Leu Val Tyr Asp Phe Thr Thr Tyr Glu Glu Gln
           195                 200                 205
   Ile Ile Thr Glu Leu Arg Thr Gln Ile Thr Glu Pro Thr Glu Ser Asn
       210                 215                 220
   Val Ile Arg Leu His Asn Tyr Leu Ser Tyr Leu Ser Leu Leu Ile Thr
   225                 230                 235                 240
   Asn Arg Ile Gln Leu Leu Gly Leu His Ser Ser Thr Ser Thr Ile Lys
                   245                 250                 255
   Gln Ile Leu Asp Glu Leu Ile Asn Phe Gly Leu Asn Pro Gln Ala Leu
               260                 265                 270
   Pro Ala Leu Phe Ala Ile Arg Thr Glu Leu Asn Gln Gln Arg Glu Gln
           275                 280                 285
   Leu Ile Gln Gln Ser Asp Lys Ile Phe Glu Ala Phe Arg Glu Gln Ile
       290                 295                 300
   Ser Thr Gln Ile Gly Asn Ser Lys Gln Gln Leu His Leu Leu His Asn
   305                 310                 315                 320
   Ile Val Glu Lys Ser Thr Thr Phe Asn Gly Ala Leu Ile Leu Leu Val
                   325                 330                 335
   Met Leu Phe Ala Gly Ile Phe Val Ile Gly Ile Asn Phe Phe Tyr Ile
               340                 345                 350
   Arg Leu Arg Leu Leu Lys Arg Phe Gln Gln Leu Asn His Ala Val Val
           355                 360                 365
   Gln Leu Thr Asn Gly Glu Pro Asn Val Lys Ile Ala Ile Tyr Gly Asn
       370                 375                 380
   Asp Glu Leu Gly Arg Ile Ala Lys Leu Leu Arg Leu Phe Leu Phe Glu
   385                 390                 395                 400
   Met Asn His Lys Thr Glu Glu Leu Lys Ser Arg Asn Gln Val Leu Leu
                   405                 410                 415
   Glu Glu Ile Glu His Arg Ile Glu Val Gln Thr Ala Leu Glu Asn Ala
               420                 425                 430
   Gln Asn Glu Leu Thr Gln Ala Ala Lys Leu Ala Ala Val Gly Lys Thr
           435                 440                 445
   Leu Thr Ser Ile Ser His Glu Ile Thr Gln Pro Leu Asn Ala Met Asn
       450                 455                 460
   Ala Tyr Leu Phe Ser Ala Lys Lys Ala Val Ser Lys Gln Asn Ser Glu
   465                 470                 475                 480
   Ala Ala Leu Glu Tyr Leu Asn Lys Ile Asn His Leu Val Glu Arg Thr
                   485                 490                 495
   Ala Leu Ile Val Lys Arg Leu Arg Gln Phe Ser Arg Gln Gly Ser Gly
               500                 505                 510
   Lys Ile Gln Ala Val Asn Leu Met Asp Cys Ile Gln Ser Ala Trp Glu
           515                 520                 525
   Leu Leu Glu Ser Gln His Lys Pro Arg Gln Ser Gln Leu Ile Thr Pro
       530                 535                 540
   Thr Asp Leu Pro Leu Val Leu Gly Glu Asp Val Leu Ile Glu Gln Val
   545                 550                 555                 560
   Phe Val Asn Leu Phe Leu Asn Ala Leu Glu Ala Ile Glu His Thr Pro
                   565                 570                 575
   Pro Gln Ile His Ile Asp Val Asp Ser Asp Asn Ala Glu Asp Leu Cys
               580                 585                 590
   Leu Trp Ile Thr Asp Asn Gly Gln Gly Trp Pro Leu Thr Asp Lys Leu
           595                 600                 605
   Leu Gln Pro Phe Ser Ser Ser Lys Ser Ile Asn Leu Gly Leu Gly Leu
       610                 615                 620
   Ser Ile Ser Gln Ser Ile Met Glu Gln Cys Gln Gly Ser Leu Thr Ile
   625                 630                 635                 640
   Ala Ser Thr Leu Thr His Asn Ala Leu Val Ile Leu Lys Phe Lys Val
                   645                 650                 655
   Ala Gln His Val
               660
<210> 51
   <211> 93
   <212> DNA
   <213> Pasteurella multocida
<400> 51
   atagaaatgg tttatttcca aatttcctca aatttcacct tggcttttaa gaattttggc      60
   gttgccacta aattacagta gctgttttgt gct                                   93
<210> 52
   <211> 525
   <212> DNA
   <213> Pasteurella multocida
<400> 52
   atgacacaac aagcgatctt tgccggcggc tgtttttggt gcgttgaggc agtatttaat      60
   caaattaaag gcgttgaaaa agcgacttca ggttatatta acgggacgac tgaaaatcca     120
   acttacaaag aagtatgtac cggtgaaacg ggtcatgcgg aagcggtaaa agtggaattc     180
   gatgcgacag tgattagtta tgaaaaatta ttagacatct tcttttctat ccataatcca     240
   acccaattaa atcaccaggg cgaagatgtg ggaacgcaat atcgcacagg gatttactat     300
   ttaaatgatg aacaagaaca gctggcaaat aagaaaattg cagaattaca accgcacttt     360
   gccgaaaaaa ttgtcactga agtgctgcca gcacaaactt tttatcccgc agaagattat     420
   caccaaggct atttattgca gaacccacaa aacagctact gtaatttagt ggcaacgcca     480
   aaattcttaa aagccaaggt gaaatttgag gaaatttgga agtaa                     525
<210> 53
   <211> 174
   <212> PRT
   <213> Pasteurella multocida
<400> 53
   Met Thr Gln Gln Ala Ile Phe Ala Gly Gly Cys Phe Trp Cys Val Glu
   1               5                   10                  15
   Ala Val Phe Asn Gln Ile Lys Gly Val Glu Lys Ala Thr Ser Gly Tyr
               20                  25                  30
   Ile Asn Gly Thr Thr Glu Asn Pro Thr Tyr Lys Glu Val Cys Thr Gly
           35                  40                  45
   Glu Thr Gly His Ala Glu Ala Val Lys Val Glu Phe Asp Ala Thr Val
       50                  55                  60
   Ile Ser Tyr Glu Lys Leu Leu Asp Ile Phe Phe Ser Ile His Asn Pro
   65                  70                  75                  80
   Thr Gln Leu Asn His Gln Gly Glu Asp Val Gly Thr Gln Tyr Arg Thr
                   85                  90                  95
   Gly Ile Tyr Tyr Leu Asn Asp Glu Gln Glu Gln Leu Ala Asn Lys Lys
               100                 105                 110
   Ile Ala Glu Leu Gln Pro His Phe Ala Glu Lys Ile Val Thr Glu Val
           115                 120                 125
   Leu Pro Ala Gln Thr Phe Tyr Pro Ala Glu Asp Tyr His Gln Gly Tyr
       130                 135                 140
   Leu Leu Gln Asn Pro Gln Asn Ser Tyr Cys Asn Leu Val Ala Thr Pro
   145                 150                 155                 160
   Lys Phe Leu Lys Ala Lys Val Lys Phe Glu Glu Ile Trp Lys
                   165                 170
<210> 54
   <211> 772
   <212> DNA
   <213> Pasteurella multocida
<220>
   <221> misc_feature
   <222> (537)..(537)
   <223> A or C or G or T
<220>
   <221> misc_feature
   <222> (540)..(540)
   <223> A or C or G or T
<220>
   <221> misc_feature
   <222> (545)..(545)
   <223> A or C or G or T
<220>
   <221> misc_feature
   <222> (581).. (581)
   <223> A or C or G or T
<220>
   <221> misc_feature
   <222> (613).. (614)
   <223> A or C or G or T
<220>
   <221> misc_feature
   <222> (665)..(665)
   <223> A or C or G or T
<220>
   <221> misc_feature
   <222> (668)..(668)
   <223> A or C or G or T
<220>
   <221> misc_feature
   <222> (685)..(686)
   <223> A or C or G or T
<220>
   <221> misc_feature
   <222> (700) .. (704)
   <223> A or C or G or T
<220>
   <221> misc_feature
   <222> (721)..(723)
   <223> A or C or G or T
<220>
   <221> misc_feature
   <222> (732)..(732)
   <223> A or C or G or T
<220>
   <221> misc_feature
   <222> (734)..(734)
   <223> A or C or G or T
<220>
   <221> misc_feature
   <222> (737) .. (738)
   <223> A or C or G or T
<220>
   <221> (misc_feature
   <222> (748)..(748)
   <223> A or C or G or T
<220>
   <221> misc_feature
   <222> (752)..(752)
   <223> A or C or G or T
<220>
   <221> misc_feature
   <222> (755)..(755)
   <223> A or C or G or T
<220>
   <221> misc_feature
   <222> (770)..(772)
   <223> A or C or G or T
<400> 54
   gcataggtat cctttgcttg acataaaatg actacaggct aaagcacagg atattaaaag     60
   ggcaaaagaa taagttactt ttgcgacacg catcgcaaaa gtaaaataaa tttagtcaat    120
   caatttagct tgttttaaga aatactcaat gccatgttct ttgatcggta aggtgacatg    180
   atcggctact gtttttagct gatgatgtgc attccccatt gcaacaccca ctcctgccgt     240
   gcttaacatt tcaatatcat tcaagccatc accaaatgcc atcacatttt ccattgcaaa    300
   gccaaaatgt tgaattgcac aagcgatacc cgtagctttt gagatttttt catcaaataa    360
   atcaaccgag tatttatgcc agcgtaccga ttgtaatcct ttcagtacac cagaatcttg    420
   gacaaattga tcttgcgtag catcataaaa agccagtatc tgaaaaacat catgactgtt    480
   taaaatagtc tttatctaca tgataatgcc cttttagcgg atccaatgca tcacganctn    540
   gatcngttat cgctgaaact gcggtatctg tcggtgacac ntgcgcataa caatctgatg    600
   ttgatcacaa aannacgaac tctggatttt gcttagataa ggatctccga tggctatcta    660
   tactnatntg acatcatgta cacanncatg tcgtgccatn nnnnacttag cgaagtgcag    720
   nnnccgtcat cngncannca gacatcantc cnacntgcta ttaggataan nn            772
<210> 55
   <211> 687
   <211> 687
   <212> DNA
   <213> Pasteurella multocida
<400> 55
   atgcggggat ttggtttttg ttgtgttcac tgccagcaac cgttagccat tgcacatcat     60
   ggattatgta gtcgctgtaa tcagcaaatc agacgttttg cttattgtgg ccattgtggt    120
   aaggaattaa cacgagatgc actacgttgt gggcattgtt tacaacataa agccagttgg    180
   gatcgcatgg tgatcgttgg tcactatgtc gatcccttat cttgtttaat tcaccgtttt    240
   aaattccaac atgccttctt tttagaccgt actttagcac gcctgctatt attagcgctc    300
   tatcatgcaa gacgtactca tggacttatt tggccagaag tacttttgcc ggtgccttta    360
   catcgtttac gtcattggca acgtggttac aatcaatctg cgttgattgc aaactatctt    420
   gcgcattggc taaagatacc ctgcgatcat gattttctac agcgtattaa acatactcat    480
   acgcaacgtg gtttaagtgc aacggaacga agaaaaaatt tacgccacgc atttcgtctt    540
   catccaaaaa gtcaaacgca tcgctatcaa tctgttgcat taattgatga tgtaattaca    600
   acaggtgcaa cgttgaatga gttggcactc ttattaaaaa aagcaggtgt tgagcatatt    660
   caagtttggg gattagcaaa aacgtaa                                        687
<216> 56
   <211> 228
   <212> PRT
   <213> Pasteurella multocida
<400> 56
<210> 57
   <211> 700
   <212> DNA
   <213> Pasteurella multocida
<220>
   <221> misc_feature
   <222> (498)..(498)
   <223> A or C or G or T
<220>
   <221> misc_feature
   <222> (540)..(540)
   <223> A or C or G or T
<220>
   <221> misc_feature
   <222> (562)..(562)
   <223> A or C or G or T
<220>
   <221> misc_feature
   <222> (565)..(565)
   <223> A or C or G or T
<220>
   <221> misc_feature
<222> (572)..(573)
   <223> A or C or G or T
<220>
   <221> misc_feature
   <222> (577)..(577)
   <223> A or C or G or T
<220>
   <221> misc_feature
   <222> (584).. (584)
   <223> A or C or G or T
<220>
   <221> misc_feature

   <222> (587) .. (588)

   <223> A or C or G or T
<220>
   <221> misc_feature
   <222> (591)..(592)
   <223> A or C or G or T
<220>
   <221> misc_feature
   <222> (594)..(595)
   <223> A or C or G or T
<220>
   <221> misc_feature
   <222> (599) .. (599)
   <223> A or C or G or T
<220>
   <221> misce_feature
   <222> (604)..(604)
   <223> A or C or G or T
<220>

   <221> misc_feature
   <222> (619)..(619)
   <223> A or C or G or T
<220>
   <221> misc_feature
   <222> (623)..(623)
   <223> A or C or G or T
<220>
   <221> misc_feature
   <222> (625) .. (625)
   <223> A or C or G or T
<220>
   <221> misc_feature
   <222> (627)..(627)
   <223> A or C or G or T
<220>
   <221> misc_feature
   <222> (633)..(633)
   <223> A or C or G or T
<220>
   <221> misc_feature
   <222> (638)..(638)
   <223> A or C or G or T
<220>
   <221> misc_feature
   <222> (644) . . (645)
   <223> A or C or G or T
<220>
   <221> misc_feature
   <222> (653) .. (653)
   <223> A or C or G or T
<220>
   <221> misc_feature
   <222> (657)..(657)
   <223> A or C or G or T
<220>
   <221> misc_feature
   <222> (661)..(661)
   <223> A or C or G or T
<220>
   <221> misc_feature
   <222> (664)..(664)
   <223> A or C or G or T
<220>
   <221> misc_feature
   <222> (666)..(666)
   <223> A or C or G or T
<220>
   <221> misc_feature
   <222> (671) .. (671)
   <223> A or C or G or T
<220>
   <221> misc_feature
   <222> (673) .. (674)
   <223> A or C or G or T
<220>
   <221> misc_feature
   <222> (678)..(678)
   <223> A or C or G or T
<220>
   <221> misc_feature
   <222> (682) .. (682)
   <223> A or C or G or T
<220>
   <221> misc_feature
   <222> (686)..(687)
   <223> A or C or G or T
<220>
   <221> misc_feature
   <222> (694) .. (694)
   <223> A or C or G or T
<220>
   <221> misc_feature

   <222> (696) .. (697)
   <223> A or C or G or T
<220>
   <221> misc_feature
   <222> (699) .. (700) <223> A or C or G or T
<400> 57
   tgccgaccac tccaaaggac aaaaaatgag cctatttgcg attttctatc tgttcctggc      60
   gtatttatta ggatctgttt ctagtgcaat tttattgtgt cgtttagcgg ggttgcctga     120
   tcctagagaa agtggttctc ataatcccgg tgcaaccaat gtattgcgta ttggtgggcg     180
   ttgggtggca ttgagtgtac tcctgtttga tatgctcaaa ggtatgttac ctgtttggtt     240
   aggctattat cttggtttga ctcattttga gttagggatg gtggcattag gtgcttgttt     300
   agggcacatt ttcccaatct tctttaaatt taaaggcgga aaaggggtag caacggcatt     360
   tggtgctatt gcgccgattt catggggtgt cgcaggcagt atgctgggca cttggttatt     420
   gattttcttc gtgagtggtt attcttcgct cagtgcagtg atgaccgcgc ttctggtacc     480
   tttctatgtg tggtggtnta agcccgagtt tactttccct gtcgcttagt gtgttgcttn     540
   tcgattatcg ccatcatgac anatncagcg tnngtgngtg ggcnagnnga nnanngtgna     600
   atanactgaa aacaaaaang atnantnagc tanttacnaa aaanngacag acngtcnttt     660
   natncncgtt nanntatnga cntatnngat ggcntnncnn                           700
<210> 58
   <211> 606
   <212> DNA
   <213> Pasteurella multocida
<400> 58
   atgagcctat ttgcgatttt ctatctgttc ctggcgtatt tattaggatc tgtttctagt    60
   gcaattttat tgtgtcgttt agcggggttg cctgatccaa gagaaagtgg ttctcataat   120
   cccggtgcaa ccaatgtctt gcgtattggt gggcgttggg tggcattgag tgtactcctg   180
   tttgatatgc ttaaaggtat gttacctgtt tggttaggct attatcttgg tttgactcat   240
   tttgaattag ggatggtggc attaggtgct tgtttagggc acatttttcc aatcttcttt   300
   aaatttaaag gcggaaaagg ggtggcaacg gcatttggtg ctattgcgcc gatctcatgg   360
   ggtgtcgctg gcagtatgct aggcacttgg ttattgattt tcttcgtgag tggttattct   420
   tcgctcagtg cggtgatgac cgcgcttctg gtacctttct atgtgtggtg gtttaagccc   480
   gagtttactt tccctgtcgc tttagtgtgt tgcttgttga tttatcgcca tcatgacaat   540
   attcagcgtt tgtggcgtgg gcaagaagac aaagtgtgga ataaactgaa aaacaaaaaa   600
   gattaa                                                               606
<210> 59
   <211> 201
   <212> PRT
   <213> Pasteurella multocida
<400> 59
   Met Ser Leu Phe Ala Ile Phe Tyr Leu Phe Leu Ala Tyr Leu Leu Gly
   1               5                   10                  15
   Ser Val Ser Ser Ala Ile Leu Leu Cys Arg Leu Ala Gly Leu Pro Asp
               20                  25                  30
   Pro Arg Glu Ser Gly Ser His Asn Pro Gly Ala Thr Asn Val Leu Arg
           35                  40                  45
   Ile Gly Gly Arg Trp Val Ala Leu Ser Val Leu Leu Phe Asp Met Leu
       50                  55                  60
   Lys Gly Met Leu Pro Val Trp Leu Gly Tyr Tyr Leu Gly Leu Thr His
   65                  70                  75                  80
   Phe Glu Leu Gly Met Val Ala Leu Gly Ala Cys Leu Gly His Ile Phe
                   85                  90                  95
   Pro Ile Phe Phe Lys Phe Lys Gly Gly Lys Gly Val Ala Thr Ala Phe
               100                 105                 110
   Gly Ala Ile Ala Pro Ile Ser Trp Gly Val Ala Gly Ser Met Leu Gly
           115                 120                 125
   Thr Trp Leu Leu Ile Phe Phe Val Ser Gly Tyr Ser Ser Leu Ser Ala
       130                 135                 140
   Val Met Thr Ala Leu Leu Val Pro Phe Tyr Val Trp Trp Phe Lys Pro
   145                 150                 155                 160
   Glu Phe Thr Phe Pro Val Ala Leu Val Cys Cys Leu Leu Ile Tyr Arg
                   165                 170                 175
   His His Asp Asn Ile Gln Arg Leu Trp Arg Gly Gln Glu Asp Lys Val
               180                 185                 190
   Trp Asn Lys Leu Lys Asn Lys Lys Asp
           195                 200
<210> 60
   <211> 188
   <212> DNA
   <213> Pasteurella multocida
<400> 60
   atcccaccga taaaaccaaa tggattacgc gcagtttcca aataaacagg ggtagcacca 60
   gcttgaatta atgcaccatg atggatagat ttgtgattgt tacgatcaaa gagcacaaga 120
   tcacccggtg tgagtaacgc attggtgacg actttatttg cagaagatgt cccatttaag 180
   acgaagta 188
<210> 61
   <211> 2163
   <212> DNA
   <213> Pasteurella multocida
<400> 61
   atgctaaatt taaaaattgc atatagccca ttaattcgac cttattttca tacaaataga 60
   gaactcgttt ctgttcaaga aacagatttc accgacattg gtgcaattat cctgagttct 120
   gaagatattg aagattatat tgatagtata caagcaactg aatttaatat tccggtcttt 180
   gttgctgtca ttgaaggtca atttttagat cctcaattct ttgacaaagt ctatcacgtt 240
   caagatctca acaactacga catcaaccta tacagtcgcc aaattgaaac tgcggcgcgg 300
   ttttacgaag aaaaaatcct cccacctttc tttaaaatgc taagtgaata tgtggaaatg 360
   gggaattctg cttttgattg tccgggacac caaggtggac aatatttccg taaacatcct 420
   gcaggacgtt atctctatga tttctacggt gaaaatattt tccgctcaga tatctgtaat 480
   gccgatgtaa aattaggcga tttgctaatc catgaaggag ccgcttgtga tgctcaaaaa 540
   cacgctgctc aagtctttaa tgctgataaa acctacttcg tcttaaatgg gacatcttct 600
   gcaaataaag tcgtcaccaa tgcgttactc acaccgggtg atcttgtgct ctttgatcgt 660
   aacaatcaca aatctatcca tcacggtgca ttaattcaag ctggtgctac ccctgtttat 720
   ttggaaactg cgcgtaatcc atttggtttt atcggtggga tcgatagcca ttgttttgat 780
   gaagattatt tgaaatcttt aattaaagat gttgcgcctg aaaaactaac acaagcacgt 840
   cctttccgtt tagccgttat tcagctcggc acttatgacg gaaccatcta taatgcgcgc 900
   caagtcgtag ataaaattgg tcatttatgt gactacatct tgtttgattc tgcgtgggta 960
   ggttatgaac aattcattcc aatgatgaaa gattgctcac cgctcttgct tgaattaaat 1020
   gaaaatgatc ccggcatcat cgtgacacaa tcagtacaca aacaacaagc cggcttctca 1080
   caagcctcac aaattcacaa aaaagacaag cacattaaag gtcaacagcg ctactgtaat 1140
   cataaacgct ttaataatgc attcatgtta cacgcctcca ccagcccatt ctaccctctt 1200
   tttgccacac ttgatgtcaa tgcaaaaatt caaggtaccc ctgcgggtat tcgtttatgg 1260
   catgactgtg tcaaaatcgg gatagaagca cgtaaaatgg tgctgaatag ttgtgatctg 1320
   atcaaaccgt ttattccgcc ttatgtcaat ggcaaaaaat ggcaagacta cgatacagaa 1380
   gaaatggcaa atgatttaac attcttcaaa ttccatgctg atgataaatg gcatcaattt 1440
   gaaggctatg tagataacca atattttgtt gatccatgta aattcatgct aacgacgccg 1500
   ggtattgata ttgaaacagg tgaatacgaa gacttcggtg tccctgctac gattcttgct 1560
   aattatttac gtgaaaacgg cattattccg gaaaaatgtg acttaaactc aattctcttc 1620
   ttattaacgc cagcagaaac cctcaccaaa atgcaaagtt tggttgcaca aattgcggca 1680
   tttgaacaac acatcaaaaa agattcctta ctaaaagaag tcttaccaag tgtttatcac 1740
   aacaatgaaa aacgctatga aggttatacc atccgtcgtc tttgccaaga aatgcatgat 1800
   ttgtatgtca gccgtaacgt gaaaacttta caacgcaact tattcagaaa agcgaccttg 1860
   cctgaatatg tgatgaatcc acatcaagct aatcttgaat ttgttcgtaa tcgtgtagaa 1920
   ctggttccac taaccgaaat cgttaatcgc attgcggcag aaggagcact tccttatcca 1980
   ccgggtgtgc tttgtgtcgt accgggtgaa aaatggagtc agactgcaca ggaatatttc 2040
   ttagcactcg aagaaggcat taatttatta ccaggtttcg caccagaaat tcaaggggta 2100
   tatctacaac aagatgcaga tggacgtatt cgtgcttatg gctacgtatt aactgaaaac 2160
   taa 2163
<210> 62
   <211> 720
   <212> PRT
   <213> Pasteurella multocida
<400> 62
   Met Leu Asn Leu Lys Ile Ala Tyr Ser Pro Leu Ile Arg Pro Tyr Phe
   1               5                   10                  15
   His Thr Asn Arg Glu Leu Val Ser Val Gln Glu Thr Asp Phe Thr Asp
               20                  25                  30
   Ile Gly Ala Ile Ile Leu Ser Ser Glu Asp Ile Glu Asp Tyr Ile Asp
           35                  40                  45
   Ser Ile Gln Ala Thr Glu Phe Asn Ile Pro Val Phe Val Ala Val Ile
       50                  55                  60
   Glu Gly Gln Phe Leu Asp Pro Gln Phe Phe Asp Lys Val Tyr His Val
   65                  70                  75                  80
   Gln Asp Leu Asn Asn Tyr Asp Ile Asn Leu Tyr Ser Arg Gln Ile Glu
                   85                  90                  95
   Thr Ala Ala Arg Phe Tyr Glu Glu Lys Ile Leu Pro Pro Phe Phe Lys
               100                 105                 110
   Met Leu Ser Glu Tyr Val Glu Met Gly Asn Ser Ala Phe Asp Cys Pro
           115                 120                 125
   Gly His Gln Gly Gly Gln Tyr Phe Arg Lys His Pro Ala Gly Arg Tyr
       130                 135                 140
   Leu Tyr Asp Phe Tyr Gly Glu Asn Ile Phe Arg Ser Asp Ile Cys Asn
   145                 150                 155                 160
   Ala Asp Val Lys Leu Gly Asp Leu Leu Ile His Glu Gly Ala Ala Cys
                   165                 170                 175
   Asp Ala Gln Lys His Ala Ala Gln Val Phe Asn Ala Asp Lys Thr Tyr
               180                 185                 190
   Phe Val Leu Asn Gly Thr Ser Ser Ala Asn Lys Val Val Thr Asn Ala
           195                 200                 205
   Leu Leu Thr Pro Gly Asp Leu Val Leu Phe Asp Arg Asn Asn His Lys
       210                 215                 220
   Ser Ile His His Gly Ala Leu Ile Gln Ala Gly Ala Thr Pro Val Tyr
   225                 230                 235                 240
   Leu Glu Thr Ala Arg Asn Pro Phe Gly Phe Ile Gly Gly Ile Asp Ser
                   245                 250                 255
   His Cys Phe Asp Glu Asp Tyr Leu Lys Ser Leu Ile Lys Asp Val Ala
               260                 265                 270
   Pro Glu Lys Leu Thr Gln Ala Arg Pro Phe Arg Leu Ala Val Ile Gln
           275                 280                 285
   Leu Gly Thr Tyr Asp Gly Thr Ile Tyr Asn Ala Arg Gln Val Val Asp
       290                 295                 300
   Lys Ile Gly His Leu Cys Asp Tyr Ile Leu Phe Asp Ser Ala Trp Val
   305                 310                 315                 320
   Gly Tyr Glu Gln Phe Ile Pro Met Met Lys Asp Cys Ser Pro Leu Leu
                   325                 330                 335
   Leu Glu Leu Asn Glu Asn Asp Pro Gly Ile Ile Val Thr Gln Ser Val
               340                 345                 350
   His Lys Gln Gln Ala Gly Phe Ser Gln Ala Ser Gln Ile His Lys Lys
           355                 360                 365
   Asp Lys His Ile Lys Gly Gln Gln Arg Tyr Cys Asn His Lys Arg Phe
       370                 375                 380
   Asn Asn Ala Phe Met Leu His Ala Ser Thr Ser Pro Phe Tyr Pro Leu
   385                 390                 395                 400
   Phe Ala Thr Leu Asp Val Asn Ala Lys Ile Gln Gly Thr Pro Ala Gly
                   405                 410                 415
   Ile Arg Leu Trp His Asp Cys Val Lys Ile Gly Ile Glu Ala Arg Lys
               420                 425                 430
   Met Val Leu Asn Ser Cys Asp Leu Ile Lys Pro Phe Ile Pro Pro Tyr
           435                 440                 445
   Val Asn Gly Lys Lys Trp Gln Asp Tyr Asp Thr Glu Glu Met Ala Asn
       450                 455                 460
   Asp Leu Thr Phe Phe Lys Phe His Ala Asp Asp Lys Trp His Gln Phe
   465                 470                 475                 480
   Glu Gly Tyr Val Asp Asn Gln Tyr Phe Val Asp Pro Cys Lys Phe Met
                   485                 490                 495
   Leu Thr Thr Pro Gly Ile Asp Ile Glu Thr Gly Glu Tyr Glu Asp Phe
               500                 505                 510
   Gly Val Pro Ala Thr Ile Leu Ala Asn Tyr Leu Arg Glu Asn Gly Ile
           515                 520                 525
   Ile Pro Glu Lys Cys Asp Leu Asn Ser Ile Leu Phe Leu Leu Thr Pro
       530                 535                 540
   Ala Glu Thr Leu Thr Lys Met Gln Ser Leu Val Ala Gln Ile Ala Ala
   545                 550                 555                 560
   Phe Glu Gln His Ile Lys Lys Asp Ser Leu Leu Lys Glu Val Leu Pro
                   565                 570                 575
   Ser Val Tyr His Asn Asn Glu Lys Arg Tyr Glu Gly Tyr Thr Ile Arg
               580                 585                 590
   Arg Leu Cys Gln Glu Met His Asp Leu Tyr Val Ser Arg Asn Val Lys
           595                 600                 605
   Thr Leu Gln Arg Asn Leu Phe Arg Lys Ala Thr Leu Pro Glu Tyr Val
       610                 615                 620
   Met Asn Pro His Gln Ala Asn Leu Glu Phe Val Arg Asn Arg Val Glu
   625                 630                 635                 640
   Leu Val Pro Leu Thr Glu Ile Val Asn Arg Ile Ala Ala Glu Gly Ala
                   645                 650                 655
   Leu Pro Tyr Pro Pro Gly Val Leu Cys Val Val Pro Gly Glu Lys Trp
               660                 665                 670
   Ser Gln Thr Ala Gln Glu Tyr Phe Leu Ala Leu Glu Glu Gly Ile Asn
           675                 680                 685
   Leu Leu Pro Gly Phe Ala Pro Glu Ile Gln Gly Val Tyr Leu Gln Gln
       690                 695                 700
   Asp Ala Asp Gly Arg Ile Arg Ala Tyr Gly Tyr Val Leu Thr Glu Asn
   705                 710                 715                 720
<210> 63
   <211> 101
   <212> DNA
   <213> Pasteurella multocida
<400> 63
   gaaaaattag agaaacaaat agaatcactc aatctacaag aagattgttt tcttttagga      60
   aataaagata atccgtatcc attaataaaa aatgctaagc t                         101
<210> 64
   <211> 1179
   <212> DNA
   <213> Pasteurella multocida
<400> 64
   atgaatattc tatttgtaca taaaagcctt gtcgtcggag gcgctgaaag aattctaatt      60
   aactatttaa atattctatc tggatttaat gaattcaaag ttacattact tttactagaa     120
   aataaaggtg aagataacaa aaacatcaat caaatcaata aaaatattaa tatagatttt     180
   attctagaca atagtgagtc aagaaaatat actgaatttg aaaataaaat aaatcagcgc     240
   agcatcttca gaaaaatata taaatataaa ctatcaaaaa ttaataagat agaagaaaat     300
   agaataaaaa aatacattaa aaacaaggaa tttgatttaa ttgttaattt taactcacac     360
   cttgatttct tcttatcaaa caatcaaatt aacatcccga taattcgttg gatacacggt     420
   caagctcatt tagatgactg gtgcaacaga agagaatggt accaaaacat tcttcctaaa     480
   cacacttatt tctttgcaat tacaaaagaa atgcaaaaaa atgctcaaaa aatcttacta     540
   tcttacggga tccaagaaga aagaatacat atcttataca atcctattga tattaatttt     600
   gtccaggaac aatcaatcaa aaatactcat gacattcatc ataaacaata cttaattaac     660
   gtttctcgtt tagatataga taagaatcat gaacaaatga ttaatattta ttatcaatta     720
   aaaaaacgag gtatccaaga aaaattatat attgttgggg atggtgagtg tcgagaaaaa     780
   ttagagaaac aaatagaatc actcaatcta caagaagatt gctttctttt aggaaataaa     840
   gataatccgt atccattaat aaaaaatgct aagctattct tacacacctc tttgaaagag     900
   gggttaccga cagttatcct agaaagcatg gcctgcggta cacctgtaat atccatggac     960
   tgccctaccg gtccgaaaga aattctccga ggaggagaat ttggaggatt agtaaattta    1020
   ggtgacgaga atgcttttat acaaaaaaca ctctctttcc ttcaaaatca agatgaatac    1080
   aaccattatt gtaataaatt agaacaagct atttctcctt ttcgctttga agaaatcagc    1140
   actatactct tatctcattt acaaaaattc aatagttaa                           1179
<210> 65
   <211> 392
   <212> PRT
   <213> Pasteurella multocida
<400> 65
   Met Asn Ile Leu Phe Val His Lys Ser Leu Val Val Gly Gly Ala Glu
   1               5                   10                  15
   Arg Ile Leu Ile Asn Tyr Leu Asn Ile Leu Ser Gly Phe Asn Glu Phe
               20                  25                  30
   Lys Val Thr Leu Leu Leu Leu Glu Asn Lys Gly Glu Asp Asn Lys Asn
           35                  40                  45
   Ile Asn Gln Ile Asn Lys Asn Ile Asn Ile Asp Phe Ile Leu Asp Asn
       50                  55                  60
   Ser Glu Ser Arg Lys Tyr Thr Glu Phe Glu Asn Lys Ile Asn Gln Arg
   65                  70                  75                  80
   Ser Ile Phe Arg Lys Ile Tyr Lys Tyr Lys Leu Ser Lys Ile Asn Lys
                   85                  90                  95
   Ile Glu Glu Asn Arg Ile Lys Lys Tyr Ile Lys Asn Lys Glu Phe Asp
               100                 105                 110
   Leu Ile Val Asn Phe Asn Ser His Leu Asp Phe Phe Leu Ser Asn Asn
           115                 120                 125
   Gln Ile Asn Ile Pro Ile Ile Arg Trp Ile His Gly Gln Ala His Leu
       130                 135                 140
   Asp Asp Trp Cys Asn Arg Arg Glu Trp Tyr Gln Asn Ile Leu Pro Lys
   145                 150                 155                 160
   His Thr Tyr Phe Phe Ala Ile Thr Lys Glu Met Gln Lys Asn Ala Gln
                   165                 170                 175
   Lys Ile Leu Leu Ser Tyr Gly Ile Gln Glu Glu Arg Ile His Ile Leu
               180                 185                 190
   Tyr Asn Pro Ile Asp Ile Asn Phe Val Gln Glu Gln Ser Ile Lys Asn
           195                 200                 205
   Thr His Asp Ile His His Lys Gln Tyr Leu Ile Asn Val Ser Arg Leu
       210                 215                 220
   Asp Ile Asp Lys Asn His Glu Gln Met Ile Asn Ile Tyr Tyr Gln Leu
   225                 230                 235                 240
   Lys Lys Arg Gly Ile Gln Glu Lys Leu Tyr Ile Val Gly Asp Gly Glu
                   245                 250                 255
   Cys Arg Glu Lys Leu Glu Lys Gln Ile Glu Ser Leu Asn Leu Gln Glu
               260                 265                 270
   Asp Cys Phe Leu Leu Gly Asn Lys Asp Asn Pro Tyr Pro Leu Ile Lys
           275                 280                 285
   Asn Ala Lys Leu Phe Leu His Thr Ser Leu Lys Glu Gly Leu Pro Thr
       290                 295                 300
   Val Ile Leu Glu Ser Met Ala Cys Gly Thr Pro Val Ile Ser Met Asp
   305                 310                 315                 320
   Cys Pro Thr Gly Pro Lys Glu Ile Leu Arg Gly Gly Glu Phe Gly Gly
                   325                 330                 335
   Leu Val Asn Leu Gly Asp Glu Asn Ala Phe Ile Gln Lys Thr Leu Ser
               340                 345                 350
   Phe Leu Gln Asn Gln Asp Glu Tyr Asn His Tyr Cys Asn Lys Leu Glu
           355                 360                 365
   Gln Ala Ile Ser Pro Phe Arg Phe Glu Glu Ile Ser Thr Ile Leu Leu
       370                 375                 380
   Ser His Leu Gln Lys Phe Asn Ser
   385                 390
<210> 66
   <211> 222
   <212> DNA
   <213> Pasteurella multocida
<400> 66
   agcagagtaa gttctttttg cttgttaagt aacaaagctt attgtgacga cacgcgggtc    60
   taaattgtgt tttccccagc gagtagcgta aagtaatctt gtccagcaag gatagcgatc   120
   ccgacagaca tcgcttatgt aatggactga gcgtaatcta attgccgcat gccatgtttc   180
   aatttctttg aactcttgta tcgtccatga aaattcaggg cg                      222
<210> 67
   <211> 831
   <212> DNA
   <213> Pasteurella multocida
<400> 67
   atgtctgaca aaatttcacc caataagata tctgcgcttt cttctacttt attaatcact    60
   ctttgggcaa aagcagttga atatgataaa gccaatccat tactgaaaga tcgcgaagca   120
   gcaagaatga aaaaacagat tgactatgac tttcaaaagt ttgaatctgc tcatttatca   180
   caagtgggat gttgtggacg cgcaaaatta tttgatcaag aaagcttaaa atttctttca   240
   cagcaccaag acgcggttgt tgtgcagctt ggtgcgggct tagatgcacg ctttgaacgc   300
   ttaggcaaac cacaagtcag tgcgtggtat gatttagact tacctgaagt catcaatata   360
   cgtcgccaac ttttaccaga aacgagtaat cattatttgg ctgactcact tttcaataca   420
   gattggatga aaacagttag tcaacataac aaacccgttt tattaattct tgaaggcgta    480
   ttgatgtttt ttcctaaaga acaagtcaaa cagtttattg cctctgtggc tgaaaactta   540
   cctaacagca caatgatttt cgatattgtg cccccaatgg cagtcggtcg tagtaaatac   600
   cacgatgcac tcaaaaaaat agacagtcaa gaacgccctg aattttcatg gacaatacaa    660
   gagatcaaag aaattgaaac atggcatgcg gcaattaaat tacgctcagt ccattacata   720
   agcgatgtct gtcgggatcg ctatccttgc tggacaagat tactttacgc tactcgctgg   780
   ggaaaacaca atttagaccc gcgtgtcgtc acaataagct ttgttactta a            831
<210> 68
   <211> 276
   <212> PRT
   <213> Pasteurella multocida
<400> 68
   Met Ser Asp Lys Ile Ser Pro Asn Lys Ile Ser Ala Leu Ser Ser Thr
   1               5                   10                  15
   Leu Leu Ile Thr Leu Trp Ala Lys Ala Val Glu Tyr Asp Lys Ala Asn
               20                  25                  30
   Pro Leu Leu Lys Asp Arg Glu Ala Ala Arg Met Lys Lys Gln Ile Asp
           35                  40                  45
   Tyr Asp Phe Gln Lys Phe Glu Ser Ala His Leu Ser Gln Val Gly Cys
       50                  55                  60
   Cys Gly Arg Ala Lys Leu Phe Asp Gln Glu Ser Leu Lys Phe Leu Ser
   65                  70                  75                  80
   Gln His Gln Asp Ala Val Val Val Gln Leu Gly Ala Gly Leu Asp Ala
                   85                  90                  95
   Arg Phe Glu Arg Leu Gly Lys Pro Gln Val Ser Ala Trp Tyr Asp Leu
               100                 105                 110
   Asp Leu Pro Glu Val Ile Asn Ile Arg Arg Gln Leu Leu Pro Glu Thr
           115                 120                 125
   Ser Asn His Tyr Leu Ala Asp Ser Leu Phe Asn Thr Asp Trp Met Lys
       130                 135                 140
   Thr Val Ser Gln His Asn Lys Pro Val Leu Leu Ile Leu Glu Gly Val
   145                 150                 155                 160
   Leu Met Phe Phe Pro Lys Glu Gln Val Lys Gln Phe Ile Ala Ser Val
                   165                 170                 175
   Ala Glu Asn Leu Pro Asn Ser Thr Met Ile Phe Asp Ile Val Pro Pro
               180                 185                 190
   Met Ala Val Gly Arg Ser Lys Tyr His Asp Ala Leu Lys Lys Ile Asp
           195                 200                 205
   Ser Gln Glu Arg Pro Glu Phe Ser Trp Thr Ile Gln Glu Ile Lys Glu
       210                 215                 220
   Ile Glu Thr Trp His Ala Ala Ile Lys Leu Arg Ser Val His Tyr Ile
   225                 230                 235                 240
   Ser Asp Val Cys Arg Asp Arg Tyr Pro Cys Trp Thr Arg Leu Leu Tyr
                   245                 250                 255
   Ala Thr Arg Trp Gly Lys His Asn Leu Asp Pro Arg Val Val Thr Ile
               260                 265                 270
   Ser Phe Val Thr
           275
<210> 69
   <211> 55
   <212> DNA
   <213> Pasteurella multocida
<400> 69
   tcgatgaaaa acgccattat ggtcatggaa tcagctgcaa aattctcact ccaca 55
<210> 70
   <211> 1314
   <212> DNA
   <213> Pasteurella multocida
<400> 70
   atggtattac actatacccc tcatcaatcc gccccacgca acacaacatt cgttgcggaa     60
   attcttgatc ttgattatca aggacgtggt gtagccaaag tacaaggcaa aacgtggttc    120
   attgaaaatg cactgccaca agaaaaagtg gaagtgcgca ttgtcgatga aaaacgccat    180
   tatggtcatg ggatcagctg caaaattctc actccacatc cagatcgcca gtcagcaaaa    240
   tgtgcttact atgcccagtg cggtggttgc caaagtcaac atattccaat tgacatgcaa    300
   cgtcaggcta aacaacaagc cttattccaa cgcttacaac aattacaacc tcaagcgacc    360
   ttcatgccca tgatcgtcgc agcgccttgg cattatcgcc gtcgtgtgcg tttaagcgtg    420
   cggtttcatc ccaaaagcaa acaacttgcg atgggtttgc gtcagagaaa tactcaacaa    480
   atcgtgaatc tgcagcattg tgatgtgctt gaaatcccct taagtcaact cttacctaaa    540
   ctacatttgt tgttttcaac atggtccctg cctaaaaacc tagggcatgt ggagttagtg    600
   catgcggata atggaattgc gatgttatta cgccatacag gaaatttagc gcaaactgac    660
   cgcactttat taaccaattt tgcgcaacaa gaaaacttaa tgttgtttgt acaagatgat    720
   caacagatca cccaactaca tggcgaggca ccttactaca tactacgcga tggcaccaaa    780
   ttacagtttg atatccgtga ctttatccaa gtgaatgctg ttgtaaatca gaaaatgatt    840
   gatactgctc ttgagtggtt ggaactcaca tcgaacgata acgtattaga tttgttttgt    900
   ggtatgggaa acttcaccct cccaatcagt cgtcaggtca atcaggttgt gggcattgaa    960
   ggcgtaggag aaatggtgga gaaagcaaaa cgaaatgcgg aacaaaatca atgtgataat   1020
   gtccaattct atcaggcgaa tttagatcaa ccttttgtgc aacaacattg ggcgagccaa   1080
   cattttaata aaattttact ggacccacca cgtacaggcg cggcatttgc cttacatgcc   1140
   ttatgtgaat tgggcgcaga aaaaatctta tatgtttcct gcaatcctgc tacattagta   1200
   cgtgatacag cgattttatt acaatttaac taccgactta agaaagtcgc aatgatcgat   1260
   atgttcccca atacaggaca tttagaatcc atcagtttat ttgaaaaaga atag           1314
<210> 71
   <211> 437
   <212> PRT
   <213> Pasteurella multocida
<400> 71
   Met Val Leu His Tyr Thr Pro His Gln Ser Ala Pro Arg Asn Thr Thr
   1               5                   10                  15
   Phe Val Ala Glu Ile Leu Asp Leu Asp Tyr Gln Gly Arg Gly Val Ala
               20                  25                  30
   Lys Val Gln Gly Lys Thr Trp Phe Ile Glu Asn Ala Leu Pro Gln Glu
           35                  40                  45
   Lys Val Glu Val Arg Ile Val Asp Glu Lys Arg His Tyr Gly His Gly
       50                  55                  60
   Ile Ser Cys Lys Ile Leu Thr Pro His Pro Asp Arg Gln Ser Ala Lys
   65                  70                  75                  80
   Cys Ala Tyr Tyr Ala Gln Cys Gly Gly Cys Gln Ser Gln His Ile Pro
                   85                  90                  95
   Ile Asp Met Gln Arg Gln Ala Lys Gln Gln Ala Leu Phe Gln Arg Leu
               100                 105                 110
   Gln Gln Leu Gln Pro Gln Ala Thr Phe Met Pro Met Ile Val Ala Ala
           115                 120                 125
   Pro Trp His Tyr Arg Arg Arg Val Arg Leu Ser Val Arg Phe His Pro
       130                 135                 140
   Lys Ser Lys Gln Leu Ala Met Gly Leu Arg Gln Arg Asn Thr Gln Gln
   145                 150                 155                 160
   Ile Val Asn Leu Gln His Cys Asp Val Leu Glu Ile Pro Leu Ser Gln
                   165                 170                 175
   Leu Leu Pro Lys Leu His Leu Leu Phe Ser Thr Trp Ser Leu Pro Lys
               180                 185                 190
   Asn Leu Gly His Val Glu Leu Val His Ala Asp Asn Gly Ile Ala Met
           195                 200                 205
   Leu Leu Arg His Thr Gly Asn Leu Ala Gln Thr Asp Arg Thr Leu Leu
       210                 215                 220
   Thr Asn Phe Ala Gln Gln Glu Asn Leu Met Leu Phe Val Gln Asp Asp
   225                 230                 235                 240
   Gln Gln Ile Thr Gln Leu His Gly Glu Ala Pro Tyr Tyr Ile Leu Arg
                   245                 250                 255
   Asp Gly Thr Lys Leu Gln Phe Asp Ile Arg Asp Phe Ile Gln Val Asn
               260                 265                 270
   Ala Val Val Asn Gln Lys Met Ile Asp Thr Ala Leu Glu Trp Leu Glu
           275                 280                 285
   Leu Thr Ser Asn Asp Asn Val Leu Asp Leu Phe Cys Gly Met Gly Asn
       290                 295                 300
   Phe Thr Leu Pro Ile Ser Arg Gln Val Asn Gln Val Val Gly Ile Glu
   305                 310                 315                 320
   Gly Val Gly Glu Met Val Glu Lys Ala Lys Arg Asn Ala Glu Gln Asn
                   325                 330                 335
   Gln Cys Asp Asn Val Gln Phe Tyr Gln Ala Asn Leu Asp Gln Pro Phe
               340                 345                 350
   Val Gln Gln His Trp Ala Ser Gln His Phe Asn Lys Ile Leu Leu Asp
           355                 360                 365
   Pro Pro Arg Thr Gly Ala Ala Phe Ala Leu His Ala Leu Cys Glu Leu
       370                 375                 380
   Gly Ala Glu Lys Ile Leu Tyr Val Ser Cys Asn Pro Ala Thr Leu Val
   385                 390                 395                 400
   Arg Asp Thr Ala Ile Leu Leu Gln Phe Asn Tyr Arg Leu Lys Lys Val
   405 410 415
   Ala Met Ile Asp Met Phe Pro Asn Thr Gly His Leu Glu Ser Ile Ser
   420 425 430
   Leu Phe Glu Lys Glu
   435
<210> 72
   <211> 598
   <212> DNA
   <213> Pasteurella multocida
<220>
   <221> misc_feature
   <222> (412) .. (412)
   <223> A or C or G or T
<220>
   <221> misc_feature
   <222> (451).. (451)
   <223> A or C or G or T
<220>
   <221> misc_feature
   <222> (471) .. (471)
   <223> A or C or G or T
<220>
   <221> misc_feature
   <222> (503)..(503)
   <223> A or C or G or T
<220>
   <221> misc_feature
   <222> (546) .. (546)
   <223> A or C or G or T
<220>
   <221> misc_feature
   <222> (562)..(564)
   <223> A or C or G or T
<220>
   <221> mis_feature
   <222> (576) .. (576)
   <223> A or C or G or T
<220>
   <221> misc_feature
   <222> (588) .. (590)
   <223> A or C or G or T
<220>
   <221> misc_feature
   <222> (595) .. (595)
   <223> A or C or G or T.
<400> 72
   tcagtacttt gcttgcttaa gcaagtaaaa agtgcggtca tttttagcaa aaataaaggg 60
   cttctgtttg gaagcccttt gtgtattgca agttagtctt ttttatgagt gtattcttta 120
   tacatcgctt caatttgtgc tttatagcgt tccaaaatca ctttacgacg gagtttcaat 180
   gtcggagtaa tttcttccat ttttgtggta aatgcctgag gtaataaagt gaatttttta 240
   atttgctcaa agctaggcaa ttctttttgt aaatcattaa tacgctgttc aaacatttga 300
   agaatatcag aatgtttaat gagttctaaa cgatcgtgat attttatatt taattgtttg 360
   gcgtattctt caagactatt aaagcaaggc acaataagcg ctgagacata tnttttggca 420
   tccgcaatga ctgcaatttg ttcaataaat ntatctttac ccactttggt ntcaatatat 480
   tgtggagcaa tatattttcc atnggaggtt ttcattaact ctttgatacg atctgtaata 540
   tataantacc ttgtggatca annncccagc atcacnagtt tttaaaannn gtctnctg 598
<210> 73
   <211> 561
   <212> DNA
   <213> Pasteurella multocida
<220>
   <221> misc_feature
   <222> (560) .. (561)
   <223> A or C or G or T
<400> 73
   gagcaaagta gctgtccgcc gtatattgta agaagttggc ataagaatta acgcctaact 60
   tgaccgcatc gcccatcgga tctaaacgac ctacatgtac gccggtacct ttacttaaac 120
   tttcaattac ttttggtgtg aattgtggct ccgcaaataa gcaattcact ttatgttctt 180
   taatttcccg cttaatttcc gctaacgtct tagctcccgg cgccaccaac ggattaattg 240
   tgaaataacc ggtttgtttt aagccataag cattattgaa ataactatac gcatcatgga 300
   aaacataaaa ccctttttct ttaactggtg cgagttgctg tttaatttcc tcgctttgtt 360
   cagctaaagt gcggttaaat tctgccaaat tttgcgcaat tttctctttt ctctctggat 420
   aagcttccgt taaacgtgtt gctaagcgtg tcgcgacaat tttgctaatc tctggcgaat 480
   accacacatg ccagttagta ctgtgatcat gctcgtgttc atgtgcgtgg tcatgtttat 540
   gctcatggtc gtgtttgtgn n 561
<210> 74
   <211> 575
   <212> DNA
   <213> Pasteurella multocida
<220>
   <221> misc_feature
   <222> (574) . . (575)
   <223> A or C or G or T
<400> 74
   ttacttgctt aagcaagcaa agtagctgtc cgccgtatat tgtaagaagt tggcataaga    60
   attaacgcct aacttgaccg catcgcccat cggatctaaa cgacctacat gtacgccggt   120
   acctttactt aaactttcaa ttacttttgg tgtgaattgt ggctccgcaa ataagcaatt   180
   cactttatgt tctttaattt cccgcttaat tttcgctaac gtcttagctc ccggcgccac   240
   caacggatta attgtgaaat aaccggtttg ttttaagcca taagcattat tgaaataact   300
   atacgcatca tggaaaacat aaaacccttt ttctttaact ggtgcgagtt gctgtttaat   360
   tttctcgctt tgttcagcta aagtgcggtt aaattctgcc aaattttgcg caattttctc    420
   ttttctctct ggataagctt ccgttaaacg tgttgctaag cgtgtcgcga caattttgct   480
   aatctctggc gaataccaca catgccagtt agtactgtga tcatgctcgt gttcatgtgc   540
   gtggtcatgt ttatgctcat ggtcgtgttt gtgnn                              575
<210> 75
   <211> 1101
   <212> DNA
   <213> Pasteurella multocida
<400> 75
   atggcacgtt tcattaagac attgaaaaaa accgcattag cggcaagtat tgcttcttta    60
   gcaactgtgg caaatgcgac gattgtgact tcgattaaac cattaggttt tattgcttca   120
   tcgattgctg atggggtaac agacactgaa gtattagttc ctgcgggtgc ttcaccacat   180
   gattacagct taaaaccctc agatatacaa aaattacagg gggcggaatt aatcctgtgg   240
   gtcggggaag acattgatgc tttccttgat aaaacattac gtccaatgcc ttttaaaaag   300
   gtgttaagta ttgctgattt tgcggaaatt ggtggtttgc ttgaaggtga agcacatgat   360
   cataaacatg agcatgatca tactcacaaa cacgaccacg atcacaaaca cgaccacgat   420
   cacaaacacg accacgatca caaacatgag cacgatcata aacacgacca cgatcacaaa   480
   catgaccacg atcacaaaca cgaccatgct cacaagcatg agcacgatca caaacacgac   540
   catgagcata aacatgacca cgcacatgga cacgagcatg atcacagtac taactggcat   600
   gtgtggtatt cgccagagat tagcaaaatt gtcgcgacac gcttagcaac acgtttaacg   660
   gaagcttatc cagagaaaaa agagaaaatt gcgcaaaatt tggcagaatt taaccgtact   720
   ttagctgaac aaagcgagaa aattaaacag caactcgcac cagttaaaga aaaagggttt   780
   tatgttttcc atgatgcgta tagctatttc aataatgctt atggcttaaa acaaaccggt   840
   tatttcacaa ttaatccgtt ggtggcgccg ggagctaaga cgttagcgaa aattaagcag   900
   gaaattaaag aacataaagt gaattgctta tttgcggagc cacaattcac accaaaagta   960
   attgaaagtt taagtaaagg taccggtgta catgtaggtc gtttagatcc gatgggcgat  1020
   gcggtcaagt taggcgttaa ttcttatgcc aacttcttac aatatacggc ggacagctac  1080
   tttgcttgct taagcaagta a                                            1101
<210> 76
   <211> 366
   <212> PRT
   <213> Pasteurella multocida
<400> 76
   Met Ala Arg Phe Ile Lys Thr Leu Lys Lys Thr Ala Leu Ala Ala Ser
   1               5                   10                  15
   Ile Ala Ser Leu Ala Thr Val Ala Asn Ala Thr Ile Val Thr Ser Ile
               20                  25                  30
   Lys Pro Leu Gly Phe Ile Ala Ser Ser Ile Ala Asp Gly Val Thr Asp
           35                  40                  45
   Thr Glu Val Leu Val Pro Ala Gly Ala Ser Pro His Asp Tyr Ser Leu
       50                  55                  60
   Lys Pro Ser Asp Ile Gln Lys Leu Gln Gly Ala Glu Leu Ile Leu Trp
   65                  70                  75                  80
   Val Gly Glu Asp Ile Asp Ala Phe Leu Asp Lys Thr Leu Arg Pro Met
                   85                  90                  95
   Pro Phe Lys Lys Val Leu Ser Ile Ala Asp Phe Ala Glu Ile Gly Gly
               100                 105                 110
   Leu Leu Glu Gly Glu Ala His Asp His Lys His Glu His Asp His Thr
           115                 120                 125
   His Lys His Asp His Asp His Lys His Asp His Asp His Lys His Asp
       130                 135                 140
   His Asp His Lys His Glu His Asp His Lys His Asp His Asp His Lys
   145                 150                 155                 160
   His Asp His Asp His Lys His Asp His Ala His Lys His Glu His Asp
                   165                 170                 175
   His Lys His Asp His Glu His Lys His Asp His Ala His Gly His Glu
               180                 185                 190
   His Asp His Ser Thr Asn Trp His Val Trp Tyr Ser Pro Glu Ile Ser
           195                 200                 205
   Lys Ile Val Ala Thr Arg Leu Ala Thr Arg Leu Thr Glu Ala Tyr Pro
       210                 215                 220
   Glu Lys Lys Glu Lys Ile Ala Gln Asn Leu Ala Glu Phe Asn Arg Thr
   225                 230                 235                 240
   Leu Ala Glu Gln Ser Glu Lys Ile Lys Gln Gln Leu Ala Pro Val Lys
                   245                 250                 255
   Glu Lys Gly Phe Tyr Val Phe His Asp Ala Tyr Ser Tyr Phe Asn Asn
               260                 265                 270
   Ala Tyr Gly Leu Lys Gln Thr Gly Tyr Phe Thr Ile Asn Pro Leu Val
           275                 280                 285
   Ala Pro Gly Ala Lys Thr Leu Ala Lys Ile Lys Gln Glu Ile Lys Glu
       290                 295                 300
   His Lys Val Asn Cys Leu Phe Ala Glu Pro Gln Phe Thr Pro Lys Val
   305                 310                 315                 320
   Ile Glu Ser Leu Ser Lys Gly Thr Gly Val His Val Gly Arg Leu Asp
                   325                 330                 335
   Pro Met Gly Asp Ala Val Lys Leu Gly Val Asn Ser Tyr Ala Asn Phe
               340                 345                 350
   Leu Gln Tyr Thr Ala Asp Ser Tyr Phe Ala Cys Leu Ser Lys
           355                 360                 365
<210> 77
   <211> 70
   <212> DNA
   <213> Pasteurella multocida
<400> 77
   gctttgcatt tgagtcataa aatagtacag tacggtaatt ttctggatga ataccttttt     60
   tcatattggc                                                           70
<210> 78
   <211> 267
   <212> DNA
   <213> Pasteurella multocida
<400> 78
   atgaaaaaag gtattcatcc agaaaattac cgtactgtac tattttatga ctcaaatgca    60
   aagcaaggtt ttttaatccg ctcttgcgcc agaaccacaa cgaccatgaa atgggaagat   120
   ggtcatgaat atcctgtctt tatgtgtgat acctcctcag catcacaccc gtactataca   180
   ggtaaaacac gtcaaattgc gaatgaaggt cgtgcaagcg actttgtcaa tcgctacggc   240
   aaatttggca cattaaaatc aaaataa                                       267
<210> 79
   <211> 88
   <212> PRT
   <213> Pasteurella multocida
<400> 79
   Met Lys Lys Gly Ile His Pro Glu Asn Tyr Arg Thr Val Leu Phe Tyr
   1               5                   10                  15
   Asp Ser Asn Ala Lys Gln Gly Phe Leu Ile Arg Ser Cys Ala Arg Thr
               20                  25                  30
   Thr Thr Thr Met Lys Trp Glu Asp Gly His Glu Tyr Pro Val Phe Met
           35                  40                  45
   Cys Asp Thr Ser Ser Ala Ser His Pro Tyr Tyr Thr Gly Lys Thr Arg
       50                  55                  60
   Gln Ile Ala Asn Glu Gly Arg Ala Ser Asp Phe Val Asn Arg Tyr Gly
   65                  70                  75                  80
   Lys Phe Gly Thr Leu Lys Ser Lys
                   85
<210> 80
   <211> 506
   <212> DNA
   <213> Pasteurella multocida
<400> 80
   tgctccaact ctactttcaa cctatcctct gtccatgttc ttggaaacat cgtggataca    60
   cctttatttc cctttttctc caaaacttcg gggcagtagg agatcaacac cctcgcttca   120
   tagaccccat ttgggtattc cttaatcacc ttatctacaa tcacattgcc taagatggtg   180
   tgtcttaacg ctcccatgta aaaaaatggt caatttctca aaacaaaact ttttcaaaat   240
   tgaccgcact ttttcttcta actgttcctt ttcagaaaat caacaccttc acttaagaaa   300
   acccctacgc atatttctcc atcagggcaa tgatagcttg agagctagga cgatgggact   360
   catatttttt tatcccctca agtaattcat gttgtccatt aaaataatgt acgtttccac   420
   ctttatccag catcaattta agcagatcta gcgctttcag ggacataacc tgtcattgcc   480
   aatggaatca cttggtctcg atttgg                                        506
<210> 81
   <211> 348
   <212> DNA
   <213> Pasteurella multocida
<400> 81
   atgagagcgt taagacacac caccttaggc aatgtgattg tggataaggt gattaaggaa      60
   tacccaaatg gggtttatga agcgagggtg ttgatcccta acccgaaagc ccaaaccgat     120
   cctaccgccc cgaagttttt ggagaaaagg ggaaataaag gtgtatccac gatgtttcca     180
   agaacatgga cagaggatag gttgaaagtg gagttggagc atgcgtttaa aaatggtata     240
   cacgataaag ggcaagtatg gactgggata actaaatcag gtgttaaagt acaatggtat     300
   agaagtgaaa aaggtgagat aaccagtgtt catccaatct tagaataa                 348
<210> .. 82
   <211> 115
   <212> PRT
   <213> Pasteurella multocida
<400> 82
   Met Arg Ala Leu Arg His Thr Thr Leu Gly Asn Val Ile Val Asp Lys
   1               5                   10                  15
   Val Ile Lys Glu Tyr Pro Asn Gly Val Tyr Glu Ala Arg Val Leu Ile
               20                  25                  30
   Pro Asn Pro Lys Ala Gln Thr Asp Pro Thr Ala Pro Lys Phe Leu Glu
           35                  40                  45
   Lys Arg Gly Asn Lys Gly Val Ser Thr Met Phe Pro Arg Thr Trp Thr
       50                  55                  60
   Glu Asp Arg Leu Lys Val Glu Leu Glu His Ala Phe Lys Asn Gly Ile
   65                  70                  75                  80
   His Asp Lys Gly Gln Val Trp Thr Gly Ile Thr Lys Ser Gly Val Lys
                   85                  90                  95
   Val Gln Trp Tyr Arg Ser Glu Lys Gly Glu Ile Thr Ser Val His Pro
               100                 105                 110
   Ile Leu Glu
           115
<210> 83
   <211> 243
   <212> DNA
   <213> Pasteurella multocida
<400> 83
   gccgatatgg tacgtgtcga cattatgatc aatggtgagc gtgtcgatgc gttagcgtta      60
   atcgtgcata aagataatgc accttatcgt ggtcgtgaat tagtggaaaa aatgcgtgag     120
   ctcattccac gtcaacaatt tgatattgcg attcaagcgg cgattggtaa ccacattatt     180
   gcccgttcta ccgtcaaaca attacgtaaa aacgtattag caaaatgtta tggtggtgac     240
   gtg                                                                  243
<210> 84
   <211> 1797
   <212> DNA
   <213> Pasteurella multocida
<400> 84
   atgaagaata tacgtaactt ttctattatt gcacacattg accacggtaa atcgacactc 60
   tctgaccgcc ttattcaaac ttgcggtggc ttatctgatc gtgaaatgga agcccaagtg 120
   ttggattcca tggatcttga acgtgaacgt gggattacga tcaaagcaca aagtgtgacc 180
   ttaaattaca aagcgaaaga tggcgaaacc tatcaattaa atttcatcga tacgccaggt 240
   cacgttgact tctcttatga agtatcgcgt tctttagccg cttgtgaagg cgcattatta 300
   gtggtggatg cgggacaagg tgtcgaggca caaactttgg ctaactgcta taccgcaatt 360
   gaaatgaatt tagaagtggt gccgatttta aacaaaatcg acttgcccgc ggcagatcct 420
   gaacgcgttg cagaagaaat tgaagacatt gtcggtattg acgcgatgga agcggtgcgc 480
   tgttcagcaa aaaccggtgt gggtattgaa gatgtgttgg aagaaattgt gcataaaatc 540
   cctgcacccg aaggggatcc gaatgcacca ttacaagcct tgattatcga ctcgtggttt 600
   gataactact taggcgtagt atctttagtg cgcattaaaa acggcacatt acgcaaaggc 660
   gataaaatca aagtgatgtc tacagggcaa tcttacaatg tggatcgtct tggtattttc 720
   accccaaaac aagtcgatac caccatttta aattgtggtg aagtgggttg ggtggtgtgc 780
   gccattaaag atattttagg ggcacccgtg ggtgatacgc ttacttcgca caacaatcca 840
   gcttcttctg tcctgccggg ttttaagaaa gttaagccac aggtgtatgc cggtttattc 900
   ccaattagct ctgatgatta tgaagcattc cgtgatgcgc tcggtaaact tagtctaaac 960
   gatgcgtcat tattctatga accagaaaac tccaccgcac ttggtttcgg tttccgttgt 1020
   ggtttcttag gacttctcca catggagatt attcaagagc gtttagagcg cgaatacgat 1080
   cttgatctga ttaccacagc accgacagta gtgtatgaag tggaaaaaac cgacggtgaa 1140
   gtgatttatg tggatagccc atcaaaatta ccgccactca acaacattac ggagattcgt 1200
   gaaccgattg cagaatgtaa catgctgtta ccacaaacct acttaggtaa cgtcattacg 1260
   ctctgtgtag aaaaacgcgg tgtacaaacc aatatggttt accatggtaa ccaagtggca 1320
   ttgacctatg aaatcccaat gggcgaagtg gtactggatt tcttcgaccg cttaaaatca 1380
   acttctcgtg gttatgcttc cttagattat ggtttcaaac gtttccaagc cgccgatatg 1440
   gtacgtgtcg acattatgat caatggtgag cgtgtcgatg cgttagcgtt aatcgtgcat 1500
   aaagataatg caccttatcg tggtcgtgaa ttagtggaaa aaatgcgtga gctcattcca 1560
   cgtcaacaat ttgatattgc gattcaagcg gcgattggta accacattat tgcccgttct 1620
   actgtcaaac aattacgtaa aaacgtatta gcaaaatgtt atggtggtga cgttagccgt 1680
   aagaaaaaac tcttacagaa acaaaaagaa ggtaaaaaac gcatgaagtc tttgggtaac 1740
   gtcgaagtac cacaagaagc cttcttagcg attttacatg tcggaaaaga caaataa 1797
<210> 85
   <211> 598
   <212> PRT
   <213> Pasteurella multocida
<400> 85
   Met Lys Asn Ile Arg Asn Phe Ser Ile Ile Ala His Ile Asp His Gly
   1               5                   10                  15
   Lys Ser Thr Leu Ser Asp Arg Leu Ile Gln Thr Cys Gly Gly Leu Ser
               20                  25                  30
   Asp Arg Glu Met Glu Ala Gln Val Leu Asp Ser Met Asp Leu Glu Arg
           35                  40                  45
   Glu Arg Gly Ile Thr Ile Lys Ala Gln Ser Val Thr Leu Asn Tyr Lys
       50                  55                  60
   Ala Lys Asp Gly Glu Thr Tyr Gln Leu Asn Phe Ile Asp Thr Pro Gly
   65                  70                  75                  80
   His Val Asp Phe Ser Tyr Glu Val Ser Arg Ser Leu Ala Ala Cys Glu
                   85                  90                  95
   Gly Ala Leu Leu Val Val Asp Ala Gly Gln Gly Val Glu Ala Gln Thr
               100                 105                 110
   Leu Ala Asn Cys Tyr Thr Ala Ile Glu Met Asn Leu Glu Val Val Pro
           115                 120                 125
   Ile Leu Asn Lys Ile Asp Leu Pro Ala Ala Asp Pro Glu Arg Val Ala
       130                 135                 140
   Glu Glu Ile Glu Asp Ile Val Gly Ile Asp Ala Met Glu Ala Val Arg
   145                 150                 155                 160
   Cys Ser Ala Lys Thr Gly Val Gly Ile Glu Asp Val Leu Glu Glu Ile
                   165                 170                 175
   Val His Lys Ile Pro Ala Pro Glu Gly Asp Pro Asn Ala Pro Leu Gln
               180                 185                 190
   Ala Leu Ile Ile Asp Ser Trp Phe Asp Asn Tyr Leu Gly Val Val Ser
           195                 200                 205
   Leu Val Arg Ile Lys Asn Gly Thr Leu Arg Lys Gly Asp Lys Ile Lys
       210                 215                 220
   Val Met Ser Thr Gly Gln Ser Tyr Asn Val Asp Arg Leu Gly Ile Phe
   225                 230                 235                 240
   Thr Pro Lys Gln Val Asp Thr Thr Ile Leu Asn Cys Gly Glu Val Gly
                   245                 250                 255
   Trp Val Val Cys Ala Ile Lys Asp Ile Leu Gly Ala Pro Val Gly Asp
               260                 265                 270
   Thr Leu Thr Ser His Asn Asn Pro Ala Ser Ser Val Leu Pro Gly Phe
           275                 280                 285
   Lys Lys Val Lys Pro Gln Val Tyr Ala Gly Leu Phe Pro Ile Ser Ser
       290                 295                 300
   Asp Asp Tyr Glu Ala Phe Arg Asp Ala Leu Gly Lys Leu Ser Leu Asn
   305                 310                 315                 320
   Asp Ala Ser Leu Phe Tyr Glu Pro Glu Asn Ser Thr Ala Leu Gly Phe
                   325                 330                 335
   Gly Phe Arg Cys Gly Phe Leu Gly Leu Leu His Met Glu Ile Ile Gln
               340                 345                 350
   Glu Arg Leu Glu Arg Glu Tyr Asp Leu Asp Leu Ile Thr Thr Ala Pro
           355                 360                 365
   Thr Val Val Tyr Glu Val Glu Lys Thr Asp Gly Glu Val Ile Tyr Val
       370                 375                 380
   Asp Ser Pro Ser Lys Leu Pro Pro Leu Asn Asn Ile Thr Glu Ile Arg
   385                 390                 395                 400
   Glu Pro Ile Ala Glu Cys Asn Met Leu Leu Pro Gln Thr Tyr Leu Gly
                   405                 410                 415
   Asn Val Ile Thr Leu Cys Val Glu Lys Arg Gly Val Gln Thr Asn Met
               420                 425                 430
   Val Tyr His Gly Asn Gln Val Ala Leu Thr Tyr Glu Ile Pro Met Gly
           435                 440                 445
   Glu Val Val Leu Asp Phe Phe Asp Arg Leu Lys Ser Thr Ser Arg Gly
       450                 455                 460
   Tyr Ala Ser Leu Asp Tyr Gly Phe Lys Arg Phe Gln Ala Ala Asp Met
   465                 470                 475                 480
   Val Arg Val Asp Ile Met Ile Asn Gly Glu Arg Val Asp Ala Leu Ala
                   485                 490                 495
   Leu Ile Val His Lys Asp Asn Ala Pro Tyr Arg Gly Arg Glu Leu Val
               500                 505                 510
   Glu Lys Met Arg Glu Leu Ile Pro Arg Gln Gln Phe Asp Ile Ala Ile
           515                 520                 525
   Gln Ala Ala Ile Gly Asn His Ile Ile Ala Arg Ser Thr Val Lys Gln
       530                 535                 540
   Leu Arg Lys Asn Val Leu Ala Lys Cys Tyr Gly Gly Asp Val Ser Arg
   545                 550                 555                 560
   Lys Lys Lys Leu Leu Gln Lys Gln Lys Glu Gly Lys Lys Arg Met Lys
                   565                 570                 575
   Ser Leu Gly Asn Val Glu Val Pro Gln Glu Ala Phe Leu Ala Ile Leu
               580                 585                 590
   His Val Gly Lys Asp Lys
           595
<210> 86
   <211> 147
   <212> DNA
   <213> Pasteurella multocida
<400> 86
   aaaagttcga cttccgtaat cggtttttta gttaattgtt caatattgcg taataaacga     60
   cgttctcttg gttcaacaaa taacaatgca cgccctgtac gtccggcacg ccctgtacga    120
   ccaatacggt ggacataaga ctcagca                                         147
<210> 87
   <211> 1833
   <212> DNA
   <213> Pasteurella multocida
<400> 87
   atgactgaaa caacaatgac tttcaatgat ttaggcttgc ctgaatttct tcttaacgcc     60
   gtctctgact taggctttga aaccccttct ccaattcaac aaagttgtat cccaaacctg    120
   ttaaatgggc atgatgtgct aggtatggca caaactggaa gtggtaaaac cgccgccttt    180
   tcactccctt tattagcaca aattgattta gataaaaaat atccacaaat gttagtgatg    240
   gcaccgacac gtgagttagc catccaagta gcagatgcct gtgagcactt ttgcaaatat    300
   gcgaaaaata ccaatattgt taccctttat ggtggtcaac gctatgacat tcaattgcgt    360
   gctttacgcc aaggtgctca ggttgtagtg gggacacctg gtcgtatttt agatcacatt    420
   cgtcgtggca ctttagattt gtctaattta cgttttatgg tgttagatga agcggacgaa    480
   atgttacgta tgggctttat tgatgatgtt gaaacggtga tggcagaatt accagaacaa    540
   catcagactg cacttttctc agccaccatg ccagatccaa ttcgtcgtat tactaagcgt    600
   tttatgaaag atccgaaaga gattaaaatt aaatcgacgc aaacgacgaa tccagatatt    660
   acacagagtt gttggtatgt gcatggtttc cgtaaaaatg atgccttatt acgtttctta    720
   gaagtagaaa aatttgatgc cgcgattatc tttactcgta ctaaaacggg gacattagat    780
   gtaacggaat tgttggaaaa acatggtttc cgtgccgcag cattaaatgg cgatatgaca    840
   caacaattac gtgaacaaac gcttgatcgt ttaagaaatg gtagtttaga tatccttgtg    900
   gcaaccgatg tggcggcgcg tggtttagat gtggagcgca ttagcctcgt agtgaactat    960
   gatattccat tagatgctga gtcttatgtt caccgtattg gtcgtacagg gcgtgcagga   1020
   cgtacagggc gtgcattgtt atttgttgaa ccaagagaac gtcgtttatt acgtaatatt   1080
   gaacaattaa ctaaaaaacc gattacggaa gtcgaagtgc caaatcatga ggtactacaa   1140
   gcttgtcgcc gtgagaaatt taaagccaaa attacagtcc aattagagca tcatgattta   1200
   ggactttatc gtagcttact agaagatatg ttcaccgcgg atcaagatca ggaagatatt   1260
   gcggcggcga tgttgatgtt gttgcaaggt aaacaaaagc ttattttacc agccgatcca   1320
   attattgatc gtaaaacttc acgtggtgat cgtggcgagc gtcgtgaacg tggtggacgt   1380
   gaaaatccac gttcagcaga gcgtcgtggt tacggtacac cgcaggcgat ggatttatat   1440
   cgtattgaag taggacgttt agatggcgcg gaagtccgtc atattgttgg ggcgattgcc   1500
   aatgaaggtg atatcaatag tcgttatatt ggtcatatta aattatatga tgattacacc   1560
   acgattgaat taccacaagg tatgccgaaa gaattattag gtgtatttgc gaaaacacgc   1620
   gtgatgaaca aacaaatgca gatgtcattt gtgggagcgt ctaatgcagg ttcaagccgt   1680
   gatcgcgatg atttcgctga ccgccgtggt ggaaaacgta aaggacgcgg cgatgaacca   1740
   cgttttgggc gtgaagatcg taaatttaaa gaaaaaagtc agcgcacttt taatgatcgc   1800
   ccacgcagag aaagacgtga acgccaaaag taa                                1833
<210> 88
   <211> 610
   <212> PRT
   <213> Pasteurella multocida
<400> 88
   Met Thr Glu Thr Thr Met Thr Phe Asn Asp Leu Gly Leu Pro Glu Phe
   1               5                   10                  15
   Leu Leu Asn Ala Val Ser Asp Leu Gly Phe Glu Thr Pro Ser Pro Ile
               20                  25                  30
   Gln Gln Ser Cys Ile Pro Asn Leu Leu Asn Gly His Asp Val Leu Gly
           35                  40                  45
   Met Ala Gln Thr Gly Ser Gly Lys Thr Ala Ala Phe Ser Leu Pro Leu
       50                  55                  60
   Leu Ala Gln Ile Asp Leu Asp Lys Lys Tyr Pro Gln Met Leu Val Met
   65                  70                  75                  80
   Ala Pro Thr Arg Glu Leu Ala Ile Gln Val Ala Asp Ala Cys Glu His
                   85                  90                  95
   Phe Cys Lys Tyr Ala Lys Asn Thr Asn Ile Val Thr Leu Tyr Gly Gly
               100                 105                 110
   Gln Arg Tyr Asp Ile Gln Leu Arg Ala Leu Arg Gln Gly Ala Gln Val
           115                 120                 125
   Val Val Gly Thr Pro Gly Arg Ile Leu Asp His Ile Arg Arg Gly Thr
       130                 135                 140
   Leu Asp Leu Ser Asn Leu Arg Phe Met Val Leu Asp Glu Ala Asp Glu
   145                 150                 155                 160
   Met Leu Arg Met Gly Phe Ile Asp Asp Val Glu Thr Val Met Ala Glu
                   165                 170                 175
   Leu Pro Glu Gln His Gln Thr Ala Leu Phe Ser Ala Thr Met Pro Asp
               180                 185                 190
   Pro Ile Arg Arg Ile Thr Lys Arg Phe Met Lys Asp Pro Lys Glu Ile
           195                 200                 205
   Lys Ile Lys Ser Thr Gln Thr Thr Asn Pro Asp Ile Thr Gln Ser Cys
       210                 215                 220
   Trp Tyr Val His Gly Phe Arg Lys Asn Asp Ala Leu Leu Arg Phe Leu
   225                 230                 235                 240
   Glu Val Glu Lys Phe Asp Ala Ala Ile Ile Phe Thr Arg Thr Lys Thr
                   245                 250                 255
   Gly Thr Leu Asp Val Thr Glu Leu Leu Glu Lys His Gly Phe Arg Ala
               260                 265                 270
   Ala Ala Leu Asn Gly Asp Met Thr Gln Gln Leu Arg Glu Gln Thr Leu
           275                 280                 285
   Asp Arg Leu Arg Asn Gly Ser Leu Asp Ile Leu Val Ala Thr Asp Val
       290                 295                 300
   Ala Ala Arg Gly Leu Asp Val Glu Arg Ile Ser Leu Val Val Asn Tyr
   305                 310                 315                 320
   Asp Ile Pro Leu Asp Ala Glu Ser Tyr Val His Arg Ile Gly Arg Thr
                   325                 330                 335
   Gly Arg Ala Gly Arg Thr Gly Arg Ala Leu Leu Phe Val Glu Pro Arg
               340                 345                 350
   Glu Arg Arg Leu Leu Arg Asn Ile Glu Gln Leu Thr Lys Lys Pro Ile
           355                 360                 365
   Thr Glu Val Glu Val Pro Asn His Glu Val Leu Gln Ala Cys Arg Arg
       370                 375                 380
   Glu Lys Phe Lys Ala Lys Ile Thr Val Gln Leu Glu His His Asp Leu
   385                 390                 395                 400
   Gly Leu Tyr Arg Ser Leu Leu Glu Asp Met Phe Thr Ala Asp Gln Asp
                   405                 410                 415
   Gln Glu Asp Ile Ala Ala Ala Met Leu Met Leu Leu Gln Gly Lys Gln
               420                 425                 430
   Lys Leu Ile Leu Pro Ala Asp Pro Ile Ile Asp Arg Lys Thr Ser Arg
           435                 440                 445
   Gly Asp Arg Gly Glu Arg Arg Glu Arg Gly Gly Arg Glu Asn Pro Arg
       450                 455                 460
   Ser Ala Glu Arg Arg Gly Tyr Gly Thr Pro Gln Ala Met Asp Leu Tyr
   465                 470                 475                 480
   Arg Ile Glu Val Gly Arg Leu Asp Gly Ala Glu Val Arg His Ile Val
                   485                 490                 495
   Gly Ala Ile Ala Asn Glu Gly Asp Ile Asn Ser Arg Tyr Ile Gly His
               500                 505                 510
   Ile Lys Leu Tyr Asp Asp Tyr Thr Thr Ile Glu Leu Pro Gln Gly Met
           515                 520                 525
   Pro Lys Glu Leu Leu Gly Val Phe Ala Lys Thr Arg Val Met Asn Lys
       530                 535                 540
   Gln Met Gln Met Ser Phe Val Gly Ala Ser Asn Ala Gly Ser Ser Arg
   545                 550                 555                 560
   Asp Arg Asp Asp Phe Ala Asp Arg Arg Gly Gly Lys Arg Lys Gly Arg
                   565                 570                 575
   Gly Asp Glu Pro Arg Phe Gly Arg Glu Asp Arg Lys Phe Lys Glu Lys
               580                 585                 590
   Ser Gln Arg Thr Phe Asn Asp Arg Pro Arg Arg Glu Arg Arg Glu Arg
           595                 600                 605
   Gln Lys
       610
<210> 89
   <211> 187
   <212> DNA
   <213> Pasteurella multocida
<400> 89
   tctacgttaa cgccacccgt tgtattaata acattggcaa agccagaagc agcgatcatc 60
   acaaaaccaa tcatcgccat taaacgtaag ccttgttgga aaatgtcatt actttctttt 120
   aatttgaaaa taccacaaac agcaaaaata atcagaccgg ctaatccacc aataatagtt 180
   gaactct 187
<210> 90
   <211> 1359
   <212> DNA
   <213> Pasteurella multocida
<400> 90
<210> 91
   <211> 452
   <212> PRT
   <213> Pasteurella multocida
<400> 91
   Met Leu Leu Thr Asn Pro Val Val Ile Ser Ile Val Val Leu Leu Ala
   1 5 10 15
   Leu Ser Leu Leu Arg Ile Asn Val Val Ile Ala Leu Val Ile Ser Ala
   20 25 30
   Leu Val Ala Gly Leu Thr Gly Asn Leu Gly Val Ser Glu Thr Ile Lys
   35 40 45
   Thr Phe Thr Asn Gly Leu Gly Gly Gly Ala Glu Val Ala Met Asn Tyr
   50 55 60
   Ala Ile Leu Gly Ala Phe Ala Val Ala Ile Ser Lys Ser Gly Ile Thr
   65 70 75 80
   Asp Leu Leu Ala Tyr Lys Val Ile Lys Arg Leu Gly Asn Thr Pro Ser
                               85                  90                  95
   Ser Arg Ser Met Ala Gly Phe Lys Tyr Phe Ile Leu Thr Ile Leu Thr
                   100                 105                 110
   Leu Phe Ala Val Ser Ser Gln Asn Leu Leu Pro Val His Ile Ala Phe
               115                 120                 125
   Ile Pro Ile Val Ile Pro Pro Leu Leu Ala Ile Phe Asn Lys Leu Lys
       130                 135                 140
   Leu Asp Arg Arg Ala Val Ala Cys Val Leu Thr Phe Gly Leu Thr Ala
   145                 150                 155                 160
   Thr Tyr Met Leu Leu Pro Val Gly Phe Gly Lys Ile Phe Ile Glu Ser
                   165                 170                 175
   Ile Leu Val Lys Asn Ile Asn Gln Ala Gly Ala Thr Leu Gly Leu Gln
               180                 185                 190
   Thr Ser Val Ala Glu Val Ser Leu Ala Met Ala Val Pro Val Ile Gly
           195                 200                 205
   Met Ile Leu Gly Leu Leu Thr Ala Ile Phe Ile Ser Tyr Arg Lys Pro
       210                 215                 220
   Arg Glu Tyr Ala Met Met Arg Ser Glu Ile Ser Thr Gln Asp Ile Glu
   225                 230                 235                 240
   Ser His Val Ala Gln Ile Lys Pro Phe His Val Gly Ala Ser Leu Val
                   245                 250                 255
   Ala Ile Ile Val Thr Phe Ala Leu Gln Leu Phe Thr Ser Ser Thr Ile
               260                 265                 270
   Ile Gly Gly Leu Ala Gly Leu Ile Ile Phe Ala Val Cys Gly Ile Phe
           275                 280                 285
   Lys Leu Lys Glu Ser Asn Asp Ile Phe Gln Gln Gly Leu Arg Leu Met
       290                 295                 300
   Ala Met Ile Gly Phe Val Met Ile Ala Ala Ser Gly Phe Ala Asn Val
   305                 310                 315                 320
   Ile Asn Thr Thr Gly Gly Val Thr Ala Leu Val Glu Thr Phe Ser Gln
                   325                 330                 335
   Gly Phe Gly Ala Glu Asn Lys Gly Ile Ala Ala Phe Leu Met Leu Leu
               340                 345                 350
   Val Gly Leu Phe Ile Thr Met Gly Ile Gly Ser Ser Phe Ser Thr Val
           355                 360                 365
   Pro Ile Ile Ala Ser Ile Tyr Val Pro Leu Cys Leu Ser Leu Gly Phe
       370                 375                 380
   Ser Pro Leu Ala Thr Val Ser Leu Ile Gly Val Ser Ala Ala Leu Gly
   385                 390                 395                 400
   Asp Ala Gly Ser Pro Ala Ser Asp Ser Thr Leu Gly Pro Thr Ser Gly
                   405                 410                 415
   Leu Asn Ala Asp Gly Lys His Asp His Ile Trp Asp Ser Val Val Pro
               420                 425                 430
   Thr Phe Ile His Tyr Asn Ile Pro Leu Ile Leu Phe Gly Trp Leu Ala
           435                 440                 445
   Ala Met Tyr Leu
       450